# EUROPEAN PATENT SPECIFICATION

(11) **EP 2 828 238 B1**
(45) Date of publication and mention of the grant of the patent: **25.05.2016**
(21) Application number: 13721084.5
(22) Date of filing: 20.03.2013
(51) Int. Cl.: C07C 311/21, C07D 403/12, C07D 413/14, C07D 319/18, C07D 405/12, C07D 407/12, C07D 409/12, C07D 413/12, C07D 417/12, C07D 261/20, C07D 487/04, C07D 209/08, C07D 307/79, A61K 31/18, A61P 25/00

(54) **SULPHONAMIDE DERIVATIVES OF BENZYLAMINE FOR THE TREATMENT OF CNS DISEASES**
SULFONAMIDDERIVATE VON BENZYLAMIN ZUR BEHANDLUNG VON ZNS-ERKRANKUNGEN
DÉRIVÉS DE SULFONAMIDE DE LA BENZYLAMINE POUR LE TRAITEMENT DE MALADIES DU SYSTÈME NERVEUX CENTRAL (SNC)

(30) Priority: 20.03.2012 PL 39853312
(43) Date of publication of application: 28.01.2015
(73) Proprietor: Adamed Sp. z o.o., 05-152 Czosnów k/Warszawy (PL)
(72) Inventor: KOLACZKOWSKI, Marcin, 32-020 Wieliczka (PL); MARCINKOWSKA, Monika, 31-267 Kraków (PL); BUCKI, Adam, 32-100 Proszowice (PL); LYSAKOWSKI, Tomasz, 97-400 Belchatów (PL); PAWLOWSKI, Maciej, 32-020 Wieliczka (PL)
(74) Representative: Sitkowska, Jadwiga
(86) International application number: PCT/IB2013/052204
(87) International publication number: WO 2013/140347

(56) References cited:
- WO-A2-02/078693

## Description

### Field of the invention

The present invention relates to novel sulphonamide derivatives of benzylamine having affinity for dopaminergic, serotoninergic and adrenergic receptors, to a method for manufacturing thereof, to pharmaceutical compositions containing the same and to the use thereof. The compounds may be useful for the treatment of diseases of the central nervous system (CNS), such as schizophrenia, bipolar affective disorder, depression, anxiety disorders, sleep disorders or Alzheimer disease.

### State of the art

CNS disorders are considered a global and growing medical problem. A number of people suffering from those diseases constantly grows, particularly in highly developed countries and intensively developing ones. Approximately 20% of population in highly developed societies may suffer from CNS disorders. In addition a cost of treatment of such disorders represents nearly 35% of total expenses spent for treatment of all medical diseases in seven countries considered the biggest pharmaceutical markets.

Among all psychiatric diseases, schizophrenia, bipolar affective disorder, depression, anxiety, sleep disorders and addictions are the major ones. Among many others, the main neurologic disorders are Alzheimer's disease, Parkinson's disease, epilepsy and different pain disorders.

Antipsychotic drugs, which are main treatment of schizophrenia, are divided into two main classes on the basis of their liability to induce neurological side effects after long-term treatment. Typical antipsychotic drugs, such as chlorpromazine and haloperidol, induce after repeated administration various extrapyramidal side effects (EPS) including Parkinson-like symptoms and tardive dyskinesia. Repeated treatment with so called atypical antipsychotic drugs, such as clozapine, risperidone, olanzapine, quetiapine, ziprasidone and aripiprazole, is associated with a lower incidence of neurological side effects. Typical antipsychotics reduce positive symptoms but do not reduce negative symptoms and cognitive dysfunctions. Plasma prolactin levels are increased in humans, and there is a gain in body weight, potentially leading to the development of metabolic syndrome. Atypical antipsychotic drugs effectively reduce positive symptoms and also to some extent negative symptoms and cognitive disturbances, producing less serious EPS. Atypical antipsychotic drugs differ in their propensity to elevate plasma prolactin levels in humans. Typical antipsychotic drugs block dopamine D2 receptors in the mesolimbic and nigrostriatal system. This mechanism is responsible for the antipsychotic effect (reduction of positive symptoms) as well as induction of EPS. Clinical support for the dopamine hypothesis of antipsychotic drug action was provided by PET findings of high dopamine D2 receptor occupancy in the striatum of patients responding to different antipsychotic drug treatments. Patients with a good response show dopamine D2 receptor occupancy of more than 65% (Nord M, Farde L. Antipsychotic occupancy of dopamine receptors in schizophrenia. CNS Neuroscience & Therapeutics. 2011;17:97). The occurrence of EPS seems to be related to a higher occupancy of dopamine D2 receptors (above 80%). Atypical antipsychotics, also called second generation antipsychotic drugs, have clinical approvals for the treatment of psychosis and mania. Each drug has a unique pharmacodynamic and pharmacokinetic profile. Some of atypical antipsychotic drugs have additional antidepressant, anxiolytic or hypnotic profile (Schwartz T.L., Stahl S.M., CNS Neurosci. Ther.; 17(2), 110-7, 2011). Atypical antipsychotic drugs have in common a potent serotonin 5-HT2A receptor antagonism in relation to a weaker dopamine D2 receptor antagonism. This pharmacodynamic property is the basis of "atypicality" (Meltzer H.Y., Neuropsychopharmacology; 1, 193-6, 1989). Antagonism of 5-HT2A receptors likely allows more dopamine activity and neurotransmission to occur in the nigrostriatal system to avoid EPS. The same mechanism may allow small improvement in negative symptoms, and 5-HT2 antagonism in the tuberoinfundibular pathway may help to avoid hyper-prolactinemia (Schwartz T.L., Stahl S.M., CNS Neurosci. Ther.; 17(2),110-7, 2011).
The atypical antipsychotics have not fulfilled the initial expectations of improved negative symptoms and cognitive dysfunctions in schizophrenia. Therefore, more molecular targets are presently under investigation for the development of new drugs for the treatment of schizophrenia (Gray, J.A., Roth B.L.; Schizophr. Bull.; 33 (5, 1100-19, 2007).
Dopaminergic D2 receptors are the primary biological target of antipsychotic therapy. It is a recognized fact that that blockade of these receptors in the mesolimbic system is responsible for the antipsychotic activity of neuroleptics, in particular for preventing the positive symptoms. All antipsychotic drugs currently used reveal at least moderate affinity for dopamine D2 receptors. However, blockade of these receptors in the nigrostriatal system, if not compensated by a partial agonism to these receptors or by affecting other receptors (5-HT2A, 5-HT1A, alfa2c), may be a cause of extrapyramidal disorders, such as drug-induced parkinsonism, and within tuberoinfundibular pathway - of hyperprolactinaemia (Miyamoto S. et al., Mol. Psychiatry; 10(1), 79-104, 2005).

Dopaminergic D3 receptors are localized in limbic cortex and thus a preferential blockade of these receptors offers locally selective antidopaminergic activity. This results in increased effectiveness in reducing positive symptoms of schizophrenia without blockade of extrapyramidal system and therefore reduces the risk of the main side effect such as pseudoparkinson's syndrome. Moreover, several preclinical data suggest that D3 dopamine receptor antagonism is more efficient in reducing the negative symptoms of schizophrenia and improves working memory.
Serotoninergic neurons interact with dopaminergic neurons. Antagonistic activity of antipsychotics against serotoninergic receptors 5-HT2A type can stimulate the release of dopamine in the extrapyramidal, tuberoinfundibular systems and prefrontal cortex but not in the limbic system, what can result in alleviation of undesirable extrapyramidal symptoms and hyperprolactinaemia induced by D2 receptor blockade and in increased effectiveness of the drug against some of the negative symptoms of schizophrenia, without increasing the positive symptoms. It is considered that high affinity for 5-HT2A receptors, higher than for D2 receptors, is one of the reasons of atypicality of the second-generation antipsychotics. Similar effects to those caused by the blockade of 5-HT2A receptors, are achieved by stimulation of serotonin receptor type 5-HT1A (aripiprazole, ziprasidone). It is assumed that stimulation of 5-HT1A receptors takes part in the antipsychotic effect in combination with D2 receptor blockade, especially in the safety profile of a drug as well as is beneficial in fighting mood and cognitive symptoms of schizophrenia (Kim D. et al., Neurotherapeutics, 6(1), 78-85, 2009).
Serotoninergic receptors type 5-HT6 are exclusively localized in the central nervous system (CNS). Both the localization of the 5-HT6 receptors in limbic and cortical brain areas and relatively potent affinity and antagonistic activity of several antipsychotics (clozapine, olanzapine, sertindole) and antidepressants (mianserin, amitryptiline) at 5-HT6 receptors are suggestive of a potential role in pathophysiology and treatment of CNS disorders. Recent data in the literature indicate that blockade of 5-HT6 receptors may be implicated in a pro-cognitive effect due to the increase in cholinergic transmission, in antidepressant activity due to the increase in noradrenergic and dopaminergic one, as well as in anxiolytic effect. It is evident that the 5-HT6 receptor has emerged as a very interesting molecular target and antagonists of that receptor may serve as potential drugs in treatment of disorders characterized by cognitive impairments, such as Alzheimer's disease, schizophrenia, depression, anxiety (Liu K., Robichaud A., Drug Development Research 70,145-168, 2009; Wesotowska, A; Nikiforuk, A, Neuropharmacology 52(5), 1274-83, 2007). Moreover, 5-HT6 receptor antagonists have been demonstrated to be active in reduction of food intake and body weight by clinically approved mechanism that is consistent with an enhancement of satiety. Hence, several compounds with 5-HT6 receptor antagonistic activity are currently being clinically evaluated for the treatment of obesity (Heal D. et al., Pharmacology therapeutics, 117(2), 207-231, 2008).

Intensive research conducted since 1993 indicates that serotoninergic 5-HT7 receptors may play some role in the control of circadian rhythms, sleep, thermoregulation, cognitive processes, pain and migraine, as well as in neuronal excitability. Potent affinity and antagonistic activity of several antipsychotic and antidepressant drugs at 5-HT7 receptors suggest a potential role of these receptors in the pathophysiology of many neuropsychiatric disorders. Taking account of the behavioral data presented in the literature, it has been established that selective 5-HT7 receptor antagonists produce antidepressant and anxiolytic activity in rats and mice (Wesotowska A. et al., Neuropharmacology 51, 578-586, 2006). Using mouse models of antipsychotic activity, Galici et al. showed that a selective 5-HT7 receptor antagonist SB-269970 may also evoke antipsychotic-like effects (Galici R. et al., Behav. Pharmacol.; 19(2), 153-9, 2008).

Serotoninergic 5-HT2C and histaminergic H1 receptors localized in hypothalamus play an important role in food intake regulation. Blockade of both types of these receptors produced by antipsychotic drugs is most closely correlated with an increased risk of weight gain and diabetes. On the other hand, blockade of 5-HT2C receptors, mostly localized in cortical areas and in the hippocampus, striatum, septal nuclei, thalamic and midbrain nuclei, may produce beneficial antidepressant and pro-cognitive effects. In the substantia nigra, 5-HT2C receptors are co-localised with GABA, indicating that they yield indirect control of dopaminergic transmission. Consequently, the blockade of 5-HT2C receptors, together with the 5-HT2A receptor one, would potentiate the D2 receptor-mediated tonic inhibitory control of dopaminergic projection, with protective effect against extrapyramidal symptoms (Kim D. et al., Neuro therapeutics, 6(1), 78-85, 2009). Histaminergic H1 receptor blockade produced by antipsychotic drugs may be implicated in sedative effect that is clinically beneficial in controlling arousal accompanies the acute phase of psychosis. It seems that simultaneous reduction in affinity of new molecule for both types of these receptors may be an element that protects against excessive body weight. However, the total elimination of affinity for these receptors may not be necessary because of certain benefits of blockade of 5-HT2C and H1 receptors.

Blockade of alpha1 adrenergic receptors, despite potential peripheral adverse effects involving hypotension, may cause some central nervous system benefits involving decrease in the risk of extrapyramidal side effects caused be antipsychotics. This may be associated with interaction between noradrenergic and serotoninergic neurons (Horacek J. et al., CNS Drugs, 20(5), 389-409, 2006).

Because of important role of cholinergic system in the cognitive processes, current research is focused on substances which can directly or indirectly potentiate the activity of cholinergic system. This includes substances which are agonists of selected subtypes of nicotinic or muscarinic receptors and antagonists of 5-HT6 receptors. On the other hand, potential procognitive effects evoked by interaction with the above receptors may be masked by cholinolytic activity. Thus, in the scope of interest are substances free of antagonistic properties against cholinergic receptors. Moreover this strategy allows elimination of many undesired peripheral autonomic effects like constipations, dry mouth or tachycardia (Miyamoto S. et al., Mol. Psychiatry; 10(1), 79-104, 2005). In addition, it has been found that M3 muscarinic receptors are engaged in the control of insulin secretion, and their activation stimulates pancreas to secrete insulin. Hence, it can be expected that M3 receptors blockade may be unfavorable in terms of the risk of development of type II diabetes in patients treated with second generation antipsychotics (ex. olanzapine, clozapine, quetiapine). Recent research is focused on substances free of this undesired effect (Silvestre J.S., Prous J., Methods Find. Exp. Clin. Pharmacol.; 27(5), 289-304, 2005).

Another serious side effects caused by antipsychotic drugs, e.g. sertindole, ziprasidone, are cardiac arrhythmias associated with delayed repolarization of cardiomyocytes. This condition appears on electrocardiograms (ECG) as prolonged corrected QT interval (QTc), what is most often evoked by substances which block hERG potassium channels. To prevent introduction to the developmental pipelines drugs with pro-arrhythmic potential, at a very early stage of research new substances are screened in vitro for their potency to block hERG potassium channels, using electrophysiological methods (Recanatini M. et al., Med. Res. Rev., 25(2), 133-66, 2005).

Despite the advances that have been made in the development of antidepressants, there are clearly still unmet clinical needs with respect to both efficacy and side effects. These needs range from efficacy in treatment resistant patients (about 30%) to improved onset, to reductions in side effects such as sexual dysfunction, gastrointestinal events, sedation, weight gain. There are multiple approaches to improve current pharmacological means of modulating biogenic amines neurotransmission by either combining mechanisms or alternatively selectively stimulating/blocking receptor subtypes that may trigger improved efficacy or fewer side effects. One of them is combination therapies that maintain the benefits associated with selective serotonin reuptake inhibitors (SSRIs) (blockers of serotonin transporter) but attempt to either improve efficacy or reduce side effects by adding additional mechanism involving blockade of 5-HT2A or 5-HT2C receptors (Millan M., Neuro therapeutics, 6(1), 53-77, 2009). 5-HT2A receptor antagonists administered alone may produce antidepressant activity and also co-administered with SSRIs augment their antidepressant effects. The mechanism for this interaction may be a further increase in extracellular serotonin levels produced when SSRIs are given with 5-HT2A antagonists. Moreover, blockade of 5-HT2A receptors is part of the pharmacological profile of antidepressant drugs such as mianserin and mirtazapine. Presynaptic 5-HT1A receptors are associated with the risk for depressive behavior and their blockade augments the effects of SSRIs. Postsynaptic 5-HT1A receptors are essential for producing the antidepressant effects of 5-HT1A receptor agonists and possibly SSRIs. Thus partial agonism of 5-HT1A receptors is a preferred feature for new molecules the more that this mechanism occurs in approved anxiolytic buspirone and antidepressant/anxiolytic tandospirone.
Although introduction of new psychotropic drugs (among others neuroleptics, antidepressants, benzodiazepines, acetylocholinesterase inhibitors) since fifties of the XX century was an unquestioned breakthrough, therapy of neuropsychiatric disorders is still far from satisfactory both because of limited efficacy and wide spectrum of side effects evoked by available drugs. These disadvantages are a challenge for modern pharmacotherapy and there is a continuous effort to search for new, more effective psychotropic drugs.
In WO96/40100 arylsulphonamide derivatives acting as factor Xa inhibitors have been described, which are useful for the treatment of arterial and venous thrombotic occlusive disorders, inflammation, cancer and neurodegenerative diseases.

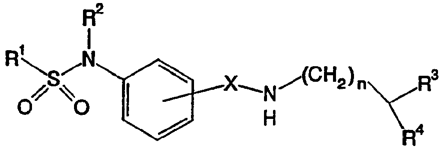

In WO98/27081 sulphonamides of the following general formula were disclosed

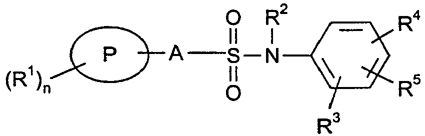

The above compounds reveal antagonistic activity towards 5-HT6 receptors and are potentially useful for the treatment of CNS diseases, such as anxiety, depression, epilepsy, Alzheimer disease etc.

Broad group of compounds having affinity for CyP proteins and potentially useful i.a. for the treatment of neurological diseases has been described in WO02/44126:

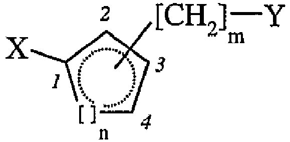

In DE10053794 sulphonamide derivatives of the following formula were described:

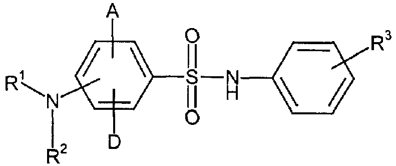

The above compounds reveal antagonistic acivity towards 5-HT6 receptors and are potentially useful for the treatment of CNS diseases.

GB2228933A discloses compounds having i.a. a vessel smooth muscle relaxation activity, and thus potentially useful as vasodilators or brain-circulation improving agents.

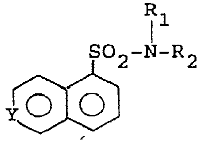

In WO2010/023448 tertiary sulphonamide derivatives of the following general formula have been described:

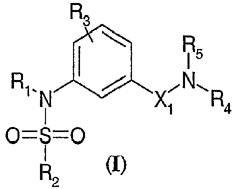

The above compounds act as potassium channel inhibitors and are useful for the treatment of autoimmune diseases, chronic inflammatory diseases and metabolic diseases.

WO2010/118055 discloses compounds of the following general formula: 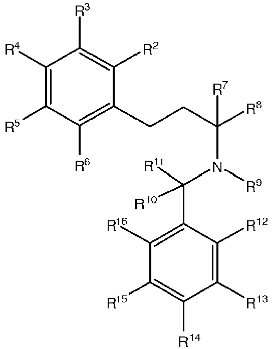 which are useful for inhibiting, treatment and ameliorating of cognitive disorders and/or of Alzheimer's disease.

In JP6072979 (general formula S1) and in JP6107614 (general formula S2) sulphonamide derivatives were disclosed, useful as antiulcer agents:

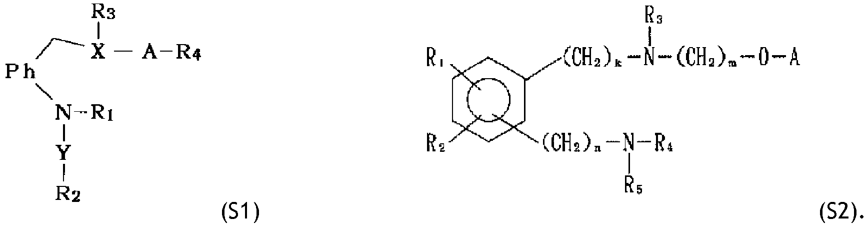

In DE19837638 and in DE19740785 a very broad group of compounds has been disclosed (general formula as depicted below), useful for the treatment of i.a. neurodegenerative diseases, cerebral ischemia, infections, autoimmune diseases, inflammatory diseases.

R¹-A-D-E-G-L-R²

In WO02/078693 arylethylbenzylamines of the following general formula have been described:

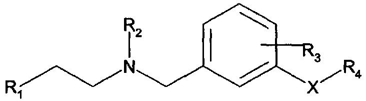

The above compounds have high affinity for 5-HT6 receptors and are thus useful for the treatment of CNS diseases.

In CN101817767A compounds of the following general formula have been described, useful for the treatment of diseases mediated by GPR40 receptor, such as diabetes or breast cancer:

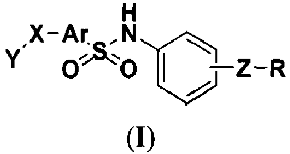

None of the above mentioned publications discloses a particular group of compounds-sulphonamide derivatives of benzylamine as described in the present invention.

### Aim of the invention

The aim of the present invention is to provide novel compounds potentially useful for the treatment of diseases of the central nervous system. A further aim of the invention is to provide novel compounds useful for the treatment of diseases of central nervous system having higher effectiveness compared to currently used medicaments. Yet further aim of the present invention is to provide novel compounds useful for the treatment of diseases of the central nervous system, which could allow to eliminate or minimize adverse effects associated with currently used therapies.

### Disclosure of the invention

The present invention relates to novel sulphonamide derivatives of benzylamine, having the structure represented by the general formula (I) 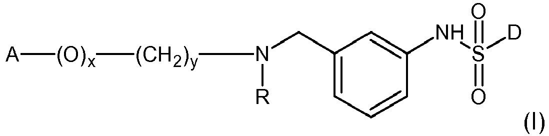 wherein
A represents:
   - phenyl unsubstituted or substituted with one substituent selected from the group consisting of halogen atom, C₁-C₃-alkyl, C₁-C₃-alkyloxy, OH and phenyl; or
   - 9- or 10-membered bicyclic group, linked to -(O)ₓ-(CH₂)_{y}- through one of its aromatic carbon atoms, consisting of benzene ring fused with:
      - 5-membered heteroaromatic ring containing 1 or 2 heteroatoms independently selected from the group consisting of N and O, wherein if such heteroaromatic ring contains 2 heteroatoms, then at least one is N, and wherein such bicyclic group is unsubstituted or substituted with halogen atom; or
      - 5- or 6-membered non-aromatic heterocyclic ring having 1 or 2 atoms of O, wherein heterocyclic ring is unsubstituted or substituted with one or more C₁-C₃-alkyl;
D represents a group selected from:
   - phenyl unsubstituted or substituted with one or two substituent independently selected from a group consisting of C₁-C₄-alkyl, C₁-C₃-alkyloxy, halogeno-C₁-C₃-alkyl, halogeno-C₁-C₃-alkyloxy, halogen atom, -CN, and phenyl;
   - naphthyl unsubstituted or substituted with one halogen atom;
   - thiophene unsubstituted or substituted with one or two substituents independently selected from a group consisting of C₁-C₃-alkyl, halogen atom, 5-membered heteroaromatic ring having 1 or 2 heteroatoms independently selected from N and O, wherein if such heteroaromatic ring contains 2 heteroatoms, then at least one is N;
   - bicyclic group consisting of imidazole ring fused with 5-membered non-aromatic carbocyclic ring, linked to sulphonamide moiety through one of its aromatic carbon atoms;
   - bicyclic group consisting of a benzene ring fused with 5-membered heteroaromatic ring, having 1 or 2 heteroatoms independently selected from a group consisting of N, O and S, wherein if such heteroaromatic ring contains 2 heteroatoms, then at least one is N and wherein the bicyclic group is unsubstituted or substituted with one or more substituent independently selected from a group consisting of C₁-C₃-alkyl and halogen atom, and is linked to sulphonamide moiety through one of its aromatic carbon atoms; and
   - bicyclic group consisting of a benzene ring fused with 5- or 6-membered non-aromatic heterocyclic ring having 1 or 2 heteroatoms independently selected from a group consisting of N and O, unsubstituted or substituted with one =O, and linked to sulphonamide moiety through one of carbon atoms of benzene ring;
R represents H or -CH₃;
x is 0 or 1;
y is 2 or 3;
and pharmaceutically acceptable salts and solvates thereof,
with the provisos that
- if x is 0 and y is 2, then D is naphthyl unsubstituted or substituted with one halogen atom, and
- if R represents -CH₃, then A is not unsubstituted or substituted phenyl.

One group of compounds of the present invention are compounds of formula (I), wherein A represents phenyl unsubstituted or substituted with one substituent selected from the group consisting of halogen atom, C₁-C₃-alkyl, C₁-C₃-alkyloxy, OH and phenyl.

Another group of compounds of the present invention are compounds of formula (I), wherein A represents 9- or 10-membered bicyclic group, linked to -(O)ₓ-(CH₂)_{y}- through one of its aromatic carbon atoms, consisting of benzene ring fused with:
- 5-membered heteroaromatic ring containing 1 or 2 heteroatoms independently selected from the group consisting of N and O, wherein if such heteroaromatic ring contains 2 heteroatoms, then at least one is N, and wherein such bicyclic group is unsubstituted or substituted with halogen atom; or
- 5- or 6-membered non-aromatic heterocyclic ring having 1 or 2 atoms of O, wherein heterocyclic ring is unsubstituted or substituted with one or more C₁-C₃-alkyl.

Another group are compounds of formula (I), wherein A represents 9- or 10-membered bicyclic group, linked to -(O)ₓ-(CH₂)_{y}- through one of its aromatic carbon atoms, consisting of benzene ring fused with 5-membered heteroaromatic ring containing 1 or 2 heteroatoms independently selected from the group consisting of N and O, wherein if such heteroaromatic ring contains 2 heteroatoms, then at least one is N, and wherein such bicyclic group is unsubstituted or substituted with halogen atom. Preferably, in this group A represents 1H-indolyl, in particular 1H-indol-4-yl, 1,2-benzoxazolyl, in particular 1,2-benzoxazol-3-yl, preferably substituted with halogen atom, such as 6-fluoro-1,2-benzoxazol-3-yl.

In a further group of compounds of formula (I) of the present invention A represents 9- or 10-membered bicyclic group, linked to -(O)ₓ-(CH₂)_{y}- through one of its aromatic carbon atoms, consisting of benzene ring fused with 5- or 6-membered non-aromatic heterocyclic ring having 1 or 2 atoms of O, wherein heterocyclic ring is unsubstituted or substituted with one or more C₁-C₃-alkyl. Preferably, A represents in this group 2,3-dihydro-1,4-benzodioxanyl, in particular 2,3-dihydro-1,4-benzodioxan-5-yl or 2,3-dihydro-1-benzofuranyl, preferably substituted with C₁-C₃-alkyl, in particular 2,2-dimethyl-2,3-dihydro-1-benzofuran-7-yl (also known as: 2,2-dimethyl-3H-benzofuran-7-yl).

In yet another group of compounds of formula (I) of the present invention D represents phenyl unsubstituted or substituted with one or two substituents independently selected from a group consisting of C₁-C₄-alkyl, C₁-C₃-alkyloxy, halogeno-C₁-C₃-alkyl, halogeno-C₁-C₃-alkyloxy, halogen atom, -CN, and phenyl. Preferably, D in this group of compounds represents phenyl unsubstituted or substituted with one or two substituents independently selected from a group consisting of C₁-C₄-alkyl, C₁-C₃-alkyloxy, halogeno-C₁-C₃-alkyl, halogeno-C₁-C₃-alkyloxy and halogen atom. In particular D in this group represents phenyl, methylphenyl, preferably 3-methylphenyl, propylphenyl, preferably 4-propylphenyl, methoxyphenyl, preferably 3-methoxyphenyl, trifluoromethoxyphenyl, preferably 4-trifluoromethoxyphenyl, monohalogenophenyl, preferably 4-fluorophenyl, 3-chlorophenyl, 3-bromophenyl or 4-iodophenyl, or dihalogenophenyl, preferably 3-chloro-4-fluorophenyl.

Further group are the compounds of formula (I), wherein D represents naphthyl unsubstituted or substituted with one halogen atom. Preferably in this group D represents unsubstituted naphthyl. Naphthyl may be linked to sulphur atom of sulphonamide moiety in position 1 (alpha) or 2 (beta) of naphthyl ring.

Another group of compounds of the present invention are compounds of formula (I), wherein D represents thiophene unsubstituted or substituted with one or two substituents independently selected from a group consisting of C₁-C₃-alkyl, halogen atom and 5-membered heteroaromatic ring having 1 or 2 heteroatoms independently selected from N and O, wherein if such heteroaromatic ring contains 2 heteroatoms, then at least one is N. Preferably, D in this group represents unsubstituted thiophene. More preferably D in this group represents thien-2-yl or thien-3-yl, in particular thien-3-yl.

In yet another group of compounds of formula (I) of the invention D represents bicyclic group consisting of imidazole ring fused with 5-membered non-aromatic carbocyclic ring, linked to sulphonamide moiety through one of its aromatic carbon atoms. Preferably in this group of compounds D represents 6,7-dihydro-5H-pyrrolo[1,2-a]imidazol-3-yl.

Yet another group of compounds of the present invention are compounds of formula (I), wherein D represents bicyclic group consisting of a benzene ring fused with 5-membered heteroaromatic ring, having 1 or 2 heteroatoms independently selected from a group consisting of N, O and S, wherein if such heteroaromatic ring contains 2 heteroatoms then at least one is N, and wherein the bicyclic group is unsubstituted or substituted with one or more substituents independently selected from a group consisting of C₁-C₃-alkyl and halogen atom, and linked to sulphonamide moiety through one of its aromatic carbon atoms. Preferably, in this group of compounds D represents bicyclic group consisting of a benzene ring fused with 5-membered heteroaromatic ring, having 1 or 2 heteroatoms independently selected from a group consisting of N and S, unsubstituted or substituted with one or more substituent independently selected from a group consisting of C₁-C₃-alkyl and halogen atom. More preferably, D in this group represents 1-methyl-indol-4-yl, 1-methyl-indol-5-yl, benzothiophenyl, in particular benzothiophen-3-yl, 5-fluoro-3-methyl-benzothiophen-2-yl, 5-chloro-3-methyl-benzothiophen-2-yl or 1,3-benzoxazol-5-yl.

In yet further group of compounds of formula (I) D represents bicyclic group consisting of a benzene ring fused with 5- or 6-membered non-aromatic heterocyclic ring having 1 or 2 heteroatoms independently selected from a group consisting of N and O, unsubstituted or substituted with one =O, and linked to sulphonamide moiety through one of carbon atoms of benzene ring. Preferably, D in this group of compounds represents unsubstituted bicyclic group consisting of a benzene ring fused with 5-membered non-aromatic heterocyclic ring having 2 atoms of O, linked to sulphonamide moiety through one of carbon atoms of benzene ring. Preferably in this group of compounds D represents 1,3-benzodioxol-5-yl.

A particular group of compounds of the present invention are compounds of formula (I), wherein R represents H, having the general formula (IA): 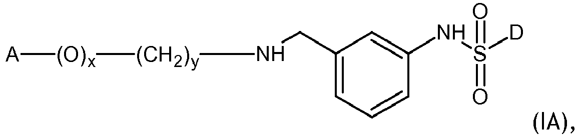 wherein A, D and x, y have the meanings as defined above for formula (I).

Another particular group of compounds of formula (I) are compounds, wherein R represents -CH₃, having the general formula (IB): 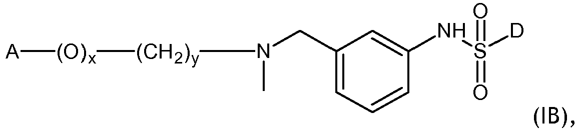 wherein A, D and x, y have the meanings as defined above for formula (I).

Another group of compounds are compounds of formula (I) of the invention, wherein x is 1 and y is 2 or x is 0 and y is 3. The compounds containing propyl (x = 0 and y =3) or oxyethyl (x = 1 and y = 2) spacer possess the exceptional activity in that they not only bind strongly to 5-HT6 receptors, but also bind strongly to D2, D3, 5-HT2A and 5-HT7 receptors (medium affinity). This is not the case for the compounds with ethyl spacer (x = 0 and y = 2) revealing strong affinity especially for 5-HT6 receptors. Such difference is caused by the required (optimal) distance between the basic nitrogen atom and the aryl moiety being achieved in the case of 3-unit, but not of 2-unit spacers.

For one of variants of the compounds of formulas (I), (IA) and (IB) of the invention x is 1 and y is 2. Further group of compounds of the invention are compounds of formula (IA), wherein x is 1 and y is 2.

In another variant for compounds of formulas (I), (IA) and (IB) x is 0 and y is 3. Further group of compounds of the invention are compounds of formula (IB), wherein x is 0 and y is 3.

One of groups of compounds of the invention are compounds of formula (I), wherein R represents H, having the general formula (IA): 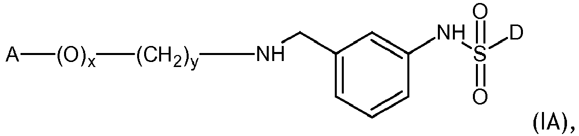 wherein A, D and x, y have the meanings as defined above for formula (I).

In a particular variant of compounds of formula (IA) moiety A represents phenyl unsubstituted or substituted with one C₁-C₃-alkyloxy. Preferably in this variant A represents phenyl or methoxyphenyl, in particular 3-methoxyphenyl.

In a further variant of compounds of formula (IA) moiety A preferably represents 9- or 10-membered bicyclic group, linked to -(O)ₓ-(CH₂)_{y}- through one of its aromatic carbon atoms, consisting of benzene ring fused with
- 5-membered heteroaromatic ring containing one atom of N, wherein such bicyclic group is unsubstituted; or
- 6-membered non-aromatic heterocyclic ring having 2 atoms of O, wherein heterocyclic ring is unsubstituted.

In the above variant the moiety A is preferably selected from the group consisting of indolyl, in particular 1H-indol-4-yl, and 2,3-dihydro-1,4-benzodioxinyl, in particular 2,3-dihydro-1,4-benzodioxin-5-yl.

Further group of compounds of the invention are compounds of formula (IA), wherein x is 1 and y is 2.

Another group of compounds of the invention are compounds of formula (I), wherein R represents -CH₃, having the general formula (IB): 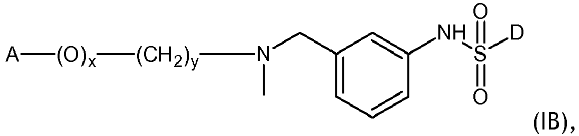 wherein A, D and x, y have the meanings as defined above for formula (I).

In a particular variant of compounds of formula (IB) moiety A represents 9-membered bicyclic group, linked to -(O)ₓ-(CH₂)_{y}- through one of its aromatic carbon atoms, consisting of benzene ring fused with 5-membered heteroaromatic ring containing two heteroatoms independently selected from a group consisting of N and O, wherein such bicyclic group is substituted with halogen atom. Preferably A in this variant represents 6-fluoro-1,2-benzoxazol-3-yl.

Further variant of compounds of the invention are compounds of formula (IB), wherein x is 0 and y is 3.

As it has been expressed by the proviso, if x is 0 and y is 2, then D is always naphthyl. Thus, a variant of compounds of the invention are compounds of formula (I), wherein x is 0, y is 2 and D is naphthyl unsubstituted or substituted with one halogen atom. Further variant of compounds of the invention are compounds of formula (IA), wherein x is 0, y is 2 and D is naphthyl. Yet further variant of compounds of the invention are compounds of formula (IB), wherein x is 0, y is 2 and D is naphthyl. In the above variants naphthyl is preferably unsubstituted.

The following specific compounds of formula (IA) of the invention can be mentioned:
1. N-[3-[(phenylethylamino)methyl]phenyl]naphthalene-2-sulphonamide,
2. N-[3-[(2-phenoxyethylamino)methyl]phenyl]naphthalene-2-sulphonamide,
3. N-[3-[(3-phenylpropylamino)methyl]phenyl]naphthalene-2-sulphonamide,
4. N-[3-[[2-(3-fluorophenoxy)ethylamino]methyl]phenyl]naphthalene-2-sulphonamide,
5. N-[3-[[3-(3-fluorophenoxy)propylamino]methyl]phenyl]naphthalene-2-sulphonamide,
6. N-[3-[[2-(3-chlorophenyl)ethylamino]methyl]phenyl]naphthalene-2-sulphonamide,
7. N-[3-[[2-(3-chlorophenoxy)ethylamino]methyl]phenyl]naphthalene-2-sulphonamide,
8. N-[3-[[2-(3-methylphenyl)ethylamino]methyl]phenyl]naphthalene-2-sulphonamide,
9. N-[3-[[2-(2-methoxyphenyl)ethylamino]methyl]phenyl]naphthalene-2-sulphonamide,
10. N-[3-[[2-(2-methoxyphenoxy)ethylamino]methyl]phenyl]naphthalene-2-sulphonamide,
11. N-[3-[[2-(3-methoxyphenyl)ethylamino]methyl]phenyl]naphthalene-2-sulphonamide,
12. N-[3-[[2-(2,3-dihydro-1,4-benzodioxin-5-yloxy)ethylamino]methyl]phenyl]-4-propyl-benzenesulphonamide,
13. N-[3-[[2-(3-methoxyphenoxy)ethylamino]methyl]phenyl]naphthalene-2-sulphonamide,
14. N-[3-[[2-(4-methoxyphenyl)ethylamino]methyl]phenyl]naphthalene-2-sulphonamide,
15. N-[3-[[2-(4-methoxyphenoxy)ethylamino]methyl]phenyl]naphthalene-2-sulphonamide,
16. N-[3-[[3-(4-methoxyphenyl)propylamino]methyl]phenyl]naphthalene-2-sulphonamide,
17. N-[3-[[2-(3-hydroxyphenyl)ethylamino]methyl]phenyl]naphthalene-2-sulphonamide,
18. N-[3-[[2-(2,3-dihydro-1,4-benzodioxin-5-yloxy)ethylamino]methyl]phenyl]benzene-sulphonamide,
19. N-[3-[[2-(2,3-dihydro-1,4-benzodioxin-5-yloxy)ethylamino]methyl]phenyl]-3-fluoro-benzenesulphonamide,
20. N-[3-[[2-(2,3-dihydro-1,4-benzodioxin-5-yloxy)ethylamino]methyl]phenyl]-4-fluoro-benzenesulphonamide,
21. 4-chloro-N-[3-[[2-(2,3-dihydro-1,4-benzodioxin-5-yloxy)ethylamino]methyl]phenyl]-benzenesulphonamide,
22. N-[3-[[2-(2,3-dihydro-1,4-benzodioxin-5-yloxy)ethylamino]methyl]phenyl]-4-iodo-benzenesulphonamide,
23. N-[3-[[2-(2,3-dihydro-1,4-benzodioxin-5-yloxy)ethylamino]methyl]phenyl]-3-methyl-benzenesulphonamide,
24. N-[3-[[2-(2,3-dihydro-1,4-benzodioxin-5-yloxy)ethylamino]methyl]phenyl]-3-(trifluoro-methyl)benzenesulphonamide,
25. N-[3-[[2-(2,3-dihydro-1,4-benzodioxin-5-yloxy)ethylamino]methyl]phenyl]-3-methoxy-benzenesulphonamide,
26. N-[3-[[2-(2,3-dihydro-1,4-benzodioxin-5-yloxy)ethylamino]methyl]phenyl]-4-methoxy-benzenesulphonamide,
27. N-[3-[[2-(2,3-dihydro-1,4-benzodioxin-5-yloxy)ethylamino]methyl]phenyl]-4-(trifluoro-methoxy)benzenesulphonamide,
28. 3-cyano-N-[3-[[2-(2,3-dihydro-1,4-benzodioxin-5-yloxy)ethylamino]methyl]phenyl]-benzenesulphonamide,
29. 4-cyano-N-[3-[[2-(2,3-dihydro-1,4-benzodioxin-5-yloxy)ethylamino]methyl]phenyl]-benzenesulphonamide,
30. N-[3-[[2-(2,3-dihydro-1,4-benzodioxin-5-yloxy)ethylamino]methyl]phenyl]-4-phenyl-benzenesulphonamide,
31. N-[3-[[2-(2,3-dihydro-1,4-benzodioxin-5-yloxy)ethylamino]methyl]phenyl]thiophene-2-sulphonamide,
32. N-[3-[[2-(2,3-dihydro-1,4-benzodioxin-5-yloxy)ethylamino]methyl]phenyl]thiophene-3-sulphonamide,
33. N-[3-[[2-(2,3-dihydro-1,4-benzodioxin-5-yloxy)ethylamino]methyl]phenyl]-2,5-dimethylthiophene-3-sulphonamide,
34. N-[3-[[2-(2,3-dihydro-1,4-benzodioxin-5-yloxy)ethylamino]methyl]phenyl]-5-isoxazol-5-yl-thiophene-2-sulphonamide,
35. N-[3-[[2-(2,3-dihydro-1,4-benzodioxin-5-yloxy)ethylamino]methyl]phenyl]naphthalene-1-sulphonamide,
36. N-[3-[[2-(2,3-dihydro-1,4-benzodioxin-5-yloxy)ethylamino]methyl]phenyl]naphthalene-2-sulphonamide,
37. 6-chloro-N-[3-[[2-(2,3-dihydro-1,4-benzodioxin-5-yloxy)ethylamino]methyl]phenyl]-naphthalene-2-sulphonamide,
38. N-[3-[[2-(2,3-dihydro-1,4-benzodioxin-5-yloxy)ethylamino]methyl]phenyl]-1,3-benzodioxol-5-sulphonamide,
39. N-[3-[[2-(2,3-dihydro-1,4-benzodioxin-5-yloxy)ethylamino]methyl]phenyl]-6,7-dihydro-5H-pyrrolo[1,2-a]imidazole-3-sulphonamide,
40. N-[3-[[2-(2,3-dihydro-1,4-benzodioxin-5-yloxy)ethylamino]methyl]phenyl]-1-methyl-indol-4-sulphonamide,
41. N-[3-[[2-(2,3-dihydro-1,4-benzodioxin-5-yloxy)ethylamino]methyl]phenyl]-1-methyl-indol-5-sulphonamide,
42. N-[3-[[2-(2,3-dihydro-1,4-benzodioxin-5-yloxy)ethylamino]methyl]phenyl]-benzofuran-2-sulphonamide,
43. N-[3-[[2-(2,3-dihydro-1,4-benzodioxin-5-yloxy)ethylamino]methyl]phenyl]benzothiophene-3-sulphonamide,
44. N-[3-[[2-(2,3-dihydro-1,4-benzodioxin-5-yloxy)ethylamino]methyl]phenyl]-5-fluoro-3-methylbenzothiophene-2-sulphonamide,
45. 3-chloro-N-[3-[[2-(2,3-dihydro-1,4-benzodioxin-5-yloxy)ethylamino]methyl]phenyl]-4-fluoro-benzenesulphonamide,
46. N-[3-[[2-[(2,2-dimethyl-3H-benzofuran-7-yl)oxy]ethylamino]methyl]phenyl]-3-methyl-benzenesulphonamide,
47. N-[3-[[2-[(2,2-dimethyl-3H-benzofuran-7-yl)oxy]ethylamino]methyl]phenyl]naphthalene-1-sulphonamide,
48. N-[3-[[2-[(2,2-dimethyl-3H-benzofuran-7-yl)oxy]ethylamino]methyl]phenyl]-naphthalene-2-sulphonamide,
49. N-[3-[[2-(1H-indol-4-yloxy)ethylamino]methyl]phenyl]-3-methylbenzenesulphonamide,
50. N-[3-[[2-(1H-indol-4-yloxy)ethylamino]methyl]phenyl]-4-phenylbenzenesulphonamide,
51. N-[3-[[2-(1 H-indol-4-yloxy)ethylamino]methyl]phenyl]naphthalene-1-sulphonamide,
52. N-[3-[[2-(1H-indol-4-yloxy)ethylamino]methyl]phenyl]naphthalene-2-sulphonamide,
53. N-[3-[[3-(5-fluoro-1,2-benzoxazol-3-yl)propylamino]methyl]phenyl]-3-methyl-benzenesulphonamide,
54. N-[3-[[3-(6-fluoro-1,2-benzoxazol-3-yl)propylamino]methyl]phenyl]naphthalene-1-sulphonamide,
55. N-[3-[[3-(6-fluoro-1,2-benzoxazol-3-yl)propylamino]methyl]phenyl]naphthalene-2-sulphonamide,
56. N-[3-[[2-(3-methylphenoxy)ethylamino]methyl]phenyl]naphthalene-2-sulphonamide,
57. N-[3-[[2-(3-hydroxyphenoxy)ethylamino]methyl]phenyl]naphthalene-2-sulphonamide,
58. N-[3-[[2-(1H-indol-4-yloxy)ethylamino]methyl]phenyl]-4-propyl-benzenesulphonamide,
59. N-[3-[[2-(1H-indol-4-yloxy)ethylamino]methyl]phenyl]thiophene-2-sulphonamide,
60.N-[3-[[2-(1H-indol-4-yloxy)ethylamino]methyl]phenyl]thiophene-3-sulphonamide,
61. N-[3-[[2-(1 H-indol-4-yloxy)ethylamino]methyl]phenyl]-2,5-dimethyl-thiophene-3-sulphonamide,
62. N-[3-[[2-(2,3-dihydro-1,4-benzodioxin-5-yloxy)ethylamino]methyl]phenyl]-3,4-difluorobenzenesulphonamide,
63. 3-chloro-N-[3-[[2-(2,3-dihydro-1,4-benzodioxin-5-yloxy)ethylamino]methyl]phenyl]-benzenesulphonamide,
64. 3,4-dichloro-N-[3-[[2-(2,3-dihydro-1,4-benzodioxin-5-yloxy)ethylamino]methyl]-phenyl]benzenesulphonamide,
65. 3-bromo-N-[3-[[2-(2,3-dihydro-1,4-benzodioxin-5-yloxy)ethylamino]methyl]phenyl]-benzenesulphonamide,
66. 4-bromo-N-[3-[[2-(2,3-dihydro-1,4-benzodioxin-5-yloxy)ethylamino]methyl]phenyl]-benzenesulphonamide,
67. N-[3-[[2-(2,3-dihydro-1,4-benzodioxin-5-yloxy)ethylamino]methyl]phenyl]-4-methyl-benzenesulphonamide,
68. 4-tert-butyl-N-[3-[[2-(2,3-dihydro-1,4-benzodioxin-5-yloxy)ethylamino]methyl]-phenyl]benzenesulphonamide,
69. N-[3-[[2-(2,3-dihydro-1,4-benzodioxin-5-yloxy)ethylamino]methyl]phenyl]-4-(trifluoro-methyl)benzenesulphonamide,
70. N-[3-[[2-(2,3-dihydro-1,4-benzodioxin-5-yloxy)ethylamino]methyl]phenyl]-2,3-dihydro-1,4-benzodioxino-7-sulphonamide,
71. N-[3-[[2-(2,3-dihydro-1,4-benzodioxin-5-yloxy)ethylamino]methyl]phenyl]-2-oxo-indoline-5-sulphonamide,
72. N-[3-[[2-(2,3-dihydro-1,4-benzodioxin-5-yloxy)ethylamino]methyl]phenyl]-1,3-benzo-thiazole-4-sulphonamide,
73. N-[3-[[2-(2,3-dihydro-1,4-benzodioxin-5-yloxy)ethylamino]methyl]phenyl]-1,3-benzo-thiazole-5-sulphonamide,
74. N-[3-[[2-(2,3-dihydro-1,4-benzodioxin-5-yloxy)ethylamino]methyl]phenyl]benzothiophene-2-sulphonamide,
75. 6-chloro-N-[3-[[2-(2,3-dihydro-1,4-benzodioxin-5-yloxy)ethylamino]methyl]phenyl]-benzothiophene-2-sulphonamide,
76. N-[3-[[2-(2,3-dihydro-1,4-benzodioxin-5-yloxy)ethylamino]methyl]phenyl]-5-methyl-benzothiophene-2-sulphonamide,
77. 5-chloro-N-[3-[[2-(2,3-dihydro-1,4-benzodioxin-5-yloxy)ethylamino]methyl]phenyl]-3-methylbenzothiophene-2-sulphonamide,
78. N-[3-[[2-(1H-indol-4-yloxy)ethylamino]methyl]phenyl]benzenesulphonamide,
79. 3-fluoro-N-[3-[[2-(1H-indol-4-yloxy)ethylamino]methyl]phenyl]benzenesulphonamide,
80. 4-fluoro-N-[3-[[2-(1H-indol-4-yloxy)ethylamino]methyl]phenyl]benzenesulphonamide,
81. 3,4-difluoro-N-[3-[[2-(1H-indol-4-yloxy)ethylamino]methyl]phenyl]benzenesulphonamide,
82. 3-chloro-N-[3-[[2-(1H-indol-4-yloxy)ethylamino]methyl]phenyl]benzenesulphonamide,
83. 4-chloro-N-[3-[[2-(1H-indol-4-yloxy)ethylamino]methyl]phenyl]benzenesulphonamide,
84. 3,4-dichloro-N-[3-[[2-(1H-indol-4-yloxy)ethylamino]methyl]phenyl]benzenesulphonamide,
85. 3-bromo-N-[3-[[2-(1H-indol-4-yloxy)ethylamino]methyl]phenyl]benzenesulphonamide,
86. 4-bromo-N-[3-[[2-(1H-indol-4-yloxy)ethylamino]methyl]phenyl]benzenesulphonamide,
87. N-[3-[[2-(1H-indol-4-yloxy)ethylamino]methyl]phenyl]-4-iodobenzenesulphonamide,
88. N-[3-[[2-(1H-indol-4-yloxy)ethylamino]methyl]phenyl]-4-methylbenzenesulphonamide,
89. 4-tert-butyl-N-[3-[[2-(1H-indol-4-yloxy)ethylamino]methyl]phenyl]benzenesulphonamide,
90. N-[3-[[2-(1H-indol-4-yloxy)ethylamino]methyl]phenyl]-3-(trifluoromethyl)benzenesulphonamide,
91. N-[3-[[2-(1H-indol-4-yloxy)ethylamino]methyl]phenyl]-4-(trifluoromethyl)benzenesulphonamide,
92. N-[3-[[2-(1 H-indol-4-yloxy)ethylamino]methyl]phenyl]-3-methoxybenzenesulphonamide,
93. N-[3-[[2-(1H-indol-4-yloxy)ethylamino]methyl]phenyl]-4-methoxybenzenesulphonamide,
94. N-[3-[[2-(1H-indol-4-yloxy)ethylamino]methyl]phenyl]-4-(trifluoromethoxy)benzenesulphonamide,
95. 3-cyano-N-[3-[[2-(1H-indol-4-yloxy)ethylamino]methyl]phenyl]benzenesulphonamide,
96. 4-cyano-N-[3-[[2-(1H-indol-4-yloxy)ethylamino]methyl]phenyl]benzenesulphonamide,
97. N-[3-[[2-(1 H-indol-4-yloxy)ethylamino]methyl]phenyl]-5-isoxazol-5-yl-thiophene-2-sulphonamide,
98. 6-chloro-N-[3-[[2-(1H-indol-4-yloxy)ethylamino]methyl]phenyl]naphthalene-2-sulphonamide,
99. N-[3-[[2-(1H-indol-4-yloxy)ethylamino]methyl]phenyl]-2,3-dihydro-1,4-benzodioxino-7-sulphonamide,
100. N-[3-[[2-(1H-indol-4-yloxy)ethylamino]methyl]phenyl]-1,3-benzodioxole-5-sulphonamide,
101. N-[3-[[2-(1H-indol-4-yloxy)ethylamino]methyl]phenyl]-2-oxo-indoline-5-sulphonamide,
102. N-[3-[[2-(1H-indol-4-yloxy)ethylamino]methyl]phenyl]-6,7-dihydro-5H-pyrrolo-[1,2-a]imidazole-3-sulphonamide,
103. N-[3-[[2-(1H-indol-4-yloxy)ethylamino]methyl]phenyl]-1-methyl-indole-4-sulphonamide,
104. N-[3-[[2-(1H-indol-4-yloxy)ethylamino]methyl]phenyl]-1-methyl-indole-5-sulphonamide,
105. N-[3-[[2-(1H-indol-4-yloxy)ethylamino]methyl]phenyl]-1,3-benzothiazole-4-sulphonamide,
106. N-[3-[[2-(1H-indol-4-yloxy)ethylamino]methyl]phenyl]-1,3-benzothiazole-5-sulphonamide,
107. N-[3-[[2-(1H-indol-4-yloxy)ethylamino]methyl]phenyl]benzofuran-2-sulphonamide,
108. N-[3-[[2-(1 H-indol-4-yloxy)ethylamino]methyl]phenyl]benzothiophene-2-sulphonamide,
109. N-[3-[[2-(1 H-indol-4-yloxy)ethylamino]methyl]phenyl]benzothiophene-3-sulphonamide,
110. 6-chloro-N-[3-[[2-(1H-indol-4-yloxy)ethylamino]methyl]phenyl]benzothiophene-2-sulphonamide,
111. N-[3-[[2-(1 H-indol-4-yloxy)ethylamino]methyl]phenyl]-5-methyl-benzothiophene-2-sulphonamide,
112. 5-fluoro-N-[3-[[2-(1H-indol-4-yloxy)ethylamino]methyl]phenyl]-2-methylbenzothiophene-3-sulphonamide,
113. 5-chloro-N-[3-[[2-(1H-indol-4-yloxy)ethylamino]methyl]phenyl]-3-methylbenzothiophene-2-sulphonamide,
114. 3-chloro-4-fluoro-N-[3-[[2-(1H-indol-4-yloxy)ethylamino]methyl]phenyl]benzenesulphonamide,
115. N-[3-[[3-(6-fluoro-1,2-benzoxazol-3-yl)propylamino]methyl]phenyl]thiophene-2-sulphonamide,
116. N-[3-[[3-(6-fluoro-1,2-benzoxazol-3-yl)propylamino]methyl]phenyl]thiophene-3-sulphonamide,
117. N-[3-[[3-(6-fluoro-1,2-benzoxazol-3-yl)propylamino]methyl]phenyl]-2,5-dimethyl-thiophene-3-sulphonamide,
118. N-[3-[[3-(6-fluoro-1,2-benzoxazol-3-yl)propylamino]methyl]phenyl]-1H-indole-5-sulphonamide,
119. N-[3-[[3-(6-fluoro-1,2-benzoxazol-3-yl)propylamino]methyl]phenyl]benzo-thiophene-2-sulphonamide,
120. N-[3-[[3-(6-fluoro-1,2-benzoxazol-3-yl)propylamino]methyl]phenyl]benzo-thiophene-3-sulphonamide,
121. 6-chloro-N-[3-[[3-(6-fluoro-1,2-benzoxazol-3-yl)propylamino]methyl]phenyl]-benzothiophene-2-sulphonamide,
122. N-[3-[[3-(6-fluoro-1,2-benzoxazol-3-yl)propylamino]methyl]phenyl]-5-methyl-benzothiophene-2-sulphonamide,
123. 5-fluoro-N-[3-[[3-(6-fluoro-1,2-benzoxazol-3-yl)propylamino]methyl]phenyl]-3-methyl-benzothiophene-2-sulphonamide,
124. N-[3-[[2-(2,3-dihydro-1,4-benzodioxin-5-yloxy)ethylmethylamino]methyl]phenyl]-benzenesulphonamide,
125. N-[3-[[2-[(2,2-dimethyl-3H-benzofuran-7-yl)oxy]ethylmethylamino]methyl]-phenyl]benzenesulphonamide,
126. N-[3-[[2-(1H-indol-4-yloxy)ethylmethylamino]methyl]phenyl]benzene-sulphonamide,
127. N-[3-[[3-(6-fluoro-1,2-benzoxazol-3-yl)propylmethylamino]methyl]phenyl]-benzenesulphonamide,
128. 4-fluoro-N-[3-[[3-(6-fluoro-1,2-benzoxazol-3-yl)propylmethylamino]methyl]-phenyl]benzenesulphonamide,
129. 3,4-difluoro-N-[3-[[3-(6-fluoro-1,2-benzoxazol-3-yl)propylmethylamino]methyl]-phenyl]benzenesulphonamide,
130. 3-chloro-N-[3-[[3-(6-fluoro-1,2-benzoxazol-3-yl)propylmethylamino]methyl]-phenyl]benzenesulphonamide,
131. 4-chloro-N-[3-[[3-(6-fluoro-1,2-benzoxazol-3-yl)propylmethylamino]methyl]-phenyl]benzenesulphonamide,
132. 3,4-dichloro-N-[3-[[3-(6-fluoro-1,2-benzoxazol-3-yl)propylmethylamino]methyl]-phenyl]benzenesulphonamide,
133. 3-bromo-N-[3-[[3-(6-fluoro-1,2-benzoxazol-3-yl)propylmethylamino]methyl]-phenyl]benzenesulphonamide,
134. 4-bromo-N-[3-[[3-(6-fluoro-1,2-benzoxazol-3-yl)propylmethylamino]methyl]-phenyl]benzenesulphonamide,
135. N-[3-[[3-(6-fluoro-1,2-benzoxazol-3-yl)propylmethylamino]methyl]phenyl]-4-iodo-benzenesulphonamide,
136. N-[3-[[3-(6-fluoro-1,2-benzoxazol-3-yl)propylmethylamino]methyl]phenyl]-3-methylbenzenesu lphonamide,
137. N-[3-[[3-(6-fluoro-1,2-benzoxazol-3-yl)propylmethylamino]methyl]phenyl]-4-methylbenzenesu lphonamide,
138. N-[3-[[3-(6-fluoro-1,2-benzoxazol-3-yl)propylmethylamino]methyl]phenyl]-4-propylbenzenesulphonamide,
139. 4-tert-butyl-N-[3-[[3-(6-fluoro-1,2-benzoxazol-3-yl)propylmethylamino]methyl]-phenyl]benzenesulphonamide,
140. N-[3-[[3-(6-fluoro-1,2-benzoxazol-3-yl)propylmethylamino]methyl]phenyl]-3-(trifluoromethyl)benzenesulphonamide,
141. N-[3-[[3-(6-fluoro-1,2-benzoxazol-3-yl)propylmethylamino]methyl]phenyl]-4-(trifluoromethyl)benzenesulphonamide,
142. N-[3-[[3-(6-fluoro-1,2-benzoxazol-3-yl)propylmethylamino]methyl]phenyl]-3-methoxybenzenesulphonamide,
143. N-[3-[[3-(6-fluoro-1,2-benzoxazol-3-yl)propylmethylamino]methyl]phenyl]-4-methoxybenzenesulphonamide,
144. N-[3-[[3-(6-fluoro-1,2-benzoxazol-3-yl)propylmethylamino]methyl]phenyl]-4-(trifluoromethoxy)benzenesulphonamide,
145. 3-cyano-N-[3-[[3-(6-fluoro-1,2-benzoxazol-3-yl)propylmethylamino]methyl]-phenyl]benzenesulphonamide,
146. N-[3-[[3-(6-fluoro-1,2-benzoxazol-3-yl)propylmethylamino]methyl]phenyl]-4-phenyl-benzenesulphonamide,
147. N-[3-[[3-(6-fluoro-1,2-benzoxazol-3-yl)propylmethylamino]methyl]phenyl]-thiophene-2-sulphonamide,
148. N-[3-[[3-(6-fluoro-1,2-benzoxazol-3-yl)propylmethylamino]methyl]phenyl]-thiophene-3-sulphonamide,
149. N-[3-[[3-(6-fluoro-1,2-benzoxazol-3-yl)propylmethylamino]methyl]phenyl]-2,5-dimethylthiophene-3-sulphonamide,
150. N-[3-[[3-(6-fluoro-1,2-benzoxazol-3-yl)propylmethylamino]methyl]phenyl]-5-isoxazol-5-yl-thiophene-2-sulphonamide,
151. N-[3-[[3-(6-fluoro-1,2-benzoxazol-3-yl)propylmethylamino]methyl]phenyl]-naphthalene-1-sulphonamide,
152. N-[3-[[3-(6-fluoro-1,2-benzoxazol-3-yl)propylmethylamino]methyl]phenyl]-naphthalene-2-sulphonamide,
153. 6-chloro-N-[3-[[3-(6-fluoro-1,2-benzoxazol-3-yl)propylmethylamino]methyl]-phenyl]naphthalene-2-sulphonamide,
154. N-[3-[[3-(6-fluoro-1,2-benzoxazol-3-yl)propylmethylamino]methyl]phenyl]-2,3-dihydro-1,4-benzodioxino-6-sulphonamide,
155. N-[3-[[3-(6-fluoro-1,2-benzoxazol-3-yl)propylmethylamino]methyl]phenyl]-1,3-benzodioxol-5-sulphonamide,
156. N-[3-[[3-(6-fluoro-1,2-benzoxazol-3-yl)propylmethylamino]methyl]phenyl]-2-oxo-indoline-5-sulphonamide,
157. N-[3-[[3-(6-fluoro-1,2-benzoxazol-3-yl)propylmethylamino]methyl]phenyl]-6,7-dihydro-5H-pyrrolo[1,2-a]imidazole-3-sulphonamide,
158. N-[3-[[3-(6-fluoro-1,2-benzoxazol-3-yl)propylmethylamino]methyl]phenyl]-1-methylindole-4-sulphonamide,
159. N-[3-[[3-(6-fluoro-1,2-benzoxazol-3-yl)propylmethylamino]methyl]phenyl]-1-methylindole-5-sulphonamide,
160. N-[3-[[3-(6-fluoro-1,2-benzoxazol-3-yl)propylmethylamino]methyl]phenyl]-1,3-benzothiazole-4-sulphonamide,
161. N-[3-[[3-(6-fluoro-1,2-benzoxazol-3-yl)propylmethylamino]methyl]phenyl]-1,3-benzothiazole-5-sulphonamide,
162. N-[3-[[3-(6-fluoro-1,2-benzoxazol-3-yl)propylmethylamino]methyl]phenyl]-benzofuran-2-sulphonamide,
163. N-[3-[[3-(6-fluoro-1,2-benzoxazol-3-yl)propylmethylamino]methyl]phenyl]-benzothiophene-3-sulphonamide,
164. 5-fluoro-N-[3-[[3-(6-fluoro-1,2-benzoxazol-3-yl)propylmethylamino]methyl]-phenyl]-3-methylbenzothiophene-2-sulphonamide,
165. 5-chloro-N-[3-[[3-(6-fluoro-1,2-benzoxazol-3-yl)propylmethylamino]methyl]-phenyl]-3-methyl-benzothiophene-2-sulphonamide,
166. 3-chloro-4-fluoro-N-[3-[[3-(6-fluoro-1,2-benzoxazol-3-yl)propylmethylamino]-methyl]phenyl]benzenesulphonamide,
167. 4-cyano-N-[3-[[3-(6-fluoro-1,2-benzoxazol-3-yl)propylmethylamino]methyl]-phenyl]benzenesulphonamide,
168. N-[3-[[3-(6-fluoro-1,2-benzoxazol-3-yl)propylmethylamino]methyl]-phenyl]-benzothiophene-2-sulphonamide,
169. 6-chloro-N-[3-[[3-(6-fluoro-1,2-benzoxazol-3-yl)propylmethylamino]methyl]-phenyl]benzothiophene-2-sulphonamide,
170. N-[3-[[3-(6-fluoro-1,2-benzoxazol-3-yl)propylmethylamino]methyl]phenyl]-5-methyl-benzothiophene-2-sulphonamide
and pharmaceutically acceptable salts and solvates thereof.

Sulphonamide derivatives of benzylamine of the above formula (I) exhibit affinity to receptors which are recognized therapeutical targets in the treatment of CNS disorders, such as dopaminergic, in particular D2 and D3, serotoninergic, in particular 5-HT1A, 5-HT2A, 5-HT6, 5-HT7, and adrenergic, in particular α2C and have low affinity for biological targets associated with adverse effects, such as potassium channel hERG, muscarinic receptors M3, histaminergic receptors H1, adrenergic receptors α1 and serotoninergic receptors 5-HT2C. Due to such pharmacological profile, the compounds of the invention may be useful in medicine as medicaments, for the treatment and/or prevention of the central nervous system disorders such as schizophrenia, schizoaffective disorders, schizophreniform disorders, delusional syndromes and other psychotic conditions related and not related to taking psychoactive substances, affective disorder, bipolar disorder, mania, depression, anxiety disorders of various etiology, stress reactions, conciousness disorders, coma, delirium of alcoholic or other etiology, aggression, psychomotor agitation and other conduct disorders, sleep disorders of various etiology, withdrawal syndromes of various etiology, addiction, pain syndromes of various etiology, intoxication with psychoactive substances, cerebral circulatory disorders of various etiology, psychosomatic disorders of various etiology, conversion disorders, dissociative disorders, urination disorders, autism and other developmental disorders, including nocturia, stuttering, tics, cognitive disorders of various types, such as Alzheimer's disease, psychopatological symptoms and neurological disorders in the course of other diseases of the central and peripheral nervous systems.
Thus, the subject of the present invention are also the compounds of formula (I) as defined above, for use as a medicament.

In the treatment of central nervous system disorders compounds of formula (I) may be administered in the form of a pharmaceutical composition or preparation containing it. Thus, the subject of the present invention is also the pharmaceutical composition containing the compound or compounds of formula (I) as defined above, as an active substance, in combination with pharmaceutically acceptable carrier(s) and/or excipient(s).

The subject of the invention are compounds of formula (I) as defined above, for the treatment of disorders of central nervous system, in particular of disorders as mentioned above.

The method for the treatment of disorders of the central nervous system in mammals, including humans, comprises administration of a therapeutically effective amount of the compound of above formula (I) or the pharmaceutical composition containing the compound of formula (I) as defined above, as an active substance.

Terms used in the description of the present invention have the following meanings. Unless otherwise specified, the term "C₁-C₃-alkyl" relates to a saturated, straight or branched hydrocarbon group, having indicated number of carbon atoms. Specific examples of groups encompassed by this term are methyl, ethyl, n-propyl, isopropyl. The term "C₁-C₄-alkyl" relates to a saturated, straight or branched hydrocarbon group, having indicated number of carbon atoms. Specific examples of groups encompassed by this term are methyl, ethyl, n-propyl, isopropyl, n-butyl, tert-butyl, and sec-butyl.

The term "C₁-C₃-alkyloxy" relates to -O-C₁-C₃-alkyl group, wherein C₁-C₃-alkyl relates to a saturated, straight or branched hydrocarbon group, having indicated number of carbon atoms. Specific examples of groups encompassed by this term are methoxy, ethoxy, n-propoxy, and isopropoxy.

The term "halogen atom" relates to a substituent selected from F, Cl, Br and I.

The term "halogeno-C₁-C₃-alkyl" relates to a saturated, straight or branched hydrocarbon group, having indicated number of carbon atoms and in which one carbon atom may be substituted with from 1 to 3 halogen atoms, i.e. 1, 2 or 3 halogen atoms, depending on the number of carbon atoms bonded to it. Halogen atom has the meaning as defined above. Particularly preferred example of a group encompassed by this term is trifluoromethyl group -CF₃.

The term "halogeno- C₁-C₃-alkyloxy" relates to -O-C₁-C₃-halogenoalkyl group, wherein C₁-C₃-halogenoalkyl relates to a saturated, straight or branched hydrocarbon group, having indicated number of carbon atoms and in which one carbon atom may be substituted with from 1 to 3 halogen atoms, i.e. 1, 2 or 3 halogen atoms, depending on the number of carbon atoms bonded to it. Halogen atom has the meaning as defined above. Particularly preferred example of a group encompassed by his term is trifluoromethoxy group -O-CF₃. Depending on the number of carbon atoms that are attached to the nitrogen atom of amine group of benzylamine, the compounds of formula (I) can be prepared using one of following methods.

The compounds of formula (I), wherein R represents H, i.e. the compounds represented by the general formula (IA) can be prepared in a process presented in the following scheme:

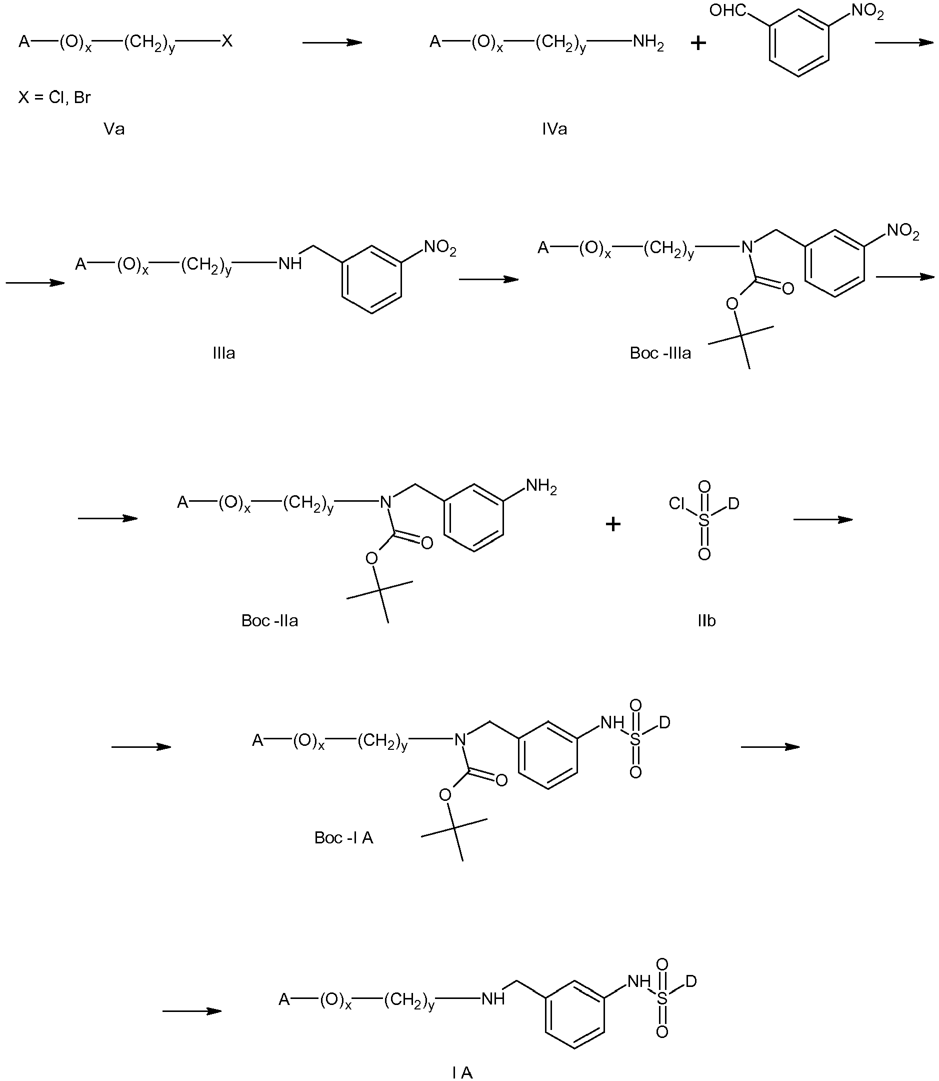

In the first step an appropriate halogen derivative (Va) is converted into a primary amine (IVa) in the presence of ammonia solution. Amine (IVa) and 3-nitrobenzaldehyde are then subjected to reductive amination in the presence of sodium triacetoxyborohydride as reducing agent, in a solvent, for example in methylene chloride. The next step consists in protection of amine group of nitroderivative (IIIa) using di-tert-butyl dicarbonate in the presence of triethylamine. Protected nitroderivative (Boc-IIIa) thus prepared is subjected to a reduction with hydrogen in the presence of Raney nickel as catalyst, to obtain a derivative (Boc-IIa), which is then reacted with sulphonyl chloride (IIb) in the presence of a base, for example pyridine, to obtain a protected compound (Boc-IA). Derivative (Boc-IA) is deprotected using trifluoroacetic acid in an organic solvent, for example in methylene chloride, to obtain the compound (IA) of the invention.

The compounds of formula (I), wherein R represents methyl, i.e. the compounds represented by the general formula (IB) can be prepared in a process presented in the following scheme:

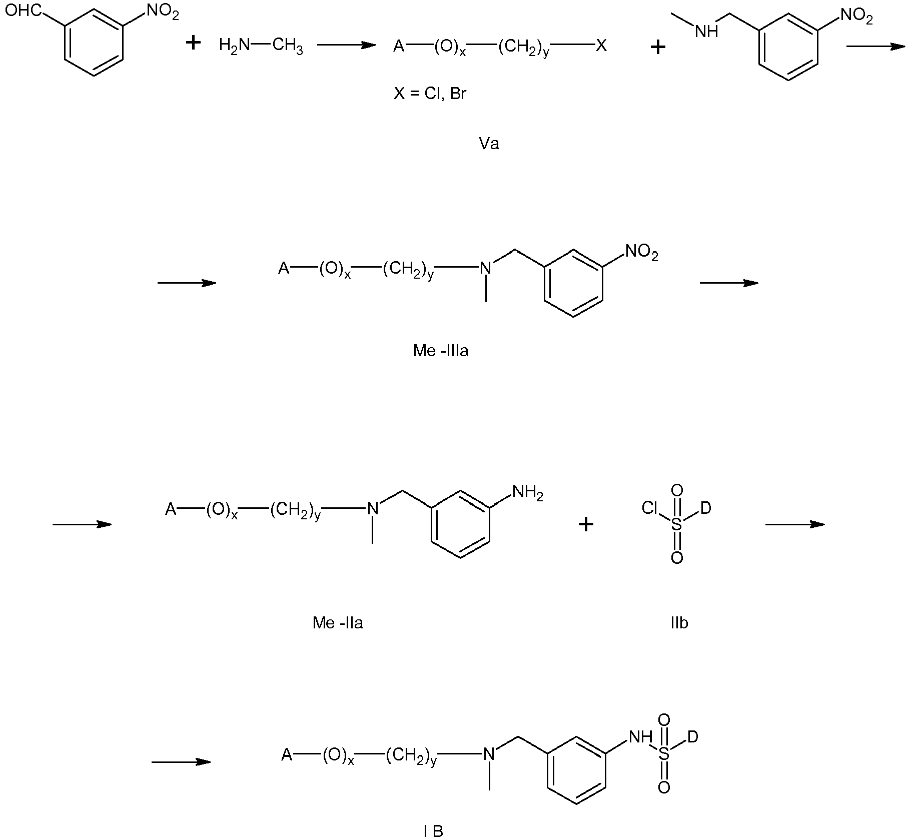

In the first step 3-nitrobenzaldehyde and methylamine are subjected to reductive amination in the presence of sodium borohydride as reducing agent. N-Methylbenzylamine derivative thus prepared is reacted with halogen derivative (Va) in the presence of a base, for example potassium carbonate, to obtain a nitroderivative (Me-IIIa). In the next step nitro group of derivative (Me-IIIa) is subjected to reduction using stannous chloride to obtain amine derivative (Me-IIa), which is then converted into a compound of the invention (IB) in a reaction with sulphonyl chloride (IIb) in the presence of a base, for example pyridine.

Starting compounds of formulas (Va), (IIb) and 3-nitrobenzaldehyde are either well known or commercially available, or can be prepared from commercially available starting materials by adapting and applying known methods.

Preparation of exemplary compounds of formula (I) of the invention and of exemplary intermediates (IVa), (IIIa), (Boc-IIIa), (Boc-IIa), (Boc-Ia), (Me-IIIa) and (Me-IIa) is described in detail in the experimental part.

Since the compounds of formula (I) are of alkaline character (contain one secondary or tertiary amine group), they can form acid addition salts.

Salts with acids can be pharmaceutically acceptable, especially when they are intended to be an active ingredient in pharmaceutical composition. The present invention relates also to salts of the compounds of formula (I) with acids other than pharmaceutically acceptable ones, which may be useful for example as intermediates suitable for purification of the compounds of the invention. In practice, it is often desirable to isolate first the compound from a reaction mixture in the form of a salt which may be not pharmaceutically acceptable to purify the compound, and then to convert the salt into free base by treatment with alkaline agent and to isolate, and optionally to convert into the pharmaceutically acceptable salt again.

Acid addition salts can be formed with inorganic (mineral) or organic acids. In particular, hydrochloric, hydrobromic, hydroiodic, phosphoric, sulphuric, nitric, carbonic, succinic, maleic, formic, acetic, propionic, fumaric, citric, tartaric, lactic, benzoic, salicylic, glutamic, aspargic, p-toluenesulphonic, benzenesulphonic, methanesulphonic, ethanesulphonic, naphthalenesulphonic such as 2-naphthalene-sulphonic, pamoic, xinafoic or hexanoic acids can be mentioned as examples of acids.

Acid addition salt can be prepared in a simple manner by reaction of the compound of formula (I) with suitable inorganic or organic acid, in an essentially equimolar amount compared to compound (I), optionally in suitable solvent, such as organic solvent, to form a salt that is usually isolated, for example by crystallization and filtration. For example, compounds in the form of a free base can be converted into corresponding hydrochloride salts by reaction of a compound in a solution, for example in methanol, with stoichiometric amount of hydrochloric acid or with solution of hydrochloric acid in methanol, ethanol or diethyl ether, followed by evaporation of solvent(s).

The term "disorders of the central nervous system" should be understood as including disorders selected from schizophrenia, schizoaffective disorders, schizophreniform disorders, delusional syndromes and other psychotic conditions related and not related to taking psychoactive substances, affective disorder, bipolar disorder, mania, depression, anxiety disorders of various etiology, stress reactions, conciousness disorders, coma, delirium of alcoholic and other etiology, aggression, psychomotor agitation and other conduct disorders, sleep disorders of various etiology, withdrawal syndromes of various etiology, addiction, pain syndromes of various etiology, intoxication with psychoactive substances, cerebral circulatory disorders of various etiology, psychosomatic disorders of various etiology, conversion disorders, dissociative disorders, urination disorders, autism and other developmental disorders, including nocturia, stuttering, tics, cognitive disorders of various types, like Alzheimer's disease, psychopathological symptoms and neurological disorders in the course of other diseases of the central and peripheral nervous systems.

In the treatment of the disorders mentioned above, compounds of formula (I) of the present invention can be administered as a chemical compound, but usually will be applied in the form of a pharmaceutical compositions containing the compound of the present invention or its pharmaceutically acceptable salt as defined above as an active ingredient in combination with pharmaceutically acceptable carrier(s) and/or excipient(s).

In the treatment of the above mentioned disorders the pharmaceutical compositions of the invention can be delivered by any route of administration, preferably oral or parenteral, and can have the form of a preparation for use in medicine, depending on the intended route of administration.

Compositions for oral administration may have the form of solid or liquid preparations. Solid preparations may be in the form, for example, tablets or capsules prepared in conventional manner using pharmaceutically acceptable inactive ingredients, such as binding agents (e.g. pregelatinized maize starch, polyvinylpyrrolidone or hydroxypropylmethylcellulose); fillers (e.g. lactose, sucrose, carboxymethylcellulose, microcrystalline cellulose or calcium hydrogen phosphate) lubricants (e.g. magnesium stearate, talc or silica); disintegrants (e.g. crospovidone, maize starch or sodium starch glycolate); wetting agents (e.g. sodium lauryl sulfate). The tablets may be coated using methods well known in the art with conventional coatings, delaying/controlling release coatings or enteric coatings. Liquid preparations for oral administration may have the form of e.g. solutions, syrups or suspensions, or may be prepared from a dry product suitable for reconstitution with water or other suitable carrier *ex tempore.* Such liquid preparations may be prepared by conventional methods with pharmaceutically acceptable inactive ingredients, such as suspending agents (e.g. sorbitol syrup, cellulose derivatives or hydrogenated edible fats), emulsifying agents (e.g. lecithin or acacia gum), non-aqueous matrix components (e.g. almond oil, oils esters, ethyl alcohol or fractionated vegetable oils) and preservatives (e.g. methyl or propyl p-hydroxybenzoates or sorbic acid). The preparations may also contain suitable buffering systems, flavouring and aroma agents, colourants and sweeteners.

Preparations for oral administration can be formulated according to methods well known to those skilled in the art to afford a controlled release of the active compound.

The parenteral route of administration comprises administration by intramuscular and intravenous injections and intravenous continuous infusions. Compositions for parenteral administration may be in the form of a dosage unit, e.g. in ampoules or in multidose containers with the addition of a preservative. The compositions may be in the form of suspensions, solutions or emulsions in oily or aqueous media, and may contain pharmaceutically acceptable excipients, such as suspending agents, stabilizing and/or dispersing agents. Alternatively, the active ingredient may be in the form of a powder for reconstitution *ex tempore* in a suitable carrier, e.g. sterile pyrogen-free water.

Method of treatment using compounds of this invention will be based on administration of a therapeutically effective amount of the compound of the invention, preferably in the form of a pharmaceutical composition, to a subject in need of such a treatment.

The proposed dose of the compounds of the invention will be comprised in the range from 1 to about 1000 mg per day, in a single dose or in divided doses. It will be apparent to those skilled in the art that selection of a dose required to achieve the desired biological effect will depend on several factors, such as the type of specific compound, the indication, route of administration, age and condition of a patient and the exact dose will be finally determined at the discretion of attending physician.

### Example 1.

### Preparation of starting materials of general formula (Boc-IIa) - general procedure

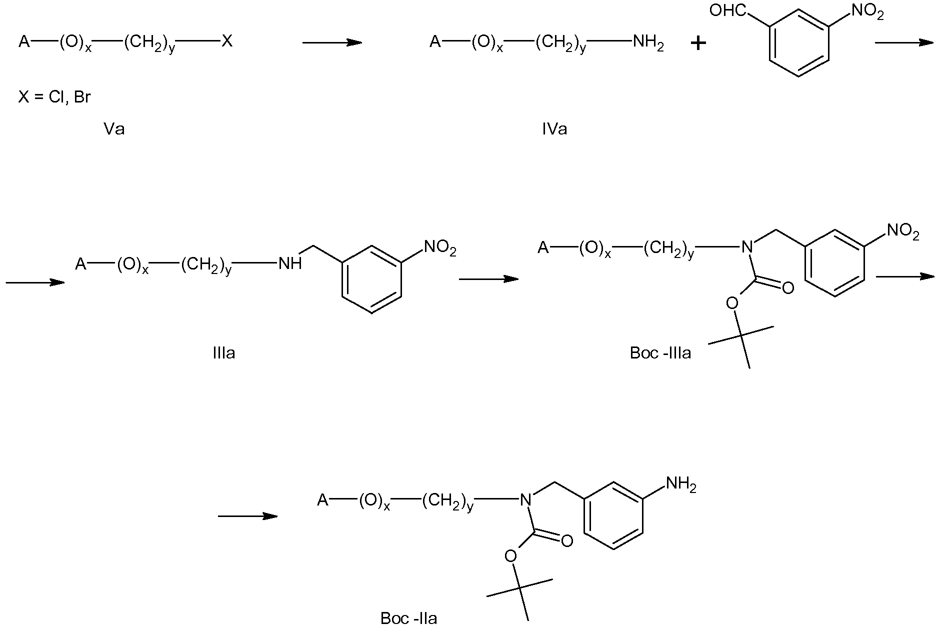

### a) Preparation of starting materials of formula (IVa)

An appropriate halogen derivative (Va) (17 mmol) is dissolved in metanol (200 ml). To the solution an excess of NH₃aq (2 × 250 ml of 25% solution) is added and the resulting mixture is stirred at room temperature for 2 days. Then methanol is evaporated under reduced pressure and the residue is partitioned between methylene chloride and 2N NaOH aq. The organic layer is dried over anhydrous magnesium sulphate, and after filtering the drying agent off, it is evaporated to dryness under reduced pressure to obtain a product of purity of above 85% (UPLC/MS) with a yield in a range of 85-98%.

Starting halogen derivatives (Va):
(2-bromoethyl)benzene (Va-1),
(2-bromoethoxy)benzene (Va-2),
1-(2-bromoethoxy)-3-fluorobenzene (Va-3),
1-(3-bromopropoxy)-3-fluorobenzene (Va-4),
1-(2-bromoethyl)-3-chlorobenzene (Va-5),
1-(2-bromoethoxy)-3-chlorobenzene (Va-6),
1-(2-bromoethyl)-3-methylbenzene (Va-7),
1-(2-bromoethoxy)-3-methylbenzene (Va-8),
1-(2-bromoethyl)-2-methoxybenzene (Va-9),
1-(2-bromoethoxy)-2-methoxybenzene (Va-10),
1-(2-bromoethyl)-3-methoxybenzene (Va-11),
1-(2-bromoethoxy)-3-methoxybenzene (Va-12),
1-(2-bromoethyl)-4-methoxybenzene (Va-13),
1-(2-bromoethoxy)-4-methoxybenzene (Va-14),
3-(2-bromoethyl)phenol (Va-15),
3-(2-bromoethoxy)phenol (Va-16),
5-(2-bromoethoxy)-2,3-dihydro-1,4-benzodioxin (Va-18),
7-(2-bromoethoxy)-2,2-dimethyl-2,3-dihydro-1-benzofuran (Va-19),
4-(2-bromoethoxy)-1 H-indole (Va-20),
3-(3-chloropropyl)-6-fluoro-1,2-benzoxazole (Va-21),
(3-bromopropyl)benzene (Va-22),
1-(3-bromopropyl)-4-methoxybenzene (Va-23).

According to the above general procedure the following compounds were prepared:
2-phenylethylamine (IVa-1),
2-phenoxyethylamine (IVa-2)
2-(3-fluorophenoxy)ethylamine (IVa-3),
3-(3-fluorophenoxy)propyl-1-amine (IVa-4),
2-(3-chlorophenyl)ethylamine (IVa-5),
2-(3-chlorophenoxy)ethylamine (IVa-6),
2-(3-methylphenyl)ethylamine (IVa-7),
2-(3-methylphenoxy)ethylamine (IVa-8),
2-(2-methoxyphenyl)ethylamine (IVa-9),
2-(2-methoxyphenoxy)ethylamine (IVa-10),
2-(3-methoxyphenyl)ethylamine (IVa-11),
2-(3-methoxyphenoxy)ethylamine (IVa-12),
2-(4-methoxyphenyl)ethylamine (IVa-13),
2-(4-methoxyphenoxy)ethylamine (IVa-14),
3-(2-aminoethyl)phenol (IVa-15),
3-(2-aminoethoxy)phenol (IVa-16),
2-(2,3-dihydro-1,4-benzodioxin-5-yloxy)ethylamine (IVa-18),
2-[(2,2-dimethyl-2,3-dihydro-1-benzofuran-7-yl)oxy]ethylamine (IVa-19),
2-(1 *H*-indol-4-yloxy)ethylamine (IVa-21),
3-(6-fluoro-1,2-benzoxazol-3-yl)propyl-1-amine (IVa-21),
3-phenylpropylamine (IVa-22),
2-(4-methoxyphenyl)propylamine (IVa-23).

### b) Preparation of starting materials of formula (IIIa)

3-Nitrobenzaldehyde (20 mmol) and the appropriate amine (IVa) are dissolved in methylene chloride (200 ml). Then sodium triacetoxyborohydride (37 mmol) is added an the mixture is stirred for 4 h, followed by pouring it onto a mixture of NaHCO₃ with ice. Once separated, the organic layer is washed with brine and water, and then dried over magnesium sulphate. After filtering the drying agent off, the filtrate is evaporated to dryness under reduced pressure to obtain a product of purity of above 90% (UPLC/MS). Yield: 75-84%.

According to the above general procedure the following compounds were prepared:
*N*-(3-nitrobenzyl)-2-phenylethylamine (IIIa-1),
*N*-(3-nitrobenzyl)-2-phenoxyethylamine (IIIa-2),
2-(3-fluorophenoxy)-*N*-(3-nitrobenzyl)ethylamine (IIIa-3),
3-(3-fluorophenoxy)-*N*-(3-nitrobenzyl)propyl-1-amine (IIIa-4),
2-(3-chlorophenyl)-*N*-(3-nitrobenzyl)ethylamine (IIIa-5),
2-(3-chlorophenoxy)-*N*-(3-nitrobenzyl)ethylamine (IIIa-6),
2-(3-methylphenyl)-*N*-(3-nitrobenzyl)ethylamine (IIIa-7),
2-(3-methylphenoxy)-*N*-(3-nitrobenzyl)ethylamine (IIIa-8),
2-(2-methoxyphenyl)-*N*-(3-nitrobenzyl)ethylamine (IIIa-9),
2-(2-methoxyphenoxy)-*N*-(3-nitrobenzyl)ethylamine (IIIa-10),
2-(3-methoxyphenyl)-*N*-(3-nitrobenzyl)ethylamine (IIIa-11),
2-(3-methoxyphenoxy)-*N*-(3-nitrobenzyl)ethylamine (IIIa-12),
2-(4-methoxyphenyl)-*N*-(3-nitrobenzyl)ethylamine (IIIa-13),
2-(4-methoxyphenoxy)-*N*-(3-nitrobenzyl)ethylamine (IIIa-14),
3-{2-[(3-nitrobenzyl)amino]ethyl}phenol (IIIa-15),
3-{2-[(3-nitrobenzyl)amino]ethoxy}phenol (IIIa-16),
2-(2,3-dihydro-1,4-benzodioxin-5-yloxy)-*N*-(3-nitrobenzyl)ethylamine (IIIa-18),
2-[(2,2-dimethyl-2,3-dihydro-1-benzofuran-7-yl)oxy]-*N*-(3-nitrobenzyl)methylamine (IIIa-19),
2-(1*H*-indol-4-yloxy)-*N*-(3-nitrobenzyl)ethylamine (IIIa-20),
3-(6-fluoro-1,2-benzoxazol-3-yl)-*N*-(3-nitrobenzyl)propyl-1-amine (IIIa-21),
*N*-(3-nitrobenzyl)-3-phenylpropylamine (IIIa-22),
3-(4-methoxyphenyl)-*N*-(3-nitrobenzyl)propylamine (IIIa-23).

### c) Preparation of starting materials of formula (Boc-IIIa) - general procedure

An appropriate nitroderivative (IIIa) (11 mmol) is dissolved in methylene chloride (100 ml), then to the resulting solution triethylamine (5 ml) followed by di-tert-butyl dicarbonate (12,7 mmol) are added. The mixture is stirred for 1 h, and then washed with 1 N NaOH_{aq}, 1 N HCl_{aq} and brine. The organic layer is dried over magnesium sulphate. After filtering the drying agent off, the filtrate is evaporated to dryness to obtain a product of purity of above 95% (UPLC/MS) with yield of 86-95%.

According to the above general procedure the following compounds were prepared:
tert-butyl (3-nitrobenzyl)(2-phenylethyl)carbamate (Boc-IIIa-1),
tert-butyl (3-nitrobenzyl)(2-phenoxyethyl)carbamate (Boc-IIIa-2)
*tert*-butyl [2-(3-fluorophenoxy)ethyl](3-nitrobenzyl)carbamate (Boc-IIIa-2),
*tert*-butyl [3-(3-fluorophenyl)propyl](3-nitrobenzyl)carbamate (Boc-IIIa-4),
*tert*-butyl [2-(3-chlorophenyl)ethyl](3-nitrobenzyl)carbamate (Boc-IIIa-5),
*tert*-butyl [2-(3-chlorophenoxy)ethyl](3-nitrobenzyl)carbamate (Boc-IIIa-6),
*tert*-butyl [2-(3-methylphenyl)ethyl](3-nitrobenzyl)carbamate (Boc-IIIa-7),
*tert*-butyl [2-(3-methylphenoxy)ethyl](3-nitrobenzyl)carbamate (Boc-IIIa-8)
*tert*-butyl [2-(2-methoxyphenyl)ethyl](3-nitrobenzyl)carbamate (Boc-IIIa-9),
*tert*-butyl [2-(2-methoxyphenoxy)ethyl](3-nitrobenzyl)carbamate (Boc-IIIa-10),
*tert*-butyl [2-(3-methoxyphenyl)ethyl](3-nitrobenzyl)carbamate (Boc-IIIa-11 ),
*tert*-butyl [2-(3-methoxyphenoxy)ethyl](3-nitrobenzyl)carbamate (Boc-IIIa-12),
*tert*-butyl [2-(4-methoxyphenyl)ethyl](3-nitrobenzyl)carbamate (Boc-IIIa-13),
*tert*-butyl [2-(4-methoxyphenoxy)ethyl](3-nitrobenzyl)carbamate (Boc-IIIa-14),
*tert*-butyl [2-(3-hydroxyphenyl)ethyl](3-nitrobenzyl)carbamate (Boc-IIIa-15),
*tert*-butyl [2-(3-hydroxyphenoxy)ethyl](3-nitrobenzyl)carbamate (Boc-IIIa-16),
*tert*-butyl [2-(2,3-dihydro-1,4-benzodioxin-5-yloxy)ethyl](3-nitrobenzyl)carbamate (Boc-IIIa-18),
*tert*-butyl [{2-[(2,2-dimethyl-2,3-dihydro-1-benzofuran-7-yl)oxy]ethyl}(3-nitrobenzyl)-carbamate (Boc-IIIa-19),
*tert*-butyl [2-(1*H*-indol-4-yloxy)ethyl](3-nitrobenzyl)carbamate (IIIa-20),
*tert*-butyl [3-(6-fluoro-1,2-benzoxazol-3-yl)propyl](3-nitrobenzyl)carbamate (IIIa-21),
*tert*-butyl (3-nitrobenzyl)(3-phenylpropyl)carbamate (Boc-IIIa-22),
*tert*-butyl [3-(4-methoxyphenyl)propyl](3-nitrobenzyl)carbamate (Boc-IIIa-23).

### d) Preparation of starting materials of formula (Boc-IIa) - general procedure

Appropriate derivative of formula (Boc-IIIa) (8 mmol) is dissolved in methanol (50 ml) and subjected to reduction with hydrogen under atmospheric pressure (reaction vessel is equipped with H₂-filled balloon) in the presence of Raney nickel as catalyst. Following 18 h stirring at room temperature the catalyst is filtered off, the filtrat is evaporated to dryness and the crude product of purity of above 90% (UPLC/MS) is used in the next step without purification. Yield: 95-99%.

According to the above general procedure the following compounds were prepared:
*tert*-butyl (3-aminobenzyl)(2-phenylethyl)carbamate (Boc-IIa-1),
*tert*-butyl (3-aminobenzyl)(2-phenoxyethyl)carbamate (Boc-IIa-2),
*tert*-butyl (3-aminobenzyl)[2-(3-fluorophenoxy)ethyl]carbamate (Boc-IIa-3),
*tert*-butyl (3-aminobenzyl)[3-(3-fluorophenyl)propyl]carbamate (Boc-IIa-4),
*tert*-butyl (3-aminobenzyl)[2-(3-chlorophenyl)ethyl]carbamate (Boc-IIa-5),
*tert*-butyl (3-aminobenzyl)[2-(3-chlorophenoxy)ethyl]carbamate (Boc-IIa-6),
*tert*-butyl (3-aminobenzyl)[2-(3-methylphenyl)ethyl]carbamate (Boc-IIa-7),
*tert*-butyl (3-aminobenzyl)[2-(3-methylphenoxy)ethyl]carbamate (Boc-IIa-8),
*tert*-butyl (3-aminobenzyl)[2-(2-methoxyphenyl)ethyl]carbamate (Boc-IIa-9),
*tert*-butyl (3-aminobenzyl)[2-(2-methoxyphenoxy)ethyl]carbamate (Boc-IIa-10),
*tert*-butyl (3-aminobenzyl)[2-(3-methoxyphenyl)ethyl]carbamate (Boc-IIa-11 ),
*tert*-butyl (3-aminobenzyl)[2-(3-methoxyphenoxy)ethyl]carbamate (Boc-IIa-12),
*tert*-butyl (3-aminobenzyl)[2-(4-methoxyphenyl)ethyl]carbamate (Boc-IIa-13),
*tert*-butyl (3-aminobenzyl)[2-(4-methoxyphenoxy)ethyl]carbamate (Boc-IIa-14),
*tert*-butyl (3-aminobenzyl)[2-(3-hydroxyphenyl)ethyl]carbamate (Boc-IIa-15),
*tert*-butyl (3-aminobenzyl)[2-(3-hydroxyphenoxy)ethyl]carbamate (Boc-IIa-16),
*tert*-butyl (3-aminobenzyl)[2-(2,3-dihydro-1,4-benzodioxin-5-yloxy)ethyl]carbamate (Boc-IIa-18)
*tert*-butyl (3-aminobenzyl){2-[(2,2-dimethyl-2,3-dihydro-1-benzofuran-7-yl)oxy]ethyl}-carbamate (Boc-IIa-19),
*tert*-butyl (3-aminobenzyl)[2-(1H-indol-4-yloxy)ethyl]carbamate (Boc-IIa-20),
*tert*-butyl (3-aminobenzyl)[3-(6-fluoro-1,2-benzoxazol-3-yl)propyl]carbamate (Boc-IIa-21),
*tert*-butyl (3-aminobenzyl)(3-phenylpropyl)carbamate (Boc-IIa-22),
*tert*-butyl (3-aminobenzyl)[3-(4-methoxyphenyl)propyl]carbamate (Boc-IIa-23).

### Example 2.

### General procedure for the preparation of compounds (IA) according to the invention

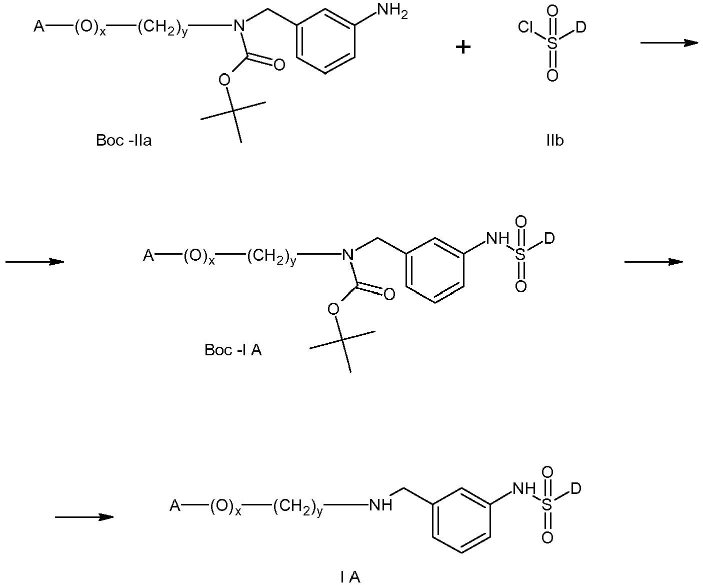

The compound of formula (Boc-IIa) (0,75 mmol), obtained as described in example 1, is dissolved in dry methylene chloride (10 ml). To the resulting solution pyridine (1 ml) and an appropriate sulphonyl chloride (IIb) (0,75 mmol) are added. The mixture is stirred overnight at room temperature. Then pyridine is removed by co-evaporation with toluene, and the residue is partitioned between water and ethyl acetate. The organic layer is dried over magnesium sulphate. After filtering the drying agent off, the filtrate is evaporated to dryness to obtain a crude product (Boc-IA) as an oil. Crude protected sulphonamide (Boc-IA) is dissolved in methylene chloride (10 ml) and trifluoroacetic acid is added (5 ml). The mixture is stirred for 1 h at room temperature, followed by evaporation of solvents under reduced pressure. The residue is partitioned between ethyl acetate and 2N NaOH aq. The organic layer is washed with water, then with brine and dried over magnesium sulphate. After filtering the drying agent off, the filtrate is evaporated to dryness. The residue is purified by means of "flash"-chromatography (methylene chloride/ methanol 9:1 v/v).

The yield of compounds (IA) was in the range of 65-90%, and HPLC purity in the range of 90-100%.

Structure of obtained compounds was confirmed by MS analysis and/or ¹H NMR.

As starting materials for the preparation of compounds (IA) of the invention the compounds of formula (Boc-IIa) as described in Example 1d) and commercially available sulphonyl chlorides (IIb) were used:
benzenesulphonyl chloride (IIb-1 ),
3-fluorobenzenesulphonyl chloride (IIb-2),
4-fluorobenzenesulphonyl chloride (IIb-3),
3,4-difluorobenzenesulphonyl chloride (IIb-4),
3-chlorobenzenesulphonyl chloride (IIb-5),
4-chlorobenzenesulphonyl chloride (IIb-6),
3,4-dichlorobenzenesulphonyl chloride (IIb-7)
3-bromobenzenesulphonyl chloride (IIb-8),
4-bromobenzenesulphonyl chloride (IIb-9),
4-iodobenzenesulphonyl chloride (IIb-10),
3-chloro-4-fluorobenzenesulphonyl chloride (IIb-48),
3-methylbenzenesulphonyl chloride (IIb-11),
4-methylbenzenesulphonyl chloride (IIb-12),
4-propylbenzenesulphonyl chloride (IIb-13),
4-tert-butylbenzenesulphonyl chloride (IIb-14),
3-(trifluoromethyl)benzenesulphonyl chloride (IIb-15),
4-(trifluoromethyl)benzenesulphonyl chloride (IIb-16),
4-fluoro-3-methylbenzenesulphonyl chloride (IIb-17),
3-methoxybenzenesulphonyl chloride (IIb-18),
4-methoxybenzenesulphonyl chloride (IIb-19),
4-(trifluoromethoxy)benzenesulphonyl chloride (IIb-20),
3-cyanobenzenesulphonyl chloride (IIb-21),
4-cyanobenzenesulphonyl chloride (IIb-22),
4-(phenyl)benzenesulphonyl chloride (IIb-23),
benzothiophene-2-sulphonyl chloride (IIb-24),
benzothiophene-3-sulphonyl chloride (IIb-25),
2,5-dimethylthiophene-2-sulphonyl chloride (IIb-26),
5-isoxazol-5-ylthiophene-2-sulphonyl chloride (IIb-27),
naphthalene-1-sulphonyl chloride (IIb-28),
naphthalene-2-sulphonyl chloride (IIb-29),
6-chloronaphthalene-2-sulphonyl chloride (IIb-30),
2,3-dihydro-1,4-benzodioxine-5-sulphonyl chloride (IIb-31),
2,3-dihydro-1,4-benzodioxine-6-sulphonyl chloride (IIb-32),
1,3-benzodioxole-5-sulphonyl chloride (IIb-33),
2-oxo-2,3-dihydro-1*H*-indole-5-sulphonyl chloride (IIb-34),
6,7-dihydro-5H-pyrrolo[1,2-a]imidazole-3-sulphonyl chloride (IIb-35),
1*H*-indole-5-sulphonyl chloride (IIb-36),
1-methyl-1*H*-indole-4-sulphonyl chloride (IIb-37),
1-methyl-1*H*-indole-5-sulphonyl chloride (IIb-38),
1,3-benzothiazole-4-sulphonyl chloride (IIb-39),
1,3-benzothiazole-5-sulphonyl chloride (IIb-40),
1-benzofuran-2-sulphonyl chloride (IIb-41),
1-benzothiophene-2-sulphonyl chloride (IIb-42),
1-benzothiophene-3-sulphonyl chloride (IIb-43),
6-chloro-1-benzothiophene-2-sulphonyl chloride (IIb-44),
5-methyl-1-benzothiophene-2-sulphonyl chloride (IIb-45),
5-fluoro-3-methyl-1-benzothiophene-2-sulphonyl chloride (IIb-46),
5-chloro-3-methyl-1-benzothiophene-2-sulphonyl chloride (IIb-47).

According to the above general procedure, the following compounds of formula (IA) according to the invention were prepared.

### Compound 1.

### N-[3-[(phenylethylamino)methyl]phenyl]naphthalene-2-sulphonamide

The title compound was prepared starting from the compound (Boc-IIa-1) and sulphonyl chloride (IIb-29). ¹H-NMR (300 MHz, CDCl₃): 8.34 (d, 1H, J = 1.8 Hz), 7.88-7.80 (m, 3H), 7.71 (dd, 1H, J = 1.8 and 8.7 Hz), 7.62-7.52 (m, 2H), 7.30-7.10 (m, 6H), 7.03-6.92 (m, 3H), 3.68 (s, 2H), 2.76-2.68 (m, 4H); MS: 417 [M+H⁺].

### Compound 2.

### N-[3-[(2-phenoxyethylamino)methyl]phenyl]naphthalene-2-sulphonamide

The title compound was prepared starting from the compound (Boc-IIa-2) and sulphonyl chloride (IIb-29). ¹H-NMR (300 MHz, CDCl₃): 8.32 (d, 1H, J = 1.8 Hz), 7.86-7.78 (m, 3H), 7.70 (dd, 1H, J = 1.8 and 8.7 Hz), 7.62-7.39 (m, 2H), 7.19-7.00 (m, 5H), 6.44-6.36 (m, 3H), 3.90 (t, 2H, J = 4.8 Hz), 3.55 (s, 2H), 2.80 (t, 2H, J = 4.8 Hz); MS: 449 [M+H⁺].

### Compound 3.

### N-[3-[(3-phenylpropylamino)methyl]phenyl]naphthalene-2-sulphonamide

The title compound was prepared starting from the compound (Boc-IIa-22) and sulphonyl chloride (IIb-29). MS: 431 [M+H⁺].

### Compound 4.

### N-[3-[[2-(3-fluorophenoxy)ethylamino]methyl]phenyl]naphthalene-2-sulphonamide

The title compound was prepared starting from the compound (Boc-IIa-3) and sulphonyl chloride (IIb-29). ¹H-NMR (300 MHz, CDCl₃): 8.21 (d, 1H, J = 1.8 Hz), 7.94-7.86 (m, 3H), 7.68-7.58 (m, 3H), 7.24-7.22 (m, 2H), 7.18-7.10 (m, 1H), 7.06-7.04 (m, 1H), 6.98-6.90 (m, 1 H), 6.66-6.58 (m, 1 H), 6.52 (dd, 1 H, J = 2.0 and 8.2 Hz), 6.42 (dt, 1 H, J = 2.4 and 11.0 Hz), 4.15-4.11 (m, 2H), 4.02-3.98 (m, 2H), 3.98 (s, 2H); MS: 451 [M+H⁺].

### Compound 5.

### N-[3-[[3-(3-fluorophenoxy)propylamino]methyl]phenyl]naphthalene-2-sulphonamide

The title compound was prepared starting from the compound (Boc-IIa-4) and sulphonyl chloride (IIb-29). ¹H-NMR (300 MHz, CDCl₃): 8.17 (d, 1H, J = 1.8 Hz), 7.92-7.84 (m, 3H), 7.68-7.54 (m, 3H), 7.25-7.20 (m, 2H), 7.19-7.12 (m, 1H), 7.06-7.02 (m, 1H), 6.92-6.88 (m, 1H), 6.65-6.54 (m, 2H), 6.48 (dt, 1 H, J = 2.3 and 10.7 Hz), 4.00-3.96 (m, 2H), 3.80-3.77 (m, 2H), 1.99-1.90 (m, 2H), 1.85-1.80 (m, 2H); MS: 465 [M+H⁺].

### Compound 6.

### N-[3-[[2-(3-chlorophenyl)ethylamino]methyl]phenyl]naphthalene-2-sulphonamide

The title compound was prepared starting from the compound (Boc-IIa-5) and sulphonyl chloride (IIb-29). ¹H-NMR (300 MHz, CDCl₃): 8.34 (d, 1H, J = 1.8 Hz), 7.89-7.80 (m, 3H), 7.70 (dd, 1H, J = 1.8 and 8.7 Hz), 7.63-7.52 (m, 2H), 7.20-7.10 (m, 4H), 7.04-6.98 (m, 4H), 3.62 (s, 2H), 2.78-2.64 (m, 4H); MS: 451 [M+H⁺].

### Compound 7.

### N-[3-[[2-(3-chlorophenoxy)ethylamino]methyl]phenyl]naphthalene-2-sulphonamide

The title compound was prepared starting from the compound (Boc-IIa-6) and sulphonyl chloride (IIb-29). ¹H-NMR (300 MHz, CDCl₃): 8.23 (d, 1H, J = 1.8 Hz), 7.92-7.84 (m, 3H), 7.64-7.52 (m, 3H), 7.40-7.34 (m, 2H), 7.16 (t, 1 H, J = 7.9 Hz), 7.04 (t, 1 H, J = 7.9 Hz), 6.90-6.80 (m, 2H), 6.64 (t, 1 H, J = 2.0 Hz), 6.54 (dd, 1 H, J = 1.8 and 8.7 Hz), 4.02-3.88 (m, 6H); MS: 467 [M+H⁺].

### Compound 8.

### N-[3-[[2-(3-methylphenyl)ethylamino]methyl]phenyl]naphthalene-2-sulphonamide

The title compound was prepared starting from the compound (Boc-IIa-7) and sulphonyl chloride (IIb-29). ¹H-NMR (300 MHz, CDCl₃): 8.31 (d, 1H, J = 1.8 Hz), 7.86-7.79 (m, 3H), 7.70 (dd, 1H, J = 1.8 and 8.7 Hz), 7.62-7.50 (m, 2H), 7.18-7.08 (m, 1H), 7.04-6.88 (m, 6H), 3.68 (s, 2H), 2.72-2.64 (m, 4H), 2.29 (s, 3H); MS: 431 [M+H⁺].

### Compound 9.

### N-[3-[[2-(2-methoxyphenyl)ethylamino]methyl]phenyl]naphthalene-2-sulphonamide

The title compound was prepared starting from the compound (Boc-IIa-9) and sulphonyl chloride (IIb-29). ¹H-NMR (300 MHz, CDCl₃): 8.33 (d, 1H, J = 1.8 Hz), 7.88-7.78 (m, 3H), 7.68 (dd, 1H, J = 2.0 and 8.7 Hz), 7.62-7.52 (m, 2H), 7.24-7.18 (m, 1H), 7.16-7.04 (m, 2H), 7.03-6.98 (m, 2H), 6.96-6.92 (m, 1 H), 6.90-6.82 (m, 2H), 3.78 (s, 3H), 3.76 (s, 2H), 2.90-2.87 (m, 4H); MS: 447 [M+H⁺].

### Compound 10.

### N-[3-[[2-(2-methoxyphenoxy)ethylamino]methyl]phenyl]naphthalene-2-sulphonamide

The title compound was prepared starting from the compound (Boc-IIa-10) and sulphonyl chloride (IIb-29). ¹H-NMR (300 MHz, CDCl₃): 8.20 (d, 1H, J = 1.8 Hz), 7.92-7.84 (m, 3H), 7.68-7.55 (m, 3H), 7.24-7.22 (m, 2H), 7.17-7.15 (m, 1H), 6.98-6.94 (m, 1H), 6.92-6.86 (m, 1H), 6.84-6.80 (m, 3H), 4.21-4.18 (m, 2H), 4.07-4.00 (m, 2H), 3.76 (s, 2H), 3.75 (s, 3H); MS: 463 [M+H⁺].

### Compound 11.

### N-[3-[[2-(3-methoxyphenyl)ethylamino]methyl]phenyl]naphthalene-2-sulphonamide

The title compound was prepared starting from the compound (Boc-IIa-11) and sulphonyl chloride (IIb-29). ¹H-NMR (300 MHz, CDCl₃): 8.32 (d, 1H, J = 1.8 Hz), 7.86-7.80 (m, 3H), 7.70 (dd, 1H, J = 1.8 and 8.7 Hz), 7.62-7.50 (m, 2H), 7.21-7.10 (m, 2H), 7.06-6.92 (m, 3H), 6.78-6.68 (m, 3H), 3.80 (s, 3H), 3.62 (s, 2H), 2.78-2.64 (m, 4H); MS: 447 [M+H⁺].

### Compound 12.

### N-[3-[[2-(2,3-dihydro-1,4-benzodioxin-5-yloxy)ethylamino]methyl]phenyl]-4-propyl-benzenesulphonamide

The title compound was prepared starting from the compound (Boc-IIa-18) and sulphonyl chloride (IIb-13). MS: 484 [M+H⁺].

### Compound 13.

### N-[3-[[2-(3-methoxyphenoxy)ethylamino]methyl]phenyl]naphthalene-2-sulphonamide

The title compound was prepared starting from the compound (Boc-IIa-12) and sulphonyl chloride (IIb-29). ¹H-NMR (300 MHz, CDCl₃): 8.20 (d, 1H, J = 1.8 Hz), 7.98-7.92 (m, 3H), 7.65-7.56 (m, 3H), 7.18-7.00 (m, 3H), 6.98-6.96 (m, 1H), 6.56-6.50 (m, 1H), 6.35-6.30 (m, 1H), 6.25-6.20 (m, 1H), 4.15-4.10 (m, 2H), 4.00-3.95 (m, 2H), 3.80-3.75 (m, 2H), 3.65 (s, 3H);
MS: 463 [M+H⁺].

### Compound 14.

### N-[3-[[2-(4-methoxyphenyl)ethylamino]methyl]phenyl]naphthalene-2-sulphonamide

The title compound was prepared starting from the compound (Boc-IIa-13) and sulphonyl chloride (IIb-29). ¹H-NMR (300 MHz, CDCl₃): 8.34 (d, 1H, J = 1.8 Hz), 7.86-7.80 (m, 3H), 7.50 (dd, 1H, J = 1.8 and 8.9 Hz), 7.62-7.51 (m, 2H), 7.12 (t, 1H, J = 8.2 Hz), 7.08-6.94 (m, 5H), 6.84-6.78 (m, 2H), 3.78 (s, 3H), 3.67 (s, 2H), 2.76-2.64 (m, 4H); MS: 447 [M+H⁺].

### Compound 15.

### N-[3-[[2-(4-methoxyphenoxy)ethylamino]methyl]phenyl]naphthalene-2-sulphonamide

The title compound was prepared starting from the compound (Boc-IIa-14) and sulphonyl chloride (IIb-29). ¹H-NMR (300 MHz, CDCl₃): 8.34 (d, 1H, J = 1.8 Hz), 7.88-7.80 (m, 3H), 7.71 (dd, 1H, J = 1.8 and 8.7 Hz), 7.62-7.50 (m, 2H), 7.20-7.14 (m, 1H), 7.07-7.00 (m, 3H), 6.84-6.76 (m, 4H), 3.86 (t, 2H, J = 4.8 Hz), 3.78 (s, 3H), 3.74 (s, 2H), 2.78 (t, 1 H, J = 4.8 Hz); MS: 463 [M+H⁺].

### Compound 16.

### N-[3-[[3-(4-methoxyphenyl)propylamino]methyl]phenyl]naphthalene-2-sulphonamide

The title compound was prepared starting from the compound (Boc-IIa-23) and sulphonyl chloride (IIb-29). MS: 461 [M+H⁺].

### Compound 17.

### N-[3-[[2-(3-hydroxyphenyl)ethylamino]methyl]phenyl]naphthalene-2-sulphonamide

The title compound was prepared starting from the compound (Boc-IIa-15) and sulphonyl chloride (IIb-29). ¹H-NMR (300 MHz, CD₃OD): 8.29 (d, 1H, J = 1.8 Hz), 7.88-7.82 (m, 3H), 7.72 (dd, 1H, J = 2.0 and 8.7 Hz), .62-7.50 (m, 2H), 7.18-7.10 (m, 1H), 7.06-7.00 (m, 3H), 6.98-6.92 (m, 1H), 6.66-6.61 (m, 1H), 6.60-6.55 (m, 2H), 3.65 (s, 2H), 2.70-2.60 (m, 2H); MS: 433 [M+H⁺].

### Compound 18.

### N-[3-[[2-(2,3-dihydro-1,4-benzodioxin-5-yloxy)ethylamino]methyl]phenyl]benzenesulphonamide

The title compound was prepared starting from the compound (Boc-IIa-18) and sulphonyl chloride (IIb-1). MS: 442 [M+H⁺].

### Compound 19.

### N-[3-[[2-(2,3-dihydro-1,4-benzodioxin-5-yloxy)ethylamino]methyl]phenyl]-3-fluoro-benzenesulphonamide

The title compound was prepared starting from the compound (Boc-IIa-18) and sulphonyl chloride (IIb-2). MS: 460 [M+H⁺].

### Compound 20.

### N-[3-[[2-(2,3-dihydro-1,4-benzodioxin-5-yloxy)ethylamino]methyl]phenyl]-4-fluoro-benzenesulphonamide

The title compound was prepared starting from the compound (Boc-IIa-18) and sulphonyl chloride (IIb-3). MS: 460 [M+H⁺].

### Compound 21.

### 4-chloro-N-[3-[[2-(2,3-dihydro-1,4-benzodioxin-5-yloxy)ethylamino]methyl]phenyl]-benzenesulphonamide

The title compound was prepared starting from the compound (Boc-IIa-18) and sulphonyl chloride (IIb-6). MS: 476 [M+H⁺].

### Compound 22.

### N-[3-[[2-(2,3-dihydro-1,4-benzodioxin-5-yloxy)ethylamino]methyl]phenyl]-4-iodo-benzenesulphonamide

The title compound was prepared starting from the compound (Boc-IIa-18) and sulphonyl chloride (IIb-10). MS: 567 [M+H⁺].

### Compound 23.

### N-[3-[[2-(2,3-dihydro-1,4-benzodioxin-5-yloxy)ethylamino]methyl]phenyl]-3-methyl-benzenesulphonamide

The title compound was prepared starting from the compound (Boc-IIa-18) and sulphonyl chloride (IIb-11). MS: 456 [M+H⁺].

### Compound 24.

### N-[3-[[2-(2,3-dihydro-1,4-benzodioxin-5-yloxy)ethylamino]methyl]phenyl]-3-(trifluoro-methyl)benzenesu lphonamide

The title compound was prepared starting from the compound (Boc-IIa-18) and sulphonyl chloride (IIb-15). MS: 510 [M+H⁺].

### Compound 25.

### N-[3-[[2-(2,3-dihydro-1,4-benzodioxin-5-yloxy)ethylamino]methyl]phenyl]-3-methoxy-benzenesulphonamide

The title compound was prepared starting from the compound (Boc-IIa-18) and sulphonyl chloride (IIb-18). MS: 472 [M+H⁺].

### Compound 26.

### N-[3-[[2-(2,3-dihydro-1,4-benzodioxin-5-yloxy)ethylamino]methyl]phenyl]-4-methoxy-benzenesulphonamide

The title compound was prepared starting from the compound (Boc-IIa-18) and sulphonyl chloride (IIb-19). MS: 472 [M+H⁺].

### Compound 27.

### N-[3-[[2-(2,3-dihydro-1,4-benzodioxin-5-yloxy)ethylamino]methyl]phenyl]-4-(trifluoro-methoxy)benzenesulphonamide

The title compound was prepared starting from the compound (Boc-IIa-18) and sulphonyl chloride (IIb-20). MS: 526 [M+H⁺].

### Compound 28.

### 3-cyano-N-[3-[[2-(2,3-dihydro-1,4-benzodioxin-5-yloxy)ethylamino]methyl]phenyl]-benzenesulphonamide

The title compound was prepared starting from the compound (Boc-IIa-18) and sulphonyl chloride (IIb-21). MS: 467 [M+H⁺].

### Compound 29.

### 4-cyano-N-[3-[[2-(2,3-dihydro-1,4-benzodioxin-5-yloxy)ethylamino]methyl]phenyl]-benzenesulphonamide

The title compound was prepared starting from the compound (Boc-IIa-18) and sulphonyl chloride (IIb-22). MS: 467 [M+H⁺].

### Compound 30.

### N-[3-[[2-(2,3-dihydro-1,4-benzodioxin-5-yloxy)ethylamino]methyl]phenyl]-4-phenyl-benzenesulphonamide

The title compound was prepared starting from the compound (Boc-IIa-18) and sulphonyl chloride (IIb-23). MS: 518 [M+H⁺].

### Compound 31.

### N-[3-[[2-(2,3-dihydro-1,4-benzodioxin-5-yloxy)ethylamino]methyl]phenyl]thiophene-2-sulphonamide

The title compound was prepared starting from the compound (Boc-IIa-18) and sulphonyl chloride (IIb-24). MS: 448 [M+H⁺].

### Compound 32.

### N-[3-[[2-(2,3-dihydro-1,4-benzodioxin-5-yloxy)ethylamino]methyl]phenyl]thiophene-3-sulphonamide

The title compound was prepared starting from the compound (Boc-IIa-18) and sulphonyl chloride (IIb-25). MS: 448 [M+H⁺].

### Compound 33.

### N-[3-[[2-(2,3-dihydro-1,4-benzodioxin-5-yloxy)ethylamino]methyl]phenyl]-2,5-dimethyl-thiophene-3-sulphonamide

The title compound was prepared starting from the compound (Boc-IIa-18) and sulphonyl chloride (IIb-26). MS: 476 [M+H⁺].

### Compound 34.

### N-[3-[[2-(2,3-dihydro-1,4-benzodioxin-5-yloxy)ethylamino]methyl]phenyl]-5-isoxazol-5-yl-thiophene-2-sulphonamide

The title compound was prepared starting from the compound (Boc-IIa-18) and sulphonyl chloride (IIb-27). MS: 515 [M+H⁺].

### Compound 35.

### N-[3-[[2-(2,3-dihydro-1,4-benzodioxin-5-yloxy)ethylamino]methyl]phenyl]naphthalene-1-sulphonamide

The title compound was prepared starting from the compound (Boc-IIa-18) and sulphonyl chloride (IIb-28). MS: 492 [M+H⁺].

### Compound 36.

### N-[3-[[2-(2,3-dihydro-1,4-benzodioxin-5-yloxy)ethylamino]methyl]phenyl]naphthalene-2-sulphonamide

The title compound was prepared starting from the compound (Boc-IIa-18) and sulphonyl chloride (IIb-29). MS: 492 [M+H⁺].

### Compound 37.

### 6-chloro-N-[3-[[2-(2,3-dihydro-1,4-benzodioxin-5-yloxy)ethylamino]methyl]phenyl]-naphthalene-2-sulphonamide

The title compound was prepared starting from the compound (Boc-IIa-18) and sulphonyl chloride (IIb-30). MS: 526 [M+H⁺].

### Compound 38.

### N-[3-[[2-(2,3-dihydro-1,4-benzodioxin-5-yloxy)ethylamino]methyl]phenyl]-1,3-benzo-dioxole-5-sulphonamide

The title compound was prepared starting from the compound (Boc-IIa-18) and sulphonyl chloride (IIb-33). MS: 486 [M+H⁺].

### Compound 39.

### N-[3-[[2-(2,3-dihydro-1,4-benzodioxin-5-yloxy)ethylamino]methyl]phenyl]-6,7-dihydro-5H-pyrrolo[1,2-a]imidazole-3-sulphonamide

The title compound was prepared starting from the compound (Boc-IIa-18) and sulphonyl chloride (IIb-35). MS: 472 [M+H⁺].

### Compound 40.

### N-[3-[[2-(2,3-dihydro-1,4-benzodioxin-5-yloxy)ethylamino]methyl]phenyl]-1-methyl-indole-4-sulphonamide

The title compound was prepared starting from the compound (Boc-IIa-18) and sulphonyl chloride (IIb-37). MS: 495 [M+H⁺].

### Compound 41.

### N-[3-[[2-(2,3-dihydro-1,4-benzodioxin-5-yloxy)ethylamino]methyl]phenyl]-1-methyl-indole-5-sulphonamide

The title compound was prepared starting from the compound (Boc-IIa-18) and sulphonyl chloride (IIb-38). MS: 495 [M+H⁺].

### Compound 42.

### N-[3-[[2-(2,3-dihydro-1,4-benzodioxin-5-yloxy)ethylamino]methyl]phenyl]benzofuran-2-sulphonamide

The title compound was prepared starting from the compound (Boc-IIa-18) and sulphonyl chloride (IIb-41). MS: 482 [M+H⁺].

### Compound 43.

### N-[3-[[2-(2,3-dihydro-1,4-benzodioxin-5-yloxy)ethylamino]methyl]phenyl]benzo-thiophene-3-sulphonamide

The title compound was prepared starting from the compound (Boc-IIa-18) and sulphonyl chloride (IIb-43). MS: 498 [M+H⁺].

### Compound 44.

### N-[3-[[2-(2,3-dihydro-1,4-benzodioxin-5-yloxy)ethylamino]methyl]phenyl]-5-fluoro-3-methyl-benzothiophene-2-sulphonamide

The title compound was prepared starting from the compound (Boc-IIa-18) and sulphonyl chloride (IIb-46). MS: 530 [M+H⁺].

### Compound 45.

### 3-chloro-N-[3-[[2-(2,3-dihydro-1,4-benzodioxin-5-yloxy)ethylamino]methyl]phenyl]-4-fluorobenzenesulphonamide

The title compound was prepared starting from the compound (Boc-IIa-18) and sulphonyl chloride (IIb-48). MS: 494 [M+H⁺].

### Compound 46.

### N-[3-[[2-[(2,2-dimethyl-3H-benzofuran-7-yl)oxy]ethylamino]methyl]phenyl]-3-methyl-benzenesulphonamide

The title compound was prepared starting from the compound (Boc-IIa-19) and sulphonyl chloride (IIb-11). MS: 468 [M+H⁺].

### Compound 47.

### N-[3-[[2-[(2,2-dimethyl-3H-benzofuran-7-yl)oxy]ethylamino]methyl]phenyl]naphthalene-1-sulphonamide

The title compound was prepared starting from the compound (Boc-IIa-19) and sulphonyl chloride (IIb-28). MS: 504 [M+H⁺].

### Compound 48.

### N-[3-[[2-[(2,2-dimethyl-3H-benzofuran-7-yl)oxy]ethylamino]methyl]phenyl]naphthalene-2-sulphonamide

The title compound was prepared starting from the compound (Boc-IIa-19) and sulphonyl chloride (IIb-29). MS: 504 [M+H⁺].

### Compound 49.

### N-[3-[[2-(1H-indol-4-yloxy)ethylamino]methyl]phenyl]-3-methyl-benzenesulphonamide

The title compound was prepared starting from the compound (Boc-IIa-20) and sulphonyl chloride (IIb-11). MS: 436 [M+H⁺].

### Compound 50.

### N-[3-[[2-(1H-indol-4-yloxy)ethylamino]methyl]phenyl]-4-phenyl-benzenesulphonamide

The title compound was prepared starting from the compound (Boc-IIa-20) and sulphonyl chloride (IIb-23). MS: 499 [M+H⁺].

### Compound 51.

### N-[3-[[2-(1H-indol-4-yloxy)ethylamino]methyl]phenyl]naphthalene-1-sulphonamide

The title compound was prepared starting from the compound (Boc-IIa-20) and sulphonyl chloride (IIb-28). MS: 472 [M+H⁺].

### Compound 52.

### N-[3-[[2-(1H-indol-4-yloxy)ethylamino]methyl]phenyl]naphthalene-2-sulphonamide

The title compound was prepared starting from the compound (Boc-IIa-20) and sulphonyl chloride (IIb-29). MS: 472 [M+H⁺].

### Compound 53.

### N-[3-[[3-(5-fluoro-1,2-benzoxazol-3-yl)propylamino]methyl]phenyl]-3-methyl-benzenesulphonamide

The title compound was prepared starting from the compound (Boc-IIa-21) and sulphonyl chloride (IIb-11). MS: 455 [M+H⁺].

### Compound 54.

### N-[3-[[3-(6-fluoro-1,2-benzoxazol-3-yl)propylamino]methyl]phenyl]naphthalene-1-sulphonamide

The title compound was prepared starting from the compound (Boc-IIa-21) and sulphonyl chloride (IIb-28). MS: 491 [M+H⁺].

### Compound 55.

### N-[3-[[3-(6-fluoro-1,2-benzoxazol-3-yl)propylamino]methyl]phenyl]naphthalene-2-sulphonamide

The title compound was prepared starting from the compound (Boc-IIa-21) and sulphonyl chloride (IIb-29). MS: 491 [M+H⁺].

### Compound 100.

### N-[3-[[2-(3-methylphenoxy)ethylamino]methyl]phenyl]naphthalene-2-sulphonamide

The title compound was prepared starting from the compound (Boc-IIa-8) and sulphonyl chloride (IIb-29). MS: 447 [M+H⁺].

### Compound 101.

### N-[3-[[2-(3-hydroxyphenoxy)ethylamino]methyl]phenyl]naphthalene-2-sulphonamide

The title compound was prepared starting from the compound (Boc-IIa-16) and sulphonyl chloride (IIb-29). MS: 449 [M+H⁺].

### Compound 102.

### N-[3-[[2-(1H-indol-4-yloxy)ethylamino]methyl]phenyl]-4-propylbenzenesulphonamide

The title compound was prepared starting from the compound (Boc-IIa-20) and sulphonyl chloride (IIb-13). MS: 464 [M+H⁺].

### Compound 103.

### N-[3-[[2-(1H-indol-4-yloxy)ethylamino]methyl]phenyl]thiophene-2-sulphonamide

The title compound was prepared starting from the compound (Boc-IIa-20) and sulphonyl chloride (IIb-24). MS: 428 [M+H⁺].

### Compound 104.

### N-[3-[[2-(1H-indol-4-yloxy)ethylamino]methyl]phenyl]thiophene-3-sulphonamide

The title compound was prepared starting from the compound (Boc-IIa-20) and sulphonyl chloride (IIb-25). MS: 428 [M+H⁺].

### Compound 105.

### N-[3-[[2-(1H-indol-4-yloxy)ethylamino]methyl]phenyl]-2,5-dimethyl-thiophene-3-sulphonamide

The title compound was prepared starting from the compound (Boc-IIa-20) and sulphonyl chloride (IIb-26). MS: 456 [M+H⁺].

### Example 3.

### Preparation of starting materials of general formula (Me-IIa) - general procedure

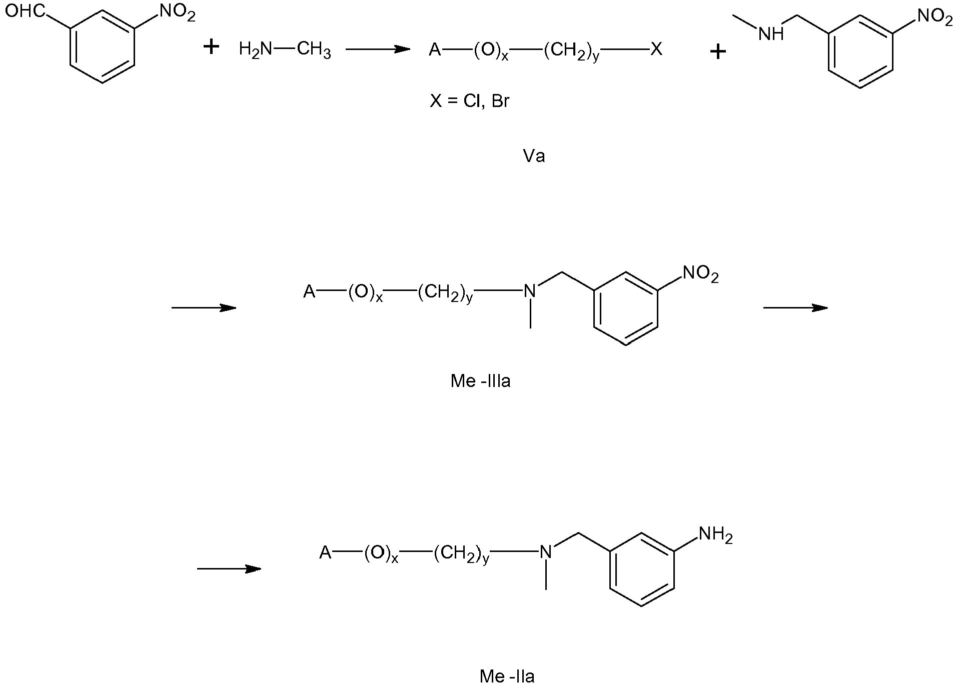

### a) Preparation of N-methyl-1-(3-nitrophenyl)methylamine

3-Nitrobenzaldehyde (80 mmol) is dissolved in methanol (60 ml) and then cooled to 0°C. To the resulting solution 40% aqueous solution of methylamine is added (93 mmol,) and the mixture is stirred for 1 h. Then sodium borohydride is added (120 mmol) portionwise. The mixture is left to warm to room temperature and then stirred for further 2h, followed by pouring onto a mixture NaHCO₃/ice. From the mixture methanol is evaporated, and the residue is extracted with ethyl acetate. The organic layer is dried over magnesium sulphate, and after filtering the drying agent off, the filtrate is concentrated under reduced pressure to obtain the crude product of purity of above 90% (UPLC/MS) with a yield of 78%.

### b) Preparation of intermediate of formula (Me-IIIa)

*N*-Methyl-1-(3-nitrophenyl)methylamine (15 mmol) is dissolved in acetonitrile (100 ml) and halogen derivative (Va) (15 mmol), potassium carbonate (45 mmol) and a catalytic amount of potassium iodide are added. The mixture is then stirred under reflux for 18 h. Then the mixture is cooled, precipitate is filtered off and solvent evaporated from the filtrate. The residue is partitioned between methylene chloride and water. The organic layer is separated and dried over magnesium sulphate. After filtering the drying agent off, the solvent is evaporated from the filtrate and the residue is purified by means of flash chromatography (methylene chloride/methanol 9:1 v/v), to obtain a product of purity of above 90% (UPLC/MS) with the yield of 65-90%.

According to the above general procedure, the following compounds were prepared: 2-(2,3-dihydro-1,4-benzodioxin-5-yloxy)-*N*-metylo-*N*-(3-nitrobenzyl)ethylamine (Me-IIIa-2), 2-[(2,2-dimethyl-2,3-dihydro-1-benzofuran-7-yl)oxy]-*N*-methyl-*N*-(3-nitrobenzyl)ethylamine (Me-IIIa-3), 2-(1*H*-indol-4-yloxy)-*N*-methyl-*N*-(3-nitrobenzyl)ethylamine (Me-IIIa-4), and 2-[(6-fluoro-1,2-benzoxazol-3-yl)oxy]-*N*-methyl-*N*-(3-nitrobenzyl)etylamine (Me-IIIa*-*5).

### c) Preparation of intermediate of formula (Me-IIa) - general procedure

Appropriate nitroderivative of formula (Me-IIIa) (3,5 mmol) is dissolved in ethanol (100 ml), followed by adding of stannous chloride (II) (17 mmol). The mixture is stirred at 60°C for 4 h, then etanol is evaporated and the residue is partitioned between etyl acetate and water. The organic layer is separated and dried over magnesium sulphate. After filtering the drying agent off, the solvent is evaporated from the filtrate to obtain a product of purity of above 90% (UPLC/MS) with the yield of 90-98%.

According to the above general procedure, the following compounds were prepared: 3-({[2-(2,3-dihydro-1,4-benzodioxin-5-yloxy)ethyl](methyl)amino}methyl)aniline (Me-IIa-2), 3-{[{2-[(2,2-dimethyl-2,3-dihydro-1-benzofuran-7-yl)oxy]ethyl}(methyl)amino]methyl}-aniline (Me-IIa-3), 3-({[2-(1H-indol-4-yloxy)ethyl](methyl)amino}methyl)aniline (Me-IIa-4), and 3-{[{2-[(6-fluoro-1,2-benzoxazol-3-yl)oxy]ethyl}(methyl)amino]methyl}aniline (Me-IIa-5).

### Example 4.

### Preparation of compounds (IB) according to the invention - general procedure

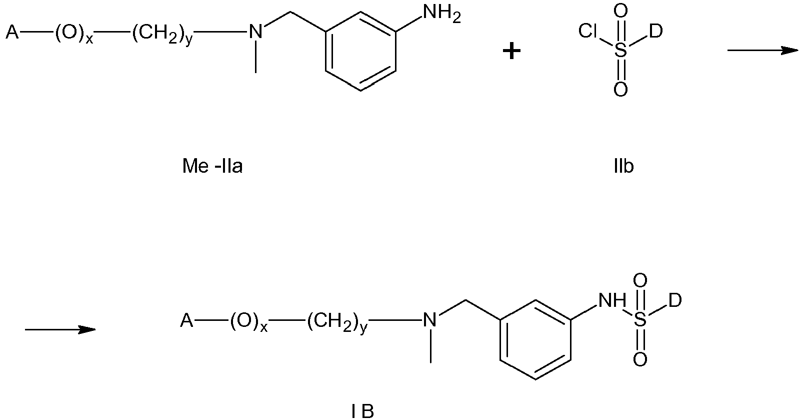

The appropriate compound of formula (Me-IIa) (0.32 mmol) prepared according to Example 3 is dissolved in dry methylene chloride (10 ml), then pyridine (1 ml), and sulphonyl chloride (0.32 mmol) were added. The mixture is stirred overnight at room temperature and subsequently pyridine is removed by co-evaporation with toluene, and the residue is partitioned between water and ethyl acetate. The organic layer is separated and dried over magnesium sulphate. After filtering the drying agent off, the solvent is evaporated from the filtrate and the residue is purified by means of "flash"-chromatography (methylene chloride/ methanol 9:1) to obtain compounds of the invention (IB) as oils.

Yield of compounds (IB) was in the range of 70-90%, and HPLC purity in the range of 90-100%.

Structure of prepared compounds was confirmed by MS analysis and/or ¹H NMR.

Following the general procedure as above and starting from appropriate intermediates: (Me-IIa) and sulphonyl chloride (IIb) (as described in Example 2) thef following compounds (IB) of the invention were prepared.

### Compound 57.

### N-[3-[[2-(2,3-dihydro-1,4-benzodioxin-5-yloxy)ethylmethylamino]methyl]phenyl]-benzenesulphonamide

The title compound was prepared starting from the compound (Me-IIa-2) and sulphonyl chloride (IIb-1). MS: 456 [M+H⁺].

### Compound 58.

### N-[3-[[2-[(2,2-dimethyl-3H-benzofuran-7-yl)oxy]ethylmethylamino]methyl]phenyl]-benzenesulphonamide

The title compound was prepared starting from the compound (Me-IIa-3) and sulphonyl chloride (IIb-1). MS: 468 [M+H⁺].

### Compound 59.

### N-[3-[[2-(1H-indol-4-yloxy)ethylmethylamino]methyl]phenyl]benzenesulphonamide

The title compound was prepared starting from the compound (Me-IIa-4) and sulphonyl chloride (IIb-1). MS: 436 [M+H⁺].

### Compound 60.

### N-[3-[[3-(6-fluoro-1,2-benzoxazol-3-yl)propylmethylamino]methyl]phenyl]benzenesulphonamide

The title compound was prepared starting from the compound (Me-IIa-5) and sulphonyl chloride (II-1). MS: 455 [M+H⁺].

### Compound 61.

### 4-fluoro-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-3). MS: 473 [M+H⁺].

### Compound 62.

### 3,4-difluoro-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-4). MS: 491 [M+H⁺].

### Compound 63.

### 3-chloro-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-5). MS: 489 [M+H⁺].

### Compound 64.

### 4-chloro-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-6). MS: 489 [M+H⁺].

### Compound 65.

### 3,4-dichloro-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-7). MS: 523 [M+H⁺].

### Compound 66.

### 3-bromo-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-8). MS: 533 [M+H⁺].

### Compound 67.

### 4-bromo-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-9). MS: 533 [M+H⁺].

### Compound 68.

### N-[3-[[3-(6-fluoro-1,2-benzoxazol-3-yl)propylmethylamino]methyl]phenyl]-4-iodo-benzenesulphonamide

The title compound was prepared starting from the compound (Me-IIa-5) and sulphonyl chloride (IIb-10). MS: 580 [M+H⁺].

### Compound 69.

### N-[3-[[3-(6-fluoro-1,2-benzoxazol-3-yl)propylmethylamino]methyl]phenyl]-3-methyl-benzenesulphonamide

The title compound was prepared starting from the compound (Me-IIa-5) and sulphonyl chloride (IIb-11). MS: 469 [M+H⁺].

### Compound 70.

### N-[3-[[3-(6-fluoro-1,2-benzoxazol-3-yl)propylmethylamino]methyl]phenyl]-4-methyl-benzenesulphonamide

The title compound was prepared starting from the compound (Me-IIa-5) and sulphonyl chloride (IIb-12). MS: 469 [M+H⁺].

### Compound 71.

### N-[3-[[3-(6-fluoro-1,2-benzoxazol-3-yl)propylmethylamino]methyl]phenyl]-4-propyl-benzenesulphonamide

The title compound was prepared starting from the compound (Me-IIa-5) and sulphonyl chloride (IIb-13). MS: 497 [M+H⁺].

### Compound 72.

### 4-tert-butylo-N-[3-[[3-(6-fluoro-1,2-benzoxazol-3-yl)propylmethylamino]methyl]-phenyl]benzenesulphonamide

The title compound was prepared starting from the compound (Me-IIa-5) and sulphonyl chloride (IIb-14). MS: 511 [M+H⁺].

### Compound 73.

### N-[3-[[3-(6-fluoro-1,2-benzoxazol-3-yl)propylmethylamino]methyl]phenyl]-3-(trifluo-romethyl)benzenesulphonamide

The title compound was prepared starting from the compound (Me-IIa-5) and sulphonyl chloride (IIb-15). MS: 523 [M+H⁺].

### Compound 74.

### N-[3-[[3-(6-fluoro-1,2-benzoxazol-3-yl)propylmethylamino]methyl]phenyl]-4-(trifluoro-methyl)benzenesu lphonamide

The title compound was prepared starting from the compound (Me-IIa-5) and sulphonyl chloride (IIb-16). MS: 523 [M+H⁺].

### Compound 75.

### N-[3-[[3-(6-fluoro-1,2-benzoxazol-3-yl)propylmethylamino]methyl]phenyl]-3-methoxy-benzenesulphonamide

The title compound was prepared starting from the compound (Me-IIa-5) and sulphonyl chloride (IIb-18). MS: 485 [M+H⁺].

### Compound 76.

### N-[3-[[3-(6-fluoro-1,2-benzoxazol-3-yl)propylmethylamino]methyl]phenyl]-4-methoxy-benzenesulphonamide

The title compound was prepared starting from the compound (Me-IIa-5) and sulphonyl chloride (IIb-19). MS: 485 [M+H⁺].

### Compound 77.

### N-[3-[[3-(6-fluoro-1,2-benzoxazol-3-yl)propylmethylamino]methyl]phenyl]-4-(trifluoro-methoxy)benzenesulphonamide

The title compound was prepared starting from the compound (Me-IIa-5) and sulphonyl chloride (IIb-20). MS: 539 [M+H⁺].

### Compound 78.

### 3-cyano-N-[3-[[3-(6-fluoro-1,2-benzoxazol-3-yl)propylmethylamino]methyl]phenyl]-benzenesulphonamide

The title compound was prepared starting from the compound (Me-IIa-5) and sulphonyl chloride (IIb-21). MS: 480 [M+H⁺].

### Compound 79.

### N-[3-[[3-(6-fluoro-1,2-benzoxazol-3-yl)propylmethylamino]methyl]phenyl]-4-phenyl-benzenesulphonamide

The title compound was prepared starting from the compound (Me-IIa-5) and sulphonyl chloride (IIb-23).

MS: 531 [M+H⁺].

### Compound 80.

### N-[3-[[3-(6-fluoro-1,2-benzoxazol-3-yl)propylmethylamino]methyl]phenyl]thiophene-2-sulphonamide

The title compound was prepared starting from the compound (Me-IIa-5) and sulphonyl chloride (IIb-24). MS: 461 [M+H⁺].

### Compound 81.

### N-[3-[[3-(6-fluoro-1,2-benzoxazol-3-yl)propylmethylamino]methyl]phenyl]thiophene-3-sulphonamide

The title compound was prepared starting from the compound (Me-IIa-5) and sulphonyl chloride (IIb-25). MS: 461 [M+H⁺].

### Compound 82.

### N-[3-[[3-(6-fluoro-1,2-benzoxazol-3-yl)propylmethylamino]methyl]phenyl]-2,5-dimethyl-thiophene-3-sulphonamide

The title compound was prepared starting from the compound (Me-IIa-5) and sulphonyl chloride (IIb-26). MS: 489 [M+H⁺].

### Compound 83.

### N-[3-[[3-(6-fluoro-1,2-benzoxazol-3-yl)propylmethylamino]methyl]phenyl]-5-isoxazol-5-yl-thiophene-2-sulphonamide

The title compound was prepared starting from the compound (Me-IIa-5) and sulphonyl chloride (IIb-27). MS: 528 [M+H⁺].

### Compound 84.

### N-[3-[[3-(6-fluoro-1,2-benzoxazol-3-yl)propylmethylamino]methyl]phenyl]naphthalene-1-sulphonamide

The title compound was prepared starting from the compound (Me-IIa-5) and sulphonyl chloride (IIb-28). MS: 505 [M+H⁺].

### Compound 85.

### N-[3-[[3-(6-fluoro-1,2-benzoxazol-3-yl)propylmethylamino]methyl]phenyl]naphthalene-2-sulphonamide

The title compound was prepared starting from the compound (Me-IIa-5) and sulphonyl chloride (IIb-29). MS: 505 [M+H⁺].

### Compound 86.

### 6-chloro-N-[3-[[3-(6-fluoro-1,2-benzoxazol-3-yl)propylmethylamino]methyl]phenyl]-naphthalene-2-sulphonamide

The title compound was prepared starting from the compound (Me-IIa-5) and sulphonyl chloride (IIb-30). MS: 539 [M+H⁺].

### Compound 87.

### N-[3-[[3-(6-fluoro-1,2-benzoxazol-3-yl)propylmethylamino]methyl]phenyl]-2,3-dihydro-1,4-benzodioxine-6-sulphonamide

The title compound was prepared starting from the compound (Me-IIa-5) and sulphonyl chloride (IIb-32). MS: 513 [M+H⁺].

### Compound 88.

### N-[3-[[3-(6-fluoro-1,2-benzoxazol-3-yl)propylmethylamino]methyl]phenyl]-1,3-benzo-dioxole-5-sulphonamide

The title compound was prepared starting from the compound (Me-IIa-5) and sulphonyl chloride (IIb-33). MS: 499 [M+H⁺].

### Compound 89.

### N-[3-[[3-(6-fluoro-1,2-benzoxazol-3-yl)propylmethylamino]methyl]phenyl]-2-oxo-indoline-5-sulphonamide

The title compound was prepared starting from the compound (Me-IIa-5) and sulphonyl chloride (IIb-34). MS: 510 [M+H⁺].

### Compound 90.

### N-[3-[[3-(6-fluoro-1,2-benzoxazol-3-yl)propylmethylamino]methyl]phenyl]-6,7-dihydro-5H-pyrrolo[1,2-a]imidazole-3-sulphonamide

The title compound was prepared starting from the compound (Me-IIa-5) and sulphonyl chloride (IIb-35). MS: 485 [M+H⁺].

### Compound 91.

### N-[3-[[3-(6-fluoro-1,2-benzoxazol-3-yl)propylmethylamino]methyl]phenyl]-1-methylindole-4-sulphonamide

The title compound was prepared starting from the compound (Me-IIa-5) and sulphonyl chloride (IIb-37). MS: 508 [M+H⁺].

### Compound 92.

### N-[3-[[3-(6-fluoro-1,2-benzoxazol-3-yl)propylmethylamino]methyl]phenyl]-1-methyl-indole-5-sulphonamide

The title compound was prepared starting from the compound (Me-IIa-5) and sulphonyl chloride (IIb-38). MS: 508 [M+H⁺].

### Compound 93.

### N-[3-[[3-(6-fluoro-1,2-benzoxazol-3-yl)propylmethylamino]methyl]phenyl]-1,3-benzothiazole-4-sulphonamide

The title compound was prepared starting from the compound (Me-IIa-5) and sulphonyl chloride (IIb-39). MS: 512 [M+H⁺].

### Compound 94.

### N-[3-[[3-(6-fluoro-1,2-benzoxazol-3-yl)propylmethylamino]methyl]phenyl]-1,3-benzothiazole-5-sulphonamide

The title compound was prepared starting from the compound (Me-IIa-5) and sulphonyl chloride (IIb-40). MS: 512 [M+H⁺].

### Compound 95.

### N-[3-[[3-(6-fluoro-1,2-benzoxazol-3-yl)propylmethylamino]methyl]phenyl]benzofuran-2-sulphonamide

The title compound was prepared starting from the compound (Me-IIa-5) and sulphonyl chloride (IIb-41). MS: 495 [M+H⁺].

### Compound 96.

### N-[3-[[3-(6-fluoro-1,2-benzoxazol-3-yl)propylmethylamino]methyl]phenyl]-benzothiophene-3-sulphonamide

The title compound was prepared starting from the compound (Me-IIa-5) and sulphonyl chloride (IIb-43). MS: 511 [M+H⁺].

### Compound 97.

### 5-fluoro-N-[3-[[3-(6-fluoro-1,2-benzoxazol-3-yl)propylmethylamino]methyl]phenyl]-3-methyl-benzothiophene-2-sulphonamide

The title compound was prepared starting from the compound (Me-IIa-5) and sulphonyl chloride (IIb-46). MS: 543 [M+H⁺].

### Compound 98.

### 5-chloro-N-[3-[[3-(6-fluoro-1,2-benzoxazol-3-yl)propylmethylamino]methyl]phenyl]-3-methyl-benzothiophene-2-sulphonamide

The title compound was prepared starting from the compound (Me-IIa-5) and sulphonyl chloride (IIb-47). MS: 559 [M+H⁺].

### Compound 99.

### 3-chloro-4-fluoro-N-[3-[[3-(6-fluoro-1,2-benzoxazol-3-yl)propylmethylamino]methyl]-phenyl]benzenesulphonamide

The title compound was prepared starting from the compound (Me-IIa-5) and sulphonyl chloride (IIb-48). MS: 507 [M+H⁺].

### Example 5.

### In Vitro Pharmacology: Binding Assays

The affinity of compounds of the present invention to dopaminergic, serotoninergic, adrenergic, muscarinic M3, histaminergic H1, sigma and serotonine trensporter SERT receptors was tested using the methods as described below, by measurement their binding to these receptors using radioreceptors methods.

Specific ligand binding to the receptors is defined as the difference between the total binding and the non-specific binding determined in the presence of an excess of unlabelled ligand.

The results are expressed as a percent of control specific binding ((measured specific binding/control specific binding) x 100%) obtained in the presence of the test compounds. The compounds were tested for their affinity to receptors at a concentration of 1 x 10⁻⁶M.

Conditions and methodology of in vitro tests are given by reference to the literature in Table 1, and the results of tests for representative compounds are given in Table 2 (dopaminergic receptors D2 and D3), in Table 3 (dopaminergic receptors D1 and D4.4), in Table 4 (serotoninergic receptors 5-HT1A, 5-HT2A, 5-HT6, 5-HT7), in table 5 (serotoninergic receptors 5-HT2C), in Table 6 (sigma σ receptors), in Table 7 (adrenergic receptors α1, histaminergic receptors H1 and muscarinic receptors M3), in Table 8 (adrenergic receptors α2C) and in Table 9 (serotonin transporter receptors SERT).

**Table 1: Conditions and methodology of in vitro affinity assays**

| **Assay** | **Origin** | **Radioligand** | **Concentration** | **Kd** | **Non-specific** | **Incubation** | **Method of Detection** | **Ref.** |
|---|---|---|---|---|---|---|---|---|
| **α₁ (non-selective)** | rat cerebral cortex | [³H]prazosin | 0.25 nM | 0.09 nM | prazosin (0.5 µM) | 60 min 22°C | Scintillation counting | 1 |
| **α_{2C} *(h)*** | human recombinant (CHO cells) | [³H]RX 821002 | 2 nM | 0.95 nM | (-)epinephrine (100 µM) | 60 min 22°C | Scintillation counting | 2 |
| **D1 *(h)*** | human recombinant (CHO cells) | [³H]SCH 23390 | 0.3 nM | 0.2 nM | SCH 23390 (1 µM) | 60 min 22°C | Scintillation counting | 3 |
| **D_{2S} *(h)*** | human recombinant (HEK-293 cells) | [³H]methyl-spiperone | 0.3 nM | 0.15 nM | (+)butaclamol (10 µM) | 60 min 22°C | Scintillation counting | 4 |
| **D3 *(h)*** | human recombinant (CHO cells) | [³H]methyl-spiperone | 0.3 nM | 0.085 nM | (+)butaclamol (10 µM) | 60 min 22°C | Scintillation counting | 5 |
| **D4.4 *(h)*** | human recombinant (CHO cells) | [³H]methyl-spiperone | 0.3 nM | 0.19 nM | (+)butaclamol (10 µM) | 60 min 22°C | Scintillation counting | 6 |
| **H₁ *(h)*** | human recombinant (HEK-293 cells) | [3H]pyrilamine | 1 nM | 1.7 nM | pyrilamine (1 µM) | 60 min 22°C | Scintillation counting | 7 |
| **M₃ *(h)*** | human recombinant (CHO cells) | [³H]4-DAMP | 0.2 nM | 0.5 nM | atropine (1 µM) | 60 min 22°C | Scintillation counting | 8 |
| **5-HT_{1A} *(h)*** | human recombinant (HEK-293 cells) | [³H]8-OH-DPAT | 0.3 nM | 0.5 nM | 8-OH-DPAT (10 µM) | 60 min 22°C | Scintillation counting | 9 |
| **5-HT_{2A} *(h)*** | human recombinant (HEK-293 cells) | [³H]ketanserin | 0.5 nM | 0.6 nM | ketanserin (1 µM) | 60 min 22°C | Scintillation counting | 10 |
| **5-HT_{2C} *(h)*** | human recombinant (HEK-293 cells) | [³H]mesulergine | 1 nM | 0.5 nM | RS 102221 (10 µM) | 120 min 37°C | Scintillation counting | 11 |
| **5-HT₆ *(h)*** | human recombinant (CHO cells) | [³H]LSD | 2 nM | 1.8 nM | serotonin (100 µM) | 120 min 37°C | Scintillation counting | 12 |
| **5-HT₇ *(h)*** | human recombinant (CHO cells) | [³H]LSD | 4 nM | 2.3 nM | serotonin (10 µM) | 120 min 22°C | Scintillation counting | 13 |
| **σ (non-selective)** | rat cerebral cortex | [³H]DTG | 8 nM | 29 nM | haloperidol (10 µM) | 120 min 22°C | Scintillation counting | 14 |
| **SERT *(h)*** | human recombinant (CHO cells) | [³H]imipramine | 2 nM | 1.7 nM | imipramine (10 µM) | 60 min 22°C | Scintillation counting | 15 |

### References:

**1.** Greengrass, P. and Bremner, R. (1979), Eur. J. Pharmacol., 55: 323-326.
**2.** Devedjian et al. (1994), Eur. J. Pharmacol., 252: 43-49
**3.** Zhou et al. (1990), Nature, 347: 76-80*.*
**4.** Grandy et al. (1989), Proc. Natl. Acad. Sci. U.S.A., 86: 9762-9766*.*
**5.** Mackenzie et al. (1994), Eur. J. Pharmacol., 266: 79-85*.*
**6.** Van Tol et al. (1992), Nature, 358: 149-152*.*
**7.** Smit et al. (1996), Brit. J. Pharmacol., 117: 1071-1080*.*
**8.** Peralta et al. (1987), Embo. J., 6: 3923-3929.
**9.** Mulheron et al. (1994), J. Biol. Chem., 269: 12954-12962*.*
**10.** Bonhaus et al. (1995), Brit. J. Pharmacol., 115: 622-628*.*
**11.** Stam et al. (1994), Eur. J. Pharmacol., 269: 339-348*.*
**12.** Monsma et al. (1993), Mol. Pharmacol., 43: 320-327*.*
**13.** Shen et al. (1993), J. Biol. Chem., 268: 18200-18204*.*
**14.** Shirayama et al. (1993), Eur. J. Pharmacol., 237: 117-126*.*
**15.** Tatsumi et al. (1999), Eur. J. Pharmacol., 368: 277-283*.*

**Table 2: Results of binding assays for receptors D2 and D3 for representative compounds of the invention**

| **Compound No.** | **D2 [%]** | **D3 [%]** | **Compound No.** | **D2 [%]** | **D3 [%]** |
|---|---|---|---|---|---|
| **1** | 77 | 65 | **52** | 99 | 97 |
| **6** | 79 | 73 | **54** | 94 | 76 |
| **8** | 59 | 37 | **60** | 96 | 82 |
| **9** | 88 | 67 | **62** | 81 | 55 |
| **10** | 97 | 88 | **64** | 86 | 72 |
| **11** | 47 | 50 | **65** | 94 | 73 |
| **12** | 97 | 97 | **66** | 94 | 87 |
| **14** | 74 | 58 | **68** | 97 | 92 |
| **17** | 49 | 56 | **70** | 93 | 76 |
| **18** | 96 | 98 | **71** | 98 | 92 |
| **19** | 98 | 96 | **72** | 93 | 63 |
| **20** | 98 | 94 | **73** | 90 | 72 |
| **22** | 96 | 95 | **77** | 96 | 80 |
| **24** | 98 | 99 | **79** | 94 | 81 |
| **25** | 98 | 94 | **80** | 92 | 74 |
| **26** | 99 | 97 | **81** | 96 | 81 |
| **31** | 97 | 96 | **82** | 96 | 79 |
| **32** | 101 | 99 | **84** | 98 | 84 |
| **33** | 97 | 91 | **85** | 99 | 92 |
| **35** | 100 | 96 | **91** | 94 | 82 |
| **36** | 99 | 95 | **92** | 99 | 89 |
| **39** | 98 | 95 | **94** | 98 | 85 |
| **43** | 98 | 97 | **95** | 88 | 73 |
| **44** | 96 | 93 | **96** | 96 | 92 |
| **45** | 98 | 96 | **97** | 98 | 89 |
| **49** | 99 | 97 | **98** | 92 | 83 |
| **51** | 99 | 96 | | | |

**Table 3: Results of binding assay for receptors D1 and D4.4 for representative compounds of the invention**

| **Compound No.** | **D1 [%]** | **D4.4 [%]** | **Compound No.** | **D1 [%]** | **D4.4 [%]** |
|---|---|---|---|---|---|
| **1** | 57 | 15 | **52** | 43 | 97 |
| **6** | 41 | 25 | **54** | 27 | 54 |
| **8** | 32 | 11 | **60** | 58 | 98 |
| **9** | 39 | 24 | **62** | 57 | 89 |
| **10** | 12 | 77 | **64** | 79 | 87 |
| **11** | 26 | 4 | **65** | 73 | 77 |
| **12** | 23 | 81 | **66** | 75 | 95 |
| **14** | 4 | 12 | **68** | 86 | 59 |
| **17** | 2 | 40 | **70** | 66 | 66 |
| **18** | 12 | 95 | **71** | 85 | 81 |
| **19** | 25 | 92 | **72** | 45 | 60 |
| **20** | 29 | 89 | **73** | 82 | 90 |
| **22** | 49 | 92 | **77** | 73 | 63 |
| **24** | 27 | 88 | **79** | 68 | 56 |
| **25** | 17 | 95 | **80** | 72 | 81 |
| **26** | 27 | 93 | **81** | 70 | 92 |
| **31** | 19 | 93 | **82** | 64 | 92 |
| **32** | 23 | 97 | **84** | 72 | 96 |
| **33** | 36 | 90 | **85** | 77 | 70 |
| **35** | 23 | 92 | **91** | 76 | 70 |
| **36** | 13 | 65 | **92** | 83 | 56 |
| **39** | 14 | 49 | **94** | 61 | 63 |
| **43** | 40 | 95 | **95** | 63 | 42 |
| **44** | 28 | 43 | **96** | 78 | 99 |
| **45** | 38 | 88 | **97** | 80 | 55 |
| **49** | 61 | 100 | **98** | 62 | 37 |
| **51** | 33 | 91 | | | |

**Table 4: Results of binding assays for serotoninergic receptors 5-HT1A, 5-HT2A, 5-HT6 and 5-HT7 for representative compounds of the invention**

| **Compound No.** | **5-HT1A [%]** | **5-HT2A [%]** | **5-HT6 [%]** | **5-HT7 [%]** |
|---|---|---|---|---|
| **1** | 55 | 97 | 94 | 61 |
| **6** | 74 | 64 | 98 | 54 |
| **8** | 83 | 81 | 95 | -159 |
| **9** | 66 | 84 | 97 | 61 |
| **10** | 93 | 83 | 93 | 74 |
| **11** | 80 | 70 | 101 | 35 |
| **12** | 98 | 93 | 79 | 99 |
| **14** | 18 | 69 | 91 | 27 |
| **17** | 90 | 61 | 96 | 87 |
| **18** | 98 | 79 | 58 | 100 |
| **19** | 98 | 84 | 72 | 103 |
| **20** | 102 | 67 | 61 | 96 |
| **22** | 98 | 97 | 94 | 101 |
| **24** | 98 | 62 | 80 | 101 |
| **25** | 98 | 85 | 64 | 99 |
| **26** | 101 | 80 | 79 | 98 |
| **31** | 97 | 77 | 40 | 100 |
| **32** | 99 | 83 | 67 | 98 |
| **33** | 98 | 82 | 73 | 100 |
| **35** | 100 | 78 | 87 | 99 |
| **36** | 100 | 82 | 88 | 99 |
| **39** | 100 | 54 | 72 | 90 |
| **43** | 98 | 85 | 99 | 101 |
| **44** | 98 | 91 | 94 | 104 |
| **45** | 99 | 74 | 80 | 101 |
| **49** | 101 | 87 | 87 | 100 |
| **51** | 100 | 66 | 88 | 99 |
| **52** | 100 | 98 | 97 | 99 |
| **54** | 43 | 80 | 88 | 90 |
| **60** | 89 | 96 | 95 | 98 |
| **62** | 46 | 91 | 95 | 95 |
| **64** | 36 | 98 | 98 | 98 |
| **65** | 33 | 89 | 100 | 95 |
| **66** | 38 | 92 | 101 | 100 |
| **68** | 81 | 101 | 100 | 96 |
| **70** | 11 | 95 | 55 | 97 |
| **71** | 45 | 101 | 101 | 97 |
| **72** | 30 | 87 | 99 | 93 |
| **73** | 55 | 91 | 97 | 96 |
| **77** | 26 | 100 | 99 | 90 |
| **79** | 69 | 101 | 94 | 92 |
| **80** | 28 | 93 | 98 | 97 |
| **81** | 10 | 96 | 100 | 99 |
| **82** | 84 | 93 | 98 | 98 |
| **84** | 46 | 88 | 101 | 94 |
| **85** | 9 | 99 | 96 | 96 |
| **91** | 33 | 97 | 101 | 97 |
| **92** | 45 | 98 | 100 | 91 |
| **94** | 51 | 99 | 90 | 94 |
| **95** | 29 | 92 | 100 | 85 |
| **96** | 46 | 94 | 101 | 100 |
| **97** | 30 | 94 | 99 | 95 |
| **98** | 4 | 96 | 100 | 92 |

**Table 5: Results of binding assays for serotoninergic receptors 5-HT2C for representative compounds of the invention**

| Compound No. | 5-HT2C [%] | Compound No. | 5-HT2C [%] | Compound No. | 5-HT2C [%] |
|---|---|---|---|---|---|
| **1** | 34 | **33** | 59 | 71 | 69 |
| **6** | 43 | **35** | 21 | 72 | 23 |
| **8** | 10 | **36** | 33 | 73 | 2 |
| **9** | 31 | **39** | 7 | 77 | 30 |
| **10** | 35 | **43** | 31 | **79** | 33 |
| **11** | -1 | **44** | 33 | **80** | 31 |
| **12** | 65 | **45** | 39 | **81** | 37 |
| **14** | 21 | **49** | 10 | **82** | 24 |
| **17** | 9 | **51** | -9 | **84** | 42 |
| **18** | 50 | **52** | 21 | **85** | 56 |
| **19** | 58 | **54** | 17 | **91** | 31 |
| **20** | 20 | **60** | 40 | **92** | 57 |
| **22** | 59 | **62** | 16 | **94** | 12 |
| **24** | 34 | **64** | 51 | **95** | 22 |
| **25** | 40 | **65** | 23 | **96** | 59 |
| **26** | 54 | **66** | 39 | **97** | 21 |
| **31** | 51 | **68** | 77 | **98** | 12 |
| **32** | 46 | **70** | 43 | | |

**Table 6: Results of binding assays for σ receptors affinity tests for representative compounds of the invention**

| **Compound No.** | **σ [%]** | **Compound No.** | **σ [%]** | **Compound No.** | **σ [%]** |
|---|---|---|---|---|---|
| **1** | 83 | **36** | 84 | **72** | 80 |
| **6** | 73 | **39** | 64 | **73** | 73 |
| **8** | 85 | **43** | 88 | **77** | 48 |
| **10** | 74 | **44** | 91 | **79** | 61 |
| **11** | 76 | **45** | 92 | **80** | 42 |
| **12** | 93 | **49** | 79 | **81** | 69 |
| **14** | 54 | **51** | 76 | **82** | 49 |
| **17** | 71 | **52** | 79 | **84** | 77 |
| **18** | 87 | **54** | 50 | **85** | 59 |
| **19** | 89 | **60** | 46 | **91** | 53 |
| **22** | 95 | **62** | 49 | **92** | 72 |
| **24** | 93 | **64** | 59 | **94** | 79 |
| **25** | 89 | **65** | 72 | **95** | 79 |
| **31** | 80 | **66** | 61 | **96** | 59 |
| **32** | 88 | **68** | 73 | **97** | 74 |
| **33** | 92 | **70** | 60 | **98** | 62 |
| **35** | 83 | **71** | 82 | | |

**Table 7: Results of binding assays for adrenergic α1, histaminergic H1 and muscarinic M3 receptors for representative compounds of the invention**

| **Compound No.** | **α1 [%]** | **H1 [%]** | **M3 [%]** | **Compound No.** | **α1 [%]** | **H1 [%]** | **M3 [%]** |
|---|---|---|---|---|---|---|---|
| **1** | 26 | 17 | 17 | **52** | 41 | 2 | 0 |
| **6** | 53 | 24 | 6 | **54** | 48 | 7 | 2 |
| **8** | 17 | 18 | 1 | **60** | 80 | 22 | 17 |
| **9** | 56 | 23 | -9 | **62** | 83 | 42 | 7 |
| **10** | 20 | -13 | 21 | **64** | 81 | 43 | 10 |
| **11** | 18 | -8 | 13 | **65** | 70 | 50 | 19 |
| **12** | 53 | 49 | 17 | **66** | 86 | 49 | 5 |
| **14** | 13 | 27 | 19 | **68** | 84 | 84 | 26 |
| **17** | 31 | -9 | 9 | **70** | 80 | 16 | 22 |
| **18** | 74 | 28 | 24 | **71** | 85 | 64 | 27 |
| **19** | 74 | 58 | 13 | **72** | 77 | 64 | 25 |
| **20** | 89 | 27 | 1 | **73** | 75 | 41 | 14 |
| **22** | 84 | 68 | 22 | **77** | 64 | 56 | 19 |
| **24** | 75 | 58 | 22 | **79** | 29 | 47 | 26 |
| **25** | 68 | 47 | 12 | **80** | 84 | 44 | 28 |
| **26** | 87 | 33 | -5 | **81** | 88 | 24 | 35 |
| **31** | 68 | 50 | 31 | **82** | 85 | 14 | |
| **32** | 83 | 0 | 15 | **84** | 75 | 20 | 13 |
| **33** | 73 | 27 | 20 | **85** | 78 | 6 | 22 |
| **35** | 57 | 31 | -8 | **91** | 85 | 16 | 15 |
| **36** | 49 | 30 | 12 | **92** | 79 | 25 | 6 |
| **39** | 80 | 49 | 6 | **94** | 86 | 58 | 49 |
| **43** | 73 | 21 | 12 | **95** | 62 | 72 | 10 |
| **44** | 52 | 88 | 19 | **96** | 78 | 56 | 16 |
| **45** | 83 | 70 | 21 | **97** | 59 | 82 | 0 |
| **49** | 59 | -3 | 7 | **98** | 42 | 59 | 16 |
| **51** | 34 | -4 | -8 | | | | |

**Table 8: Results of binding assays for adrenergic receptors α2C for representative compounds of the invention**

| **Compound No.** | **α2C [%]** | **Compound No.** | **α2C [%]** | **Compound No.** | **α2C [%]** |
|---|---|---|---|---|---|
| **1** | 44 | **33** | 93 | **71** | 76 |
| **6** | 59 | **35** | 95 | **72** | 81 |
| **8** | -30 | **36** | 87 | **73** | 51 |
| **9** | 72 | **39** | 86 | **77** | 53 |
| **10** | 75 | **43** | 98 | **79** | 87 |
| **11** | 45 | **44** | 89 | **80** | 51 |
| **12** | 90 | **45** | 90 | **81** | 48 |
| **14** | 32 | **49** | 90 | **82** | 68 |
| **17** | 91 | **51** | 84 | **84** | 79 |
| **18** | 88 | **52** | 89 | **85** | 74 |
| **19** | 88 | **54** | 32 | **91** | 80 |
| **20** | 86 | **60** | 52 | **92** | 79 |
| **22** | 95 | **62** | 43 | **94** | 71 |
| **24** | 87 | **64** | 73 | **95** | 49 |
| **25** | 88 | **65** | 73 | **96** | 81 |
| **26** | 92 | **66** | 66 | **97** | 73 |
| **31** | 83 | **68** | 83 | **98** | 84 |
| **32** | 90 | **70** | 53 | | |

**Table 9: Results of binding assays for serotonin transporter (SERT) for representative compounds of the invention**

| **Compound No.** | **SERT [%]** | **Compound No.** | **SERT [%]** | **Compound No.** | **SERT [%]** |
|---|---|---|---|---|---|
| **1** | 46 | **33** | 60 | **71** | 59 |
| **6** | 26 | **35** | 78 | **72** | 42 |
| **8** | 51 | **36** | 68 | **73** | 17 |
| **9** | 91 | **39** | 41 | **77** | 41 |
| **10** | 40 | **43** | 75 | **79** | 16 |
| **11** | 45 | **44** | 63 | **80** | 33 |
| **12** | 46 | **45** | 35 | **81** | 40 |
| **14** | 70 | **49** | 52 | **82** | 51 |
| **17** | 43 | **51** | 32 | **84** | 47 |
| **18** | 40 | **52** | 48 | **85** | 63 |
| **19** | 53 | **54** | 42 | **91** | 87 |
| **20** | 62 | **60** | 25 | **92** | 81 |
| **22** | 79 | **62** | 28 | **94** | 81 |
| **24** | 35 | **64** | 44 | **95** | 40 |
| **25** | 35 | **65** | 34 | **96** | 61 |
| **26** | 66 | **66** | 50 | **97** | 72 |
| **31** | 22 | **68** | 73 | **98** | 34 |
| **32** | 49 | **70** | 39 | | |

### Ability to block hERG potassium channel

Ability to block hERG potassium channels was determined using the electrophysiological method and cloned hERG potassium channels (KCNH2 gene, expressed in CHO cells) as biological material. The effects were evaluated using IonWorksTM Quattro system (MDS-AT).

hERG current was elicited using a pulse pattern with fixed amplitudes (conditioning prepulse: -80 mV for 25 ms; test pulse: +40 mV for 80 ms) from a holding potential of 0 mV. hERG current was measured as a difference between the peak current at 1 ms after the test step to +40 mV and the steady-state current at the end of the step to +40 mV.

Data acquisition and analyses were performed using the IonWorks QuattroTM system operation software (version 2.0.2; Molecular Devices Corporation, Union City, CA). Data were corrected for leak current.

The hERG block was calculated as: % Block = (1 - I TA / IControl) x 100%, where IControl and ITA were the currents elicited by the test pulse in control and in the presence of a test compound, respectively. Results for representative compounds are presented in Table 10.

**Table 10: Results for representative compounds**

| **Compound No.** | **hERG [%]** | **Compound No.** | **hERG [%]** | **Compound No.** | **hERG [%]** |
|---|---|---|---|---|---|
| **11** | -4 | **43** | 10 | **52** | 2 |
| **35** | -1 | **49** | 2 | **60** | 6 |
| **36** | 1 | **51** | 1 | | |

The above results for the representative compounds show that compounds of the invention demonstrate high affinity for the number of monoaminergic receptors, linked to the potential activity in the treatment of diseases of the central nervous system. Particularly preferred are compounds revealing simultaneously high affinity for dopaminergic D2 and serotoninergic 5-HT6 and/or 5-HT7 receptors. Such activity could be particularly beneficial for a new antipsychotic drug not only due to efficiency in the treatment of the positive symptoms, but also of the negative and cognitive symptoms of schizophrenia. Furthermore, particularly preferred compounds show high affinity for serotoninergic receptors 5-HT6 and/or 5-HT7, and lower affinity for dopaminergic receptors D2, what may be useful as an adjunctive therapy for the treatment of negative and cognitive symptoms of schizophrenia and in the treatment of depression, anxiety disorders and other diseases for which such a receptor modulation may bring a therapeutic effect, notably behavioral and psychological as well as cognitive symptoms of dementia. An important feature of the compounds of the present invention is also a significantly lower (from moderate to very low) affinity for biological targets associated with side effects, such as alpha 1, H1, M3, 5-HT2C receptors or hERG potassium channel. This feature is of particular advantage in the context of development of drugs having improved safety profile when compared to currently applied therapies.

### Example 6

### Antipsychotic activity in mice

Potential antipsychotic activity was tested for the representative compound 60 in mouse model of psychosis, involving the induction of locomotor hyperactivity by administering psychotomimetic substance - d-amphetamine. The ability of a test compound to remove that effect is a measure of potential antipsychotic activity.

### Animals

Male CD-1 mice weighing 20-22 g derived from accredited animal facility localized at Medical College of Jagiellonian University were group-housed for 3-4 day period in polycarbonate Makrolon type 3 cages (dimensions 26.5 x 15 x 42 cm) in an environmentally controlled, experimental room (ambient temperature 22 - 20C ; relative humidity 50-60%; 12:12 light:dark cycle, lights on at 8:00), in groups of 15. Standard laboratory food (Morigram, Agropol) and filtered water were freely available. On the day before experiments the equipment produces "white noise" was turned on for 30 minutes and mice or rats were weighted exact to 1 g. Animals were assigned randomly to treatment groups. All the experiments were performed by two observers unaware of the treatment applied between 9:00 and 14:00 on separate groups of animals. All animals were used only once and were killed immediately after the experiment. All the experimental procedures were approved by the IV Local Bioethics Commission in Warszawa.

### d-Amphetamine-induced locomotor hyperactivity

The locomotor activity was recorded with an Opto M3 multi-channel activity monitor (MultiDevice Software v.1.3, Columbus Instruments). Mice were individually placed in plastic cages (22 x 12 x 13 cm) and then the crossings of each channel (ambulation) were counted during 2 h with data recording every 5 minutes. The cages were cleaned up with 70% ethanol after each mouse. Drugs d-Amphetamine (s.c.) and Compound 60 (i.p.) were administered immediately before the test to 10 mice per treatment group.

**Table 11: Results of d-amphetamine-induced locomotor hyperactivity test**

| **Compound** | **MED* [mg/kg]** |
|---|---|
| **60** | 10 |

| | |
|---|---|
| * MED = Minimum Effective Dose | |

The exemplary test compound turned out to be active in procedure indicative of efficacy in antipsychotic activity.

## Claims

1. A compound of the general formula (I): 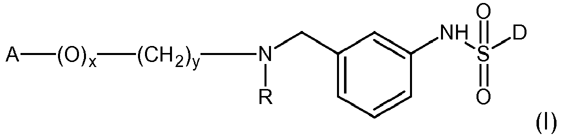 wherein
A represents:
- phenyl unsubstituted or substituted with one substituent selected from the group consisting of halogen atom, C₁-C₃-alkyl, C₁-C₃-alkyloxy, OH and phenyl; or
- 9- or 10-membered bicyclic group, linked to -(O)ₓ-(CH₂)_{y}- through one of its aromatic carbon atoms, consisting of benzene ring fused with:
- 5-membered heteroaromatic ring containing 1 or 2 heteroatoms independently selected from the group consisting of N and O, wherein if such heteroaromatic ring contains 2 heteroatoms then at least one is N and, and wherein such bicyclic group is unsubstituted or substituted with halogen atom; or
- 5- or 6-membered non-aromatic heterocyclic ring having 1 or 2 atoms of O, and wherein heterocyclic ring is unsubstituted or substituted with one or more C₁-C₃-alkyls;
D represents a group selected from:
- phenyl unsubstituted or substituted with one or two substituents independently selected from a group consisting of C₁-C₄-alkyl, C₁-C₃-alkyloxy, halogeno-C₁-C₃-alkyl, halogeno-C₁₋C₃-alkyloxy, halogen atom, -CN, and phenyl;
- naphthyl unsubstituted or substituted with one halogen atom;
- thiophene unsubstituted or substituted with one or two substituents independently selected from the group consisting of C₁-C₃-alkyl, halogen atom, and 5-membered heteroaromatic ring having 1 or 2 heteroatoms independently selected from N and O, wherein if such heteroaromatic ring contains 2 heteroatoms, then at least one is N;
- bicyclic group consisting of imidazole ring fused with 5-membered non-aromatic carbocyclic ring, linked to sulphonamide moiety through one of its aromatic carbon atoms;
- bicyclic group consisting of benzene ring fused with 5-membered heteroaromatic ring, having 1 or 2 heteroatoms independently selected from the group consisting of N, O and S, wherein if such heteroaromatic ring contains 2 heteroatoms then at least one is N and wherein the bicyclic group is unsubstituted or substituted with one or more substituents independently selected from the group consisting of C₁-C₃-alkyl and halogen atom, and linked to sulphonamide moiety through one of its aromatic carbon atoms; and
- bicyclic group consisting of benzene ring fused with 5- or 6-membered non-aromatic heterocyclic ring having 1 or 2 heteroatoms independently selected from the group consisting of N and O, unsubstituted or substituted with one =O, and linked to sulphonamide moiety through one of carbon atoms of benzene ring;
R represents H or -CH₃;
x is 0 or 1;
y is 2 or 3;
and pharmaceutically acceptable salts and solvates thereof,
with the provisos that
- if x is 0 and y is 2, then D is naphthyl unsubstituted or substituted with one halogen atom, and
- if R represents -CH₃, then A is not unsubstituted or substituted phenyl.

2. The compound according to claim 1, wherein A represents phenyl unsubstituted or substituted with one substituent selected from the group consisting of halogen atom, C₁-C₃-alkyl, C₁-C₃-alkyloxy, OH and phenyl.

3. The compound according to claim 1, wherein A represents 9- or 10-membered bicyclic group, linked to -(O)ₓ-(CH₂)_{y}- through one of its aromatic carbon atoms, consisting of benzene ring fused with 5-membered heteroaromatic ring containing 1 or 2 heteroatoms independently selected from the group consisting of N and O, wherein if such heteroaromatic ring contains 2 heteroatoms, then at least one is N, and wherein such bicyclic group is unsubstituted or substituted with halogen atom.

4. The compound according to claim 1, wherein A represents 9- or 10-membered bicyclic group, linked to -(O)ₓ-(CH₂)_{y}- through one of its aromatic carbon atoms, consisting of benzene ring fused with 5- or 6-membered non-aromatic heterocyclic ring having 1 or 2 O atoms, wherein heterocyclic ring is unsubstituted or substituted with one or more C₁-C₃-alkyls.

5. The compound according to any one of claims 1-4, wherein D represents phenyl unsubstituted or substituted with one or two substituents independently selected from the group consisting of C₁-C₄-alkyl, C₁-C₃-alkyloxy, halogeno-C₁-C₃-alkyl, halogeno-C₁-C₃-alkyloxy, halogen atom, -CN, and phenyl.

6. The compound according to any one of claims 1-4, wherein D represents naphthyl unsubstituted or substituted with one halogen atom.

7. The compound according to any one of claims 1-4, wherein D represents thiophene unsubstituted or substituted with one or two substituents independently selected from the group consisting of C₁-C₃-alkyl, halogen atom and 5-membered heteroaromatic ring having 1 or 2 heteroatoms independently selected from N and O, wherein if such heteroaromatic ring contains 2 heteroatoms, then at least one is N.

8. The compound according to any one of claims 1-4, wherein D represents bicyclic group consisting of imidazole ring fused with 5-membered non-aromatic carbocyclic ring, linked to sulphonamide moiety through one of its aromatic carbon atoms.

9. The compound according to any one of claims 1-4, wherein D represents bicyclic group consisting of a benzene ring fused with 5-membered heteroaromatic ring, having 1 or 2 heteroatoms independently selected from the group consisting of N, O and S, wherein if such heteroaromatic ring contains 2 heteroatoms, then at least one is N and wherein the bicyclic group is unsubstituted or substituted with one or more substituents independently selected from a group consisting of C₁-C₃-alkyl and halogen atom, and is linked to sulphonamide moiety through one of its aromatic carbon atoms.

10. The compound according to any one of claims 1-4, wherein D represents bicyclic group consisting of benzene ring fused with 5- or 6-membered non-aromatic heterocyclic ring having 1 or 2 heteroatoms independently selected from the group consisting of N and O, unsubstituted or substituted with one =O, and linked to sulphonamide moiety through one of carbon atoms of benzene ring.

11. The compound according to any one of claims 1-10, wherein R represents H, having the general formula (IA):

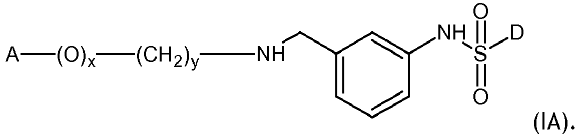

12. The compound according to any one of claims 1-10, wherein R represents -CH₃, having the general formula (IB):

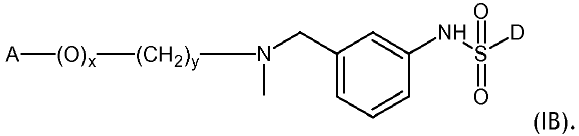

13. The compound according to any one of claims 1-12, wherein x is 1 and y is 2 or x is 0 and y is 3.

14. The compound according to claim 13, wherein x is 1 and y is 2.

15. The compound according to claim 13, wherein x is 0 and y is 3.

16. The compounds according to claim 6, wherein x is 0 and y is 2.

17. The compound according to claim 1 selected from the group consisting of the following compounds:
N-[3-[(phenylethylamino)methyl]phenyl]naphthalene-2-sulphonamide,
N-[3-[(2-phenoxyethylamino)methyl]phenyl]naphthalene-2-sulphonamide,
N-[3-[(3-phenylpropylamino)methyl]phenyl]naphthalene-2-sulphonamide,
N-[3-[[2-(3-fluorophenoxy)ethylamino]methyl]phenyl]naphthalene-2-sulphonamide,
N-[3-[[3-(3-fluorophenoxy)propylamino]methyl]phenyl]naphthalene-2-sulphonamide,
N-[3-[[2-(3-chlorophenyl)ethylamino]methyl]phenyl]naphthalene-2-sulphonamide,
N-[3-[[2-(3-chlorophenoxy)ethylamino]methyl]phenyl]naphthalene-2-sulphonamide,
N-[3-[[2-(3-methylphenyl)ethylamino]methyl]phenyl]naphthalene-2-sulphonamide,
N-[3-[[2-(2-methoxyphenyl)ethylamino]methyl]phenyl]naphthalene-2-sulphonamide,
N-[3-[[2-(2-methoxyphenoxy)ethylamino]methyl]phenyl]naphthalene-2-sulphonamide,
N-[3-[[2-(3-methoxyphenyl)ethylamino]methyl]phenyl]naphthalene-2-sulphonamide,
N-[3-[[2-(2,3-dihydro-1,4-benzodioxin-5-yloxy)ethylamino]methyl]phenyl]-4-propyl-benzenesulphonamide,
N-[3-[[2-(3-methoxyphenoxy)ethylamino]methyl]phenyl]naphthalene-2-sulphonamide,
N-[3-[[2-(4-methoxyphenyl)ethylamino]methyl]phenyl]naphthalene-2-sulphonamide,
N-[3-[[2-(4-methoxyphenoxy)ethylamino]methyl]phenyl]naphthalene-2-sulphonamide,
N-[3-[[3-(4-methoxyphenyl)propylamino]methyl]phenyl]naphthalene-2-sulphonamide,
N-[3-[[2-(3-hydroxyphenyl)ethylamino]methyl]phenyl]naphthalene-2-sulphonamide,
N-[3-[[2-(2,3-dihydro-1,4-benzodioxin-5-yloxy)ethylamino]methyl]phenyl]benzenesulphonamide,
N-[3-[[2-(2,3-dihydro-1,4-benzodioxin-5-yloxy)ethylamino]methyl]phenyl]-3-fluoro-benzenesulphonamide,
N-[3-[[2-(2,3-dihydro-1,4-benzodioxin-5-yloxy)ethylamino]methyl]phenyl]-4-fluoro-benzenesulphonamide,
4-chloro-N-[3-[[2-(2,3-dihydro-1,4-benzodioxin-5-yloxy)ethylamino]methyl]phenyl]-benzenesulphonamide,
N-[3-[[2-(2,3-dihydro-1,4-benzodioxin-5-yloxy)ethylamino]methyl]phenyl]-4-iodo-benzenesulphonamide,
N-[3-[[2-(2,3-dihydro-1,4-benzodioxin-5-yloxy)ethylamino]methyl]phenyl]-3-methyl-benzenesulphonamide,
N-[3-[[2-(2,3-dihydro-1,4-benzodioxin-5-yloxy)ethylamino]methyl]phenyl]-3-(trifluoromethyl)benzenesulphonamide,
N-[3-[[2-(2,3-dihydro-1,4-benzodioxin-5-yloxy)ethylamino]methyl]phenyl]-3-methoxy-benzenesulphonamide,
N-[3-[[2-(2,3-dihydro-1,4-benzodioxin-5-yloxy)ethylamino]methyl]phenyl]-4-methoxy-benzenesulphonamide,
N-[3-[[2-(2,3-dihydro-1,4-benzodioxin-5-yloxy)ethylamino]methyl]phenyl]-4-(trifluoro-methoxy)benzenesulphonamide,
3-cyano-N-[3-[[2-(2,3-dihydro-1,4-benzodioxin-5-yloxy)ethylamino]methyl]phenyl]-benzenesulphonamide,
4-cyano-N-[3-[[2-(2,3-dihydro-1,4-benzodioxin-5-yloxy)ethylamino]methyl]phenyl]-benzenesulphonamide,
N-[3-[[2-(2,3-dihydro-1,4-benzodioxin-5-yloxy)ethylamino]methyl]phenyl]-4-phenyl-benzenesulphonamide,
N-[3-[[2-(2,3-dihydro-1,4-benzodioxin-5-yloxy)ethylamino]methyl]phenyl]thiophene-2-sulphonamide,
N-[3-[[2-(2,3-dihydro-1,4-benzodioxin-5-yloxy)ethylamino]methyl]phenyl]thiophene-3-sulphonamide,
N-[3-[[2-(2,3-dihydro-1,4-benzodioxin-5-yloxy)ethylamino]methyl]phenyl]-2,5-dimethyl-thiophene-3-sulphonamide,
N-[3-[[2-(2,3-dihydro-1,4-benzodioxin-5-yloxy)ethylamino]methyl]phenyl]-5-isoxazol-5-yl-thiophene-2-sulphonamide,
N-[3-[[2-(2,3-dihydro-1,4-benzodioxin-5-yloxy)ethylamino]methyl]phenyl]naphthalene-1-sulphonamide,
N-[3-[[2-(2,3-dihydro-1,4-benzodioxin-5-yloxy)ethylamino]methyl]phenyl]naphthalene-2-sulphonamide,
6-chloro-N-[3-[[2-(2,3-dihydro-1,4-benzodioxin-5-yloxy)ethylamino]methyl]phenyl]-naphthalene-2-sulphonamide,
N-[3-[[2-(2,3-dihydro-1,4-benzodioxin-5-yloxy)ethylamino]methyl]phenyl]-1,3-benzo-dioxole-5-sulphonamide,
N-[3-[[2-(2,3-dihydro-1,4-benzodioxin-5-yloxy)ethylamino]methyl]phenyl]-6,7-dihydro-5H-pyrrole[1,2-a]imidazole-3-sulphonamide,
N-[3-[[2-(2,3-dihydro-1,4-benzodioxin-5-yloxy)ethylamino]methyl]phenyl]-1-methylindole-4-sulphonamide,
N-[3-[[2-(2,3-dihydro-1,4-benzodioxin-5-yloxy)ethylamino]methyl]phenyl]-1-methylindole-5-sulphonamide,
N-[3-[[2-(2,3-dihydro-1,4-benzodioxin-5-yloxy)ethylamino]methyl]phenyl]benzofuran-2-sulphonamide,
N-[3-[[2-(2,3-dihydro-1,4-benzodioxin-5-yloxy)ethylamino]methyl]phenyl]benzo-thiophene-3-sulphonamide,
N-[3-[[2-(2,3-dihydro-1,4-benzodioxin-5-yloxy)ethylamino]methyl]phenyl]-5-fluoro-3-methyl-benzothiophene-2-sulphonamide,
3-chloro-N-[3-[[2-(2,3-dihydro-1,4-benzodioxin-5-yloxy)ethylamino]methyl]phenyl]-4-fluorobenzenesulphonamide,
N-[3-[[2-[(2,2-dimethyl-3H-benzofuran-7-yl)oxy]ethylamino]methyl]phenyl]-3-methyl-benzenesu lphonamide,
N-[3-[[2-[(2,2-dimethyl-3H-benzofuran-7-yl)oxy]ethylamino]methyl]phenyl]naphthalene-1-sulphonamide,
N-[3-[[2-[(2,2-dimethyl-3H-benzofuran-7-yl)oxy]ethylamino]methyl]phenyl]naphthalene-2-sulphonamide,
N-[3-[[2-(1H-indol-4-yloxy)ethylamino]methyl]phenyl]-3-methylbenzenesulphonamide,
N-[3-[[2-(1H-indol-4-yloxy)ethylamino]methyl]phenyl]-4-phenylbenzenesulphonamide,
N-[3-[[2-(1H-indol-4-yloxy)ethylamino]methyl]phenyl]naphthalene-1-sulphonamide,
N-[3-[[2-(1H-indol-4-yloxy)ethylamino]methyl]phenyl]naphthalene-2-sulphonamide,
N-[3-[[3-(5-fluoro-1,2-benzoxazol-3-yl)propylamino]methyl]phenyl]-3-methylbenzene-sulphonamide,
N-[3-[[3-(6-fluoro-1,2-benzoxazol-3-yl)propylamino]methyl]phenyl]naphthalene-1-sulphonamide,
N-[3-[[3-(6-fluoro-1,2-benzoxazol-3-yl)propylamino]methyl]phenyl]naphthalene-2-sulphonamide,
N-[3-[[2-(3-methylphenoxy)ethylamino]methyl]phenyl]naphthalene-2-sulphonamide,
N-[3-[[2-(3-hydroxyphenoxy)ethylamino]methyl]phenyl]naphthalene-2-sulphonamide,
N-[3-[[2-(1H-indol-4-yloxy)ethylamino]methyl]phenyl]-4-propyl-benzenesulphonamide,
N-[3-[[2-(1H-indol-4-yloxy)ethylamino]methyl]phenyl]thiophene-2-sulphonamide,
N-[3-[[2-(1H-indol-4-yloxy)ethylamino]methyl]phenyl]thiophene-3-sulphonamide,
N-[3-[[2-(1H-indol-4-yloxy)ethylamino]methyl]phenyl]-2,5-dimethyl-thiophene-3-sulphonamide,
N-[3-[[2-(2,3-dihydro-1,4-benzodioxin-5-yloxy)ethyl-methyl-amino]methyl]phenyl]-benzenesulphonamide,
N-[3-[[2-[(2,2-dimethyl-3H-benzofuran-7-yl)oxy]ethyl-methyl-amino]methyl]phenyl]-benzenesulphonamide,
N-[3-[[2-(1H-indol-4-yloxy)ethylmethylamino]methyl]phenyl]benzenesulphonamide,
N-[3-[[3-(6-fluoro-1,2-benzoxazol-3-yl)propylmethylamino]methyl]phenyl]benzenesulphonamide,
4-fluoro-N-[3-[[3-(6-fluoro-1,2-benzoxazol-3-yl)propylmethylamino]methyl]phenyl]-benzenesulphonamide,
3,4-difluoro-N-[3-[[3-(6-fluoro-1,2-benzoxazol-3-yl)propylmethylamino]methyl]phenyl]-benzenesulphonamide,
3-chloro-N-[3-[[3-(6-fluoro-1,2-benzoxazol-3-yl)propylmethylamino]methyl]phenyl]-benzenesulphonamide,
4-chloro-N-[3-[[3-(6-fluoro-1,2-benzoxazol-3-yl)propylmethylamino]methyl]phenyl]-benzenesulphonamide,
3,4-dichloro-N-[3-[[3-(6-fluoro-1,2-benzoxazol-3-yl)propylmethylamino]methyl]phenyl]-benzenesulphonamide,
3-bromo-N-[3-[[3-(6-fluoro-1,2-benzoxazol-3-yl)propylmethylamino]methyl]phenyl]-benzenesulphonamide,
4-bromo-N-[3-[[3-(6-fluoro-1,2-benzoxazol-3-yl)propylmethylamino]methyl]phenyl]-benzenesulphonamide,
N-[3-[[3-(6-fluoro-1,2-benzoxazol-3-yl)propylmethylamino]methyl]phenyl]-4-iodo-benzenesulphonamide,
N-[3-[[3-(6-fluoro-1,2-benzoxazol-3-yl)propylmethylamino]methyl]phenyl]-3-methyl-benzenesulphonamide,
N-[3-[[3-(6-fluoro-1,2-benzoxazol-3-yl)propylmethylamino]methyl]phenyl]-4-methyl-benzenesulphonamide,
N-[3-[[3-(6-fluoro-1,2-benzoxazol-3-yl)propylmethylamino]methyl]phenyl]-4-propyl-benzenesulphonamide,
4-tert-butyl-N-[3-[[3-(6-fluoro-1,2-benzoxazol-3-yl)propylmethylamino]methyl]phenyl]-benzenesulphonamide,
N-[3-[[3-(6-fluoro-1,2-benzoxazol-3-yl)propylmethylamino]methyl]phenyl]-3-(trifluoro-methyl)benzenesulphonamide,
N-[3-[[3-(6-fluoro-1,2-benzoxazol-3-yl)propylmethylamino]methyl]phenyl]-4-(trifluoromethyl)benzenesulphonamide,
N-[3-[[3-(6-fluoro-1,2-benzoxazol-3-yl)propylmethylamino]methyl]phenyl]-3-methoxy-benzenesulphonamide,
N-[3-[[3-(6-fluoro-1,2-benzoxazol-3-yl)propylmethylamino]methyl]phenyl]-4-methoxy-benzenesulphonamide,
N-[3-[[3-(6-fluoro-1,2-benzoxazol-3-yl)propylmethylamino]methyl]phenyl]-4-(trifluoro-methoxy)benzenesulphonamide,
3-cyano-N-[3-[[3-(6-fluoro-1,2-benzoxazol-3-yl)propylmethylamino]methyl]phenyl]-benzenesulphonamide,
N-[3-[[3-(6-fluoro-1,2-benzoxazol-3-yl)propylmethylamino]methyl]phenyl]-4-phenyl-benzenesulphonamide,
N-[3-[[3-(6-fluoro-1,2-benzoxazol-3-yl)propylmethylamino]methyl]phenyl]thiophene-2-sulphonamide,
N-[3-[[3-(6-fluoro-1,2-benzoxazol-3-yl)propylmethylamino]methyl]phenyl]thiophene-3-sulphonamide,
N-[3-[[3-(6-fluoro-1,2-benzoxazol-3-yl)propylmethylamino]methyl]phenyl]-2,5-dimethyl-thiophene-3-sulphonamide,
N-[3-[[3-(6-fluoro-1,2-benzoxazol-3-yl)propylmethylamino]methyl]phenyl]-5-isoxazol-5-yl-thiophene-2-sulphonamide,
N-[3-[[3-(6-fluoro-1,2-benzoxazol-3-yl)propylmethylamino]methyl]phenyl]naphthalene-1-sulphonamide,
N-[3-[[3-(6-fluoro-1,2-benzoxazol-3-yl)propylmethylamino]methyl]phenyl]naphthalene-2-sulphonamide,
6-chloro-N-[3-[[3-(6-fluoro-1,2-benzoxazol-3-yl)propylmethylamino]methyl]phenyl]-naphthalene-2-sulphonamide,
N-[3-[[3-(6-fluoro-1,2-benzoxazol-3-yl)propylmethylamino]methyl]phenyl]-2,3-dihydro-1,4-benzodioxino-6-sulphonamide,
N-[3-[[3-(6-fluoro-1,2-benzoxazol-3-yl)propylmethylamino]methyl]phenyl]-1,3-benzo-dioxole-5-sulphonamide,
N-[3-[[3-(6-fluoro-1,2-benzoxazol-3-yl)propylmethylamino]methyl]phenyl]-2-okso-indolino-5-sulphonamide,
N-[3-[[3-(6-fluoro-1,2-benzoxazol-3-yl)propylmethylamino]methyl]phenyl]-6,7-dihydro-5H-pyrrole[1,2-a]imidazole-3-sulphonamide,
N-[3-[[3-(6-fluoro-1,2-benzoxazol-3-yl)propylmethylamino]methyl]phenyl]-1-methylindole-4-sulphonamide,
N-[3-[[3-(6-fluoro-1,2-benzoxazol-3-yl)propylmethylamino]methyl]phenyl]-1-methylindole-5-sulphonamide,
N-[3-[[3-(6-fluoro-1,2-benzoxazol-3-yl)propylmethylamino]methyl]phenyl]-1,3-benzo-thiazole-4-sulphonamide,
N-[3-[[3-(6-fluoro-1,2-benzoxazol-3-yl)propylmethylamino]methyl]phenyl]-1,3-benzo-thiazole-5-sulphonamide,
N-[3-[[3-(6-fluoro-1,2-benzoxazol-3-yl)propylmethylamino]methyl]phenyl]benzofuran-2-sulphonamide,
N-[3-[[3-(6-fluoro-1,2-benzoxazol-3-yl)propylmethylamino]methyl]phenyl]benzo-thiophene-3-sulphonamide,
5-fluoro-N-[3-[[3-(6-fluoro-1,2-benzoxazol-3-yl)propylmethylamino]methyl]phenyl]-3-methyl-benzothiophene-2-sulphonamide,
5-chloro-N-[3-[[3-(6-fluoro-1,2-benzoxazol-3-yl)propylmethylamino]methyl]phenyl]-3-methyl-benzothiophene-2-sulphonamide,
3-chloro-4-fluoro-N-[3-[[3-(6-fluoro-1,2-benzoxazol-3-yl)propylmethylamino]methyl]-phenyl]benzenesulphonamide,
and pharmaceutically acceptable salts and solvates thereof.

18. The compounds of formula (I) as defined in any one of claims 1-17 for use as a medicament.

19. Pharmaceutical composition comprising a compound of formula (I) as defined in any one of claims 1-17 as an active ingredient in combination with pharmaceutically acceptable carrier(s) and/or excipient(s).

20. The compound of formula (I) as defined in any one of claims 1-17 for use in a method of treatment and/or prevention of disorders of the central nervous system.

21. The compound for use according to claim 20 wherein the disorder of the central nervous system is selected from the group consisting of schizophrenia, schizoaffective disorders, schizophreniform disorders, delusional syndromes and other psychotic conditions related and not related to taking psychoactive substances, affective disorder, bipolar disorder, mania, depression, anxiety disorders of various etiology, stress reactions, conciousness disorders, coma, delirium of alcoholic and other etiology, aggression, psychomotor agitation and other conduct disorders, sleep disorders of various etiology, withdrawal syndromes of various etiology, addiction, pain syndromes of various etiology, intoxication with psychoactive substances, cerebral circulatory disorders of various etiology, psychosomatic disorders of various etiology, conversion disorders, dissociative disorders, urination disorders, autism and other developmental disorders, including nocturia, stuttering, tics, cognitive disorders of various types, like Alzheimer's disease, psychopathological symptoms and neurological disorders in the course of other diseases of the central and peripheral nervous systems.

## Patentansprüche

1. Verbindung der allgemeinen Formel (I): 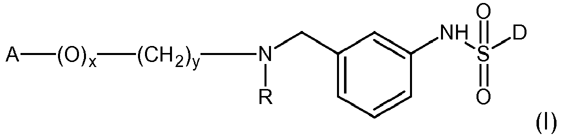 worin
A:
- Phenyl, die nicht substituiert oder mit einem Substituenten ausgewählt aus der Gruppe bestehend aus Halogenatom, C1-C3-Alkyl, C1-C3-Alkyloxy, OH und Phenyl, einmals substituiert ist; oder
- 9- oder 10-gliedrige bicyclische Gruppe, die mit -(O)ₓ₋(CH₂)_{y}- durch einen ihrer aromatischen Kohlenstoffatomen verknüpft ist, bestehend aus Benzolring kondensiert mit:
- 5-gliedrigem heteroaromatischem Ring, enthaltend 1 oder 2 Heteroatome unabhängig ausgewählt aus der Gruppe bestehend aus N und O, wobei, wenn ein solcher heteroaromatischer Ring 2 Heteroatome enthält, dann wenigstens eines N ist, und wobei solche bicyclische Gruppe nicht substituiert oder mit Halogenatom substituiert ist; oder
- 5- oder 6-gliedrigem nicht-aromatischem heterocyclischem Ring, enthaltend 1 oder 2 O Atomen, und wobei der heterocyclischer Ring nicht substituiert oder mit einem oder mehreren C1-C3-Alkylen substituiert ist;
darstellt:
D eine Gruppe darstellt, ausgewählt aus:
- Phenyl, die nicht substituiert oder mit einem oder zwei Substituenten, unabhängig ausgewählt aus einer Gruppe bestehend aus C1-C4-Alkyl, C1-C3-Alkyloxy, Halogen-C1-C3-Alkyl, Halogen-C1-C3-Alkyloxy, Halogenatom, -CN und Phenyl, substituiert ist;
- Naphthyl, die nicht substituiert oder mit einem Halogenatom einmals substituiert ist;
- Thiophen, die nicht substituiert oder mit einem oder zwei Substituenten, unabhängig ausgewählt aus der Gruppe bestehend aus C1-C3-Alkyl, Halogenatom, und 5-gliedrigem heteroaromatischem Ring enthaltend 1 oder 2 Heteroatome, unabhängig ausgewählt aus N und O, substituiert ist, wobei, wenn ein solcher heteroaromatischer Ring 2 Heteroatome enthält, dann wenigstens eines N ist;
- bicyclische Gruppe, bestehend aus Imidazolring kondensiert mit 5-gliedrigem nicht-aromatischem carbocyclischem Ring, die mit Sulfonamideinheit durch einen ihrer aromatische Kohlenstoffatome verknüpft ist;
- bicyclische Gruppe, bestehend aus Benzolring kondensiert mit 5-gliedrigem heteroaromatischem Ring enthaltend 1 oder 2 Heteroatome, unabhängig ausgewählt aus der Gruppe, bestehend aus N, 0 und S, wobei, wenn ein solcher heteroaromatischer Ring 2 Heteroatome enthält, dann wenigstens eines N ist, und wobei die bicyclische Gruppe nicht substituiert oder mit einem oder mehreren Substituenten unabhängig ausgewählt aus der Gruppe bestehend aus C1-3-Alkyl und Halogenatom substituiert ist, die mit Sulfonamideinheit durch einen ihrer aromatische Kohlenstoffatome verknüpft ist; und
- bicyclische Gruppe, bestehend aus Benzolring kondensiert mit 5- oder 6-gliedrigem nicht-aromatischem heterocyclischem Ring enthaltend 1 oder 2 Heteroatome, unabhängig ausgewählt aus der Gruppe, bestehend aus N und O, nicht substituiert oder mit =O einmals substituiert, die mit Sulfonamideinheit durch einen ihrer aromatische Kohlenstoffatome verknüpft ist;
R H oder -CH₃ darstellt;
x 0 oder 1 ist;
y 2 oder 3 ist;
und pharmazeutisch verträgliche Salze und Solvate davon,
mit den Maßgaben, dass
- wenn x 0 ist und y 2 ist, dann D nicht substituiert oder mit einem Halogenatom einmals substituiert Naphthyl darstellt, und
- wenn R -CH₃ darstellt, dann A nicht substituiert oder substituiert Phenyl darstellt.

2. Verbindung nach Anspruch 1, wobei A Phenyl, die nicht substituiert oder mit einem Substituenten ausgewählt aus der Gruppe bestehend aus Halogenatom, C1-C3-Alkyl, C1-C3-Alkyloxy, OH und Phenyl einmals substituiert ist, darstellt.

3. Verbindung nach Anspruch 1, wobei A 9- oder 10-gliedrige bicyclische Gruppe, die mit -(O)ₓ-(CH₂)_{y}- durch einen ihrer aromatischen Kohlenstoffatomen verknüpft ist, bestehend aus Benzolring kondensiert mit 5-gliedrigem heteroaromatischem Ring enthaltend 1 oder 2 Heteroatome, unabhängig ausgewählt aus der Gruppe bestehend aus N und O, wobei, wenn ein solcher heteroaromatischer Ring 2 Heteroatome enthält, dann wenigstens eines N ist, und wobei solche bicyclische Gruppe nicht substituiert oder mit Halogenatom substituiert ist, darstellt.

4. Verbindung nach Anspruch 1, wobei A 9- oder 10-gliedrige bicyclische Gruppe, die mit -(O)ₓ-(CH₂)_{y}- durch einen ihrer aromatischen Kohlenstoffatomen verknüpft ist, bestehend aus Benzolring kondensiert mit 5- oder 6-gliedrigem nicht-aromatischem heterocyclischem Ring enthaltend 1 oder 2 O Atomen, und wobei der heterocyclischer Ring nicht substituiert oder mit einem oder mehreren C1-C3-Alkylen substituiert ist, darstellt.

5. Verbindung nach einem der Ansprüche 1-4, wobei D Phenyl, die nicht substituiert oder mit einem oder zwei Substituenten, unabhängig ausgewählt aus einer Gruppe bestehend aus C1-C 4-Alkyl, C1-C3-Alkyloxy, Halogen-C1-C3-Alkyl, Halogen-C1-C3-Alkyloxy, Halogenatom, -CN und Phenyl, substituiert ist, darstellt.

6. Verbindung nach einem der Ansprüche 1-4, wobei D Naphthyl, die nicht substituiert oder mit einem Halogenatom einmals substituiert ist, darstellt.

7. Verbindung nach einem der Ansprüche 1-4, wobei D Thiophen, die nicht substituiert oder mit einem oder zwei Substituenten, unabhängig ausgewählt aus der Gruppe bestehend aus C1-C3-Alkyl, Halogenatom, und 5-gliedrigem heteroaromatischem Ring enthaltend 1 oder 2 Heteroatome, unabhängig ausgewählt aus N und O, substituiert ist, wobei, wenn ein solcher heteroaromatischer Ring 2 Heteroatome enthält, dann wenigstens eines N ist, darstellt.

8. Verbindung nach einem der Ansprüche 1-4, wobei D bicyclische Gruppe, bestehend aus Imidazolring kondensiert mit 5-gliedrigem nicht-aromatischem carbocyclischem Ring, die mit Sulfonamideinheit durch einen ihrer aromatische Kohlenstoffatome verknüpft ist, darstellt.

9. Verbindung nach einem der Ansprüche 1-4, wobei D bicyclische Gruppe, bestehend aus Benzolring kondensiert mit 5-gliedrigen heteroaromatischen Ring enthaltend 1 oder 2 Heteroatome, unabhängig ausgewählt aus der Gruppe bestehend aus N, O und S, wobei, wenn ein solcher heteroaromatischer Ring 2 Heteroatome enthält, dann wenigstens eines N ist, und wobei die bicyclische Gruppe nicht substituiert oder mit einem oder mehreren Substituenten unabhängig ausgewählt aus der Gruppe bestehend aus C1-3-Alkyl und Halogenatom substituiert ist, die mit Sulfonamideinheit durch einen ihrer aromatische Kohlenstoffatome verknüpft ist, darstellt.

10. Verbindung nach einem der Ansprüche 1-4, wobei D bicyclische Gruppe, bestehend aus Benzolring kondensiert mit 5- oder 6-gliedrigem nicht-aromatischem heterocyclischem Ring enthaltend 1 oder 2 Heteroatome, unabhängig ausgewählt aus der Gruppe bestehend aus N und O, nicht substituiert oder mit ein =O einmals substituiert, die mit Sulfonamideinheit durch einen ihrer aromatische Kohlenstoffatome verknüpft ist, darstellt.

11. Verbindung nach einem der Ansprüche 1-10, wobei R H darstellt, die der allgemeinen Formel (IA) hat:

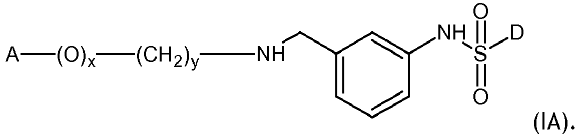

12. Verbindung nach einem der Ansprüche 1-10, wobei R -CH₃ darstellt, die der allgemeinen Formel (IB) hat:

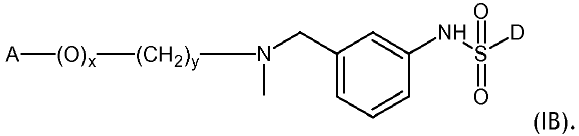

13. Verbindung nach einem der Ansprüche 1-12, wobei x 1 ist und y 2 ist oder x 0 ist und y 3 ist.

14. Verbindung nach Anspruch 13, wobei x 1 ist und y 2 ist.

15. Verbindung nach Anspruch 13, wobei x 0 ist und y 3 ist.

16. Verbindung nach Anspruch 6, wobei x 0 ist und y 2 ist.

17. Verbindung nach Anspruch 1, die aus der Gruppe bestehend aus den folgenden ausgewählt ist:
N-[3-[(Phenylethylamino)methyl]phenyl]naphthalene-2-sulfonamid,
N-[3-[(2-Phenoxyethylamino)methyl]phenyl]naphthalene-2-sulfonamid,
N-[3-[(3-Phenylpropylamino)methyl]phenyl]naphthalene-2-sulfonamid,
N-[3-[[2-(3-Fluorophenoxy)ethylamino]methyl]phenyl]naphthalene-2-sulfonamid,
N-[3-[[3-(3-Fluorophenoxy)propylamino]methyl]phenyl]naphthalene-2-sulfonamid,
N-[3-[[2-(3-Chlorophenyl)ethylamino]methyl]phenyl]naphthalene-2-sulfonamid,
N-[3-[[2-(3-Chlorophenoxy)ethylamino]methyl]phenyl]naphthalene-2-sulfonamid,
N-[3-[[2-(3-Methylphenyl)ethylamino]methyl]phenyl]naphthalene-2-sulfonamid,
N-[3-[[2-(2-Methoxyphenyl)ethylamino]methyl]phenyl]naphthalene-2-sulfonamid,
N-[3-[[2-(2-Methoxyphenoxy)ethylamino]methyl]phenyl]naphthalene-2-sulfonamid,
N-[3-[[2-(3-Methoxyphenyl)ethylamino]methyl]phenyl]naphthalene-2-sulfonamid,
N-[3-[[2-(2,3-Dihydro-1,4-benzodioxin-5-yloxy)ethylamino]methyl]phenyl]-4-propyl-benzenesulfonamid,
N-[3-[[2-(3-Methoxyphenoxy)ethylamino]methyl]phenyl]naphthalene-2-sulfonamid,
N-[3-[[2-(4-Methoxyphenyl)ethylamino]methyl]phenyl]naphthalene-2-sulfonamid,
N-[3-[[2-(4-Methoxyphenoxy)ethylamino]methyl]phenyl]naphthalene-2-sulfonamid,
N-[3-[[3-(4-Methoxyphenyl)propylamino]methyl]phenyl]naphthalene-2-sulfonamid,
N-[3-[[2-(3-Hydroxyphenyl)ethylamino]methyl]phenyl]naphthalene-2-sulfonamid,
N-[3-[[2-(2,3-Dihydro-1,4-benzodioxin-5-yloxy)ethylamino]methyl]phenyl]benzene-sulfonamid,
N-[3-[[2-(2,3-Dihydro-1,4-benzodioxin-5-yloxy)ethylamino]methyl]phenyl]-3-fluoro-benzenesulfonamid,
N-[3-[[2-(2,3-Dihydro-1,4-benzodioxin-5-yloxy)ethylamino]methyl]phenyl]-4-fluoro-benzenesulfonamid,
4-Chloro-N-[3-[[2-(2,3-dihydro-1,4-benzodioxin-5-yloxy)ethylamino]methyl]phenyl]-benzenesulfonamid,
N-[3-[[2-(2,3-Dihydro-1,4-benzodioxin-5-yloxy)ethylamino]methyl]phenyl]-4-iodo-benzenesulfonamid,
N-[3-[[2-(2,3-Dihydro-1,4-benzodioxin-5-yloxy)ethylamino]methyl]phenyl]-3-methyl-benzenesulfonamid,
N-[3-[[2-(2,3-Dihydro-1,4-benzodioxin-5-yloxy)ethylamino]methyl]phenyl]-3-(trifluoro-methyl)benzenesulfonamid,
N-[3-[[2-(2,3-Dihydro-1,4-benzodioxin-5-yloxy)ethylamino]methyl]phenyl]-3-methoxy-benzenesulfonamid,
N-[3-[[2-(2,3-Dihydro-1,4-benzodioxin-5-yloxy)ethylamino]methyl]phenyl]-4-methoxy-benzenesulfonamid,
N-[3-[[2-(2,3-Dihydro-1,4-benzodioxin-5-yloxy)ethylamino]methyl]phenyl]-4-(trifluoro-methoxy)benzenesulfonamid,
3-Cyano-N-[3-[[2-(2,3-dihydro-1,4-benzodioxin-5-yloxy)ethylamino]methyl]phenyl]-benzenesulfonamid,
4-Cyano-N-[3-[[2-(2,3-dihydro-1,4-benzodioxin-5-yloxy)ethylamino]methyl]phenyl]-benzenesulfonamid,
N-[3-[[2-(2,3-Dihydro-1,4-benzodioxin-5-yloxy)ethylamino]methyl]phenyl]-4-phenyl-benzenesulfonamid,
N-[3-[[2-(2,3-Dihydro-1,4-benzodioxin-5-yloxy)ethylamino]methyl]phenyl]thiophene-2-sulfonamid,
N-[3-[[2-(2,3-Dihydro-1,4-benzodioxin-5-yloxy)ethylamino]methyl]phenyl]thiophene-3-sulfonamid,
N-[3-[[2-(2,3-Dihydro-1,4-benzodioxin-5-yloxy)ethylamino]methyl]phenyl]-2,5-dimethyl-thiophene-3-sulfonamid,
N-[3-[[2-(2,3-Dihydro-1,4-benzodioxin-5-yloxy)ethylamino]methyl]phenyl]-5-isoxazol-5-yl-thiophene-2-sulfonamid,
N-[3-[[2-(2,3-Dihydro-1,4-benzodioxin-5-yloxy)ethylamino]methyl]phenyl]naphthalene-1-sulfonamid,
N-[3-[[2-(2,3-Dihydro-1,4-benzodioxin-5-yloxy)ethylamino]methyl]phenyl]naphthalene-2-sulfonamid,
6-Chloro-N-[3-[[2-(2,3-dihydro-1,4-benzodioxin-5-yloxy)ethylamino]methyl]phenyl]-naphthalene-2-sulfonamid,
N-[3-[[2-(2,3-Dihydro-1,4-benzodioxin-5-yloxy)ethylamino]methyl]phenyl]-1,3-benzo-dioxole-5-sulfonamid,
N-[3-[[2-(2,3-Dihydro-1,4-benzodioxin-5-yloxy)ethylamino]methyl]phenyl]-6,7-dihydro-5H-pyrrole[1,2-a]imidazole-3-sulfonamid,
N-[3-[[2-(2,3-Dihydro-1,4-benzodioxin-5-yloxy)ethylamino]methyl]phenyl]-1-methyl-indole-4-sulfonamid,
N-[3-[[2-(2,3-Dihydro-1,4-benzodioxin-5-yloxy)ethylamino]methyl]phenyl]-1-methyl-indole-5-sulfonamid,
N-[3-[[2-(2,3-Dihydro-1,4-benzodioxin-5-yloxy)ethylamino]methyl]phenyl]benzofuran-2-sulfonamid,
N-[3-[[2-(2,3-Dihydro-1,4-benzodioxin-5-yloxy)ethylamino]methyl]phenyl]benzo-thiophene-3-sulfonamid,
N-[3-[[2-(2,3-Dihydro-1,4-benzodioxin-5-yloxy)ethylamino]methyl]phenyl]-5-fluoro-3-methyl-benzothiophene-2-sulfonamid,
3-Chloro-N-[3-[[2-(2,3-dihydro-1,4-benzodioxin-5-yloxy)ethylamino]methyl]phenyl]-4-fluorobenzenesulfonamid,
N-[3-[[2-[(2,2-Dimethyl-3H-benzofuran-7-yl)oxy]ethylamino]methyl]phenyl]-3-methyl-benzenesulfonamid,
N-[3-[[2-[(2,2-Dimethyl-3H-benzofuran-7-yl)oxy]ethylamino]methyl]phenyl]naphthalene-1-sulfonamid,
N-[3-[[2-[(2,2-Dimethyl-3H-benzofuran-7-yl)oxy]ethylamino]methyl]phenyl]naphthalene-2-sulfonamid,
N-[3-[[2-(1H-Indol-4-yloxy)ethylamino]methyl]phenyl]-3-methylbenzenesulfonamid,
N-[3-[[2-(1H-Indol-4-yloxy)ethylamino]methyl]phenyl]-4-phenylbenzenesulfonamid,
N-[3-[[2-(1H-Indol-4-yloxy)ethylamino]methyl]phenyl]naphthalene-1-sulfonamid,
N-[3-[[2-(1H-Indol-4-yloxy)ethylamino]methyl]phenyl]naphthalene-2-sulfonamid,
N-[3-[[3-(5-Fluoro-1,2-benzoxazol-3-yl)propylamino]methyl]phenyl]-3-methylbenzene-sulfonamid,
N-[3-[[3-(6-Fluoro-1,2-benzoxazol-3-yl)propylamino]methyl]phenyl]naphthalene-1-sulfonamid,
N-[3-[[3-(6-Fluoro-1,2-benzoxazol-3-yl)propylamino]methyl]phenyl]naphthalene-2-sulfonamid,
N-[3-[[2-(3-Methylphenoxy)ethylamino]methyl]phenyl]naphthalene-2-sulfonamid,
N-[3-[[2-(3-Hydroxyphenoxy)ethylamino]methyl]phenyl]naphthalene-2-sulfonamid,
N-[3-[[2-(1H-Indol-4-yloxy)ethylamino]methyl]phenyl]-4-propyl-benzenesulfonamid,
N-[3-[[2-(1H-Indol-4-yloxy)ethylamino]methyl]phenyl]thiophene-2-sulfonamid,
N-[3-[[2-(1H-Indol-4-yloxy)ethylamino]methyl]phenyl]thiophene-3-sulfonamid,
N-[3-[[2-(1H-Indol-4-yloxy)ethylamino]methyl]phenyl]-2,5-dimethyl-thiophene-3-sulfonamid,
N-[3-[[2-(2,3-Dihydro-1,4-benzodioxin-5-yloxy)ethyl-methyl-amino]methyl]phenyl]-benzenesulfonamid,
N-[3-[[2-[(2,2-Dimethyl-3H-benzofuran-7-yl)oxy]ethyl-methyl-amino]methyl]phenyl]-benzenesulfonamid,
N-[3-[[2-(1H-Indol-4-yloxy)ethylmethylamino]methyl]phenyl]benzenesulfonamid,
N-[3-[[3-(6-Fluoro-1,2-benzoxazol-3-yl)propylmethylamino]methyl]phenyl]benzene-sulfonamid,
4-Fluoro-N-[3-[[3-(6-fluoro-1,2-benzoxazol-3-yl)propylmethylamino]methyl]phenyl]-benzenesulfonamid,
3,4-Difluoro-N-[3-[[3-(6-fluoro-1,2-benzoxazol-3-yl)propylmethylamino]methyl]phenyl]-benzenesulfonamid,
3-Chloro-N-[3-[[3-(6-fluoro-1,2-benzoxazol-3-yl)propylmethylamino]methyl]phenyl]-benzenesulfonamid,
4-Chloro-N-[3-[[3-(6-fluoro-1,2-benzoxazol-3-yl)propylmethylamino]methyl]phenyl]-benzenesulfonamid,
3,4-Dichloro-N-[3-[[3-(6-fluoro-1,2-benzoxazol-3-yl)propylmethylamino]methyl]phenyl]-benzenesulfonamid,
3-Bromo-N-[3-[[3-(6-fluoro-1,2-benzoxazol-3-yl)propylmethylamino]methyl]phenyl]-benzenesulfonamid, 4-Bromo-N-[3-[[3-(6-fluoro-1,2-benzoxazol-3-yl)propylmethylamino]methyl]phenyl]-benzenesulfonamid, N-[3-[[3-(6-Fluoro-1,2-benzoxazol-3-yl)propylmethylamino]methyl]phenyl]-4-iodo-benzenesulfonamid, N-[3-[[3-(6-Fluoro-1,2-benzoxazol-3-yl)propylmethylamino]methyl]phenyl]-3-methyl-benzenesulfonamid, N-[3-[[3-(6-Fluoro-1,2-benzoxazol-3-yl)propylmethylamino]methyl]phenyl]-4-methyl-benzenesulfonamid, N-[3-[[3-(6-Fluoro-1,2-benzoxazol-3-yl)propylmethylamino]methyl]phenyl]-4-propyl-benzenesulfonamid, 4-tert-Butyl-N-[3-[[3-(6-fluoro-1,2-benzoxazol-3-yl)propylmethylamino]methyl]phenyl]-benzenesulfonamid, N-[3-[[3-(6-Fluoro-1,2-benzoxazol-3-yl)propylmethylamino]methyl]phenyl]-3-(trifluoro-methyl)benzenesulfonamid, N-[3-[[3-(6-Fluoro-1,2-benzoxazol-3-yl)propylmethylamino]methyl]phenyl]-4-(trifluoro-methyl)benzenesulfonamid, N-[3-[[3-(6-Fluoro-1,2-benzoxazol-3-yl)propylmethylamino]methyl]phenyl]-3-methoxy-benzenesulfonamid, N-[3-[[3-(6-Fluoro-1,2-benzoxazol-3-yl)propylmethylamino]methyl]phenyl]-4-methoxy-benzenesulfonamid, N-[3-[[3-(6-Fluoro-1,2-benzoxazol-3-yl)propylmethylamino]methyl]phenyl]-4-(trifluoro-methoxy)benzenesulfonamid, 3-Cyano-N-[3-[[3-(6-fluoro-1,2-benzoxazol-3-yl)propylmethylamino]methyl]phenyl]-benzenesulfonamid, N-[3-[[3-(6-Fluoro-1,2-benzoxazol-3-yl)propylmethylamino]methyl]phenyl]-4-phenyl-benzenesulfonamid, N-[3-[[3-(6-fluoro-1,2-benzoxazol-3-yl)propylmethylamino]methyl]phenyl]thiophene-2-sulfonamid, N-[3-[[3-(6-Fluoro-1,2-benzoxazol-3-yl)propylmethylamino]methyl]phenyl]thiophene-3-sulfonamid, N-[3-[[3-(6-Fluoro-1,2-benzoxazol-3-yl)propylmethylamino]methyl]phenyl]-2,5-dimethyl-thiophene-3-sulfonamid, N-[3-[[3-(6-Fluoro-1,2-benzoxazol-3-yl)propylmethylamino]methyl]phenyl]-5-isoxazol-5-yl-thiophene-2-sulfonamid,
N-[3-[[3-(6-Fluoro-1,2-benzoxazol-3-yl)propylmethylamino]methyl]phenyl]naphthalene-1-sulfonamid,
N-[3-[[3-(6-Fluoro-1,2-benzoxazol-3-yl)propylmethylamino]methyl]phenyl]naphthalene-2-sulfonamid,
6-Chloro-N-[3-[[3-(6-fluoro-1,2-benzoxazol-3-yl)propylmethylamino]methyl]phenyl]-naphthalene-2-sulfonamid,
N-[3-[[3-(6-Fluoro-1,2-benzoxazol-3-yl)propylmethylamino]methyl]phenyl]-2,3-dihydro-1,4-benzodioxino-6-sulfonamid,
N-[3-[[3-(6-Fluoro-1,2-benzoxazol-3-yl)propylmethylamino]methyl]phenyl]-1,3-benzo-dioxole-5-sulfonamid,
N-[3-[[3-(6-Fluoro-1,2-benzoxazol-3-yl)propylmethylamino]methyl]phenyl]-2-okso-indolino-5-sulfonamid,
N-[3-[[3-(6-Fluoro-1,2-benzoxazol-3-yl)propylmethylamino]methyl]phenyl]-6,7-dihydro-5H-pyrrole[1,2-a]imidazole-3-sulfonamid,
N-[3-[[3-(6-Fluoro-1,2-benzoxazol-3-yl)propylmethylamino]methyl]phenyl]-1-methyl-indole-4-sulfonamid,
N-[3-[[3-(6-Fluoro-1,2-benzoxazol-3-yl)propylmethylamino]methyl]phenyl]-1-methyl-indole-5-sulfonamid,
N-[3-[[3-(6-Fluoro-1,2-benzoxazol-3-yl)propylmethylamino]methyl]phenyl]-1,3-benzo-thiazole-4-sulfonamid,
N-[3-[[3-(6-Fluoro-1,2-benzoxazol-3-yl)propylmethylamino]methyl]phenyl]-1,3-benzo-thiazole-5-sulfonamid,
N-[3-[[3-(6-Fluoro-1,2-benzoxazol-3-yl)propylmethylamino]methyl]phenyl]benzofuran-2-sulfonamid,
N-[3-[[3-(6-Fluoro-1,2-benzoxazol-3-yl)propylmethylamino]methyl]phenyl]benzo-thiophene-3-sulfonamid,
5-Fluoro-N-[3-[[3-(6-fluoro-1,2-benzoxazol-3-yl)propylmethylamino]methyl]phenyl]-3-methyl-benzothiophene-2-sulfonamid,
5-Chloro-N-[3-[[3-(6-fluoro-1,2-benzoxazol-3-yl)propylmethylamino]methyl]phenyl]-3-methyl-benzothiophene-2-sulfonamid,
3-Chloro-4-fluoro-N-[3-[[3-(6-fluoro-1,2-benzoxazol-3-yl)propylmethylamino]methyl]-phenyl]benzenesulfonamid,
und pharmazeutisch verträgliche Salze und Solvate davon.

18. Die Verbindungen der Formel (I), wie in einem der Ansprüche 1 -17 definiert sind, zur Anwendung als Medikament.

19. Pharmazeutische Zusammensetzung, enthaltend als Wirkstoff eine Verbindung der Formel (I), wie in einem der Ansprüche 1 -17 definiert ist, in Kombination mit einem oder mehreren pharmazeutisch verträglichen Trägerstoff(en) und/oder Hilfsstoff(en).

20. Die Verbindung der Formel (I), wie in einem der Ansprüche 1 -17 definiert ist, zur Anwendung in einem Verfahren zur Behandlung und/oder Prävention von Störungen des zentralen Nervensystems.

21. Die Verbindung zur Anwendung nach Anspruch 20, wobei die Störung des zentralen Nervensystems ausgewählt ist aus der Gruppe bestehend aus Schizophrenie, schizoaffektiven Störungen, schizophreniformen Störungen, wahnhafter Syndrome und andere psychotische Zustände bezogen und nicht unter psychoaktiven Substanzen, affektive Störung, bipolare Störung, Manie, Depression, Angststörungen verschiedener Ätiologie, Stressreaktionen, Bewusstsein Störungen, Koma, Delirium von alkoholischen und andere Ätiologie, Aggression, psychomotorische Unruhe und andere Verhaltensstörungen, Schlafstörungen verwandter verschiedener Ätiologie, Entzugssyndromen verschiedener Ätiologie, Sucht, Schmerzsyndromen verschiedener Ätiologie, Intoxikation mit psychoaktiven Substanzen, zerebrale Durchblutungsstörungen verschiedener Ätiologie, psychosomatischen Störungen verschiedener Ätiologie, Konversionsstörungen, dissoziative Störungen, Störungen beim Wasserlassen, Autismus und anderen Entwicklungsstörungen, einschließlich Nykturie, Stottern, Tics, kognitiven Störungen verschiedener Art, wie Alzheimer-Krankheit, psychopathologische Symptome und neurologischen Störungen im Verlauf anderer Erkrankungen des zentralen und peripheren Nervensystems.

## Revendications

1. Composé de formule générale (I): 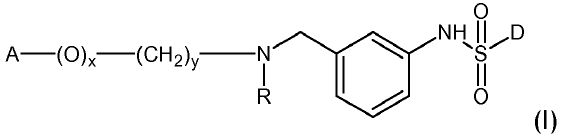 dans laquelle
A représente:
- un phényle non substitué ou substitué par un sel substituant choisi dans le groupe consistant en un atome d'halogène, un C₁-C₃ alkyle, un C₁-C₃ alkyloxy, OH et un phényle; ou
- un groupe bicyclique à 9 ou 10 chaînons, lié à - (O)ₓ-(CH₂)- par un de ses atomes de carbone aromatiques, consistant en cycle benzènique fusionné avec:
- un cycle hétéroaromatique à 5 chaînons contenant 1 ou 2 hétéroatomes choisis indépendamment dans le groupe consistant en N et O, dans lequel, si tel cycle hétéroaromatique contient 2 hétéroatomes alors au moins un est N et dans lequel un tel groupe bicyclique est non substitué ou substitué par un atome d'halogène ; ou
- un cycle hétérocyclique à 5 ou 6 chaînons non aromatique ayant 1 ou 2 atomes de O, et dans lequel le cycle hétérocyclique est non substitué ou substitué par un ou plus d'alkyles en C1-C3 ;
D représente un groupe choisi parmi:
- un phényle non substitué ou substitué par un ou deux substituants choisis indépendamment parmi un groupe consistant en un C1-C4-alkyle, un C1-C3-alkyloxy, un halogéno-C1-C3-alkyle, un halogéno-C1-C3-alkyloxy, un atome d'halogène, -CN, et un phényle;
- un naphtyle non substitué ou substitué par un atome d'halogène;
- thiophène non substitué ou substitué avec un ou deux substituants choisis indépendamment dans le groupe consistant en C1-C3-alkyle, un atome d'halogène, et un cycle hétéroaromatique à 5 chaînons ayant 1 ou 2 hétéroatomes indépendamment choisis parmi N et O, dans lequel, si tel cycle hétéroaromatique contient 2 hétéroatomes, alors au moins un est N;
- un groupe bicyclique consistant en cycle imidazole fusionné avec un cycle carbocyclique non aromatique à 5 chaînons, lié à groupement sulfonamide par l'un de ses atomes de carbone aromatiques;
- un groupe bicyclique consistant en cycle benzénique condensé avec un cycle hétéroaromatique à 5 chaînons, ayant 1 ou 2 hétéroatomes choisis indépendamment dans le groupe consistant en N, O et S, dans lequel, si tel cycle hétéroaromatique contient 2 hétéroatomes alors au moins un est N, et dans lequel le groupe bicyclique est non substitué ou substitué par un ou plusieurs substituants choisis indépendamment dans le groupe consistant en un groupe C1-C3 alkyle et un atome d'halogène, et lié à groupement sulfonamide par l'un de ses atomes de carbone aromatiques; et
- un groupe bicyclique consistant en un cycle benzénique fusionné avec un cycle hétérocyclique nonaromatique à 5 ou 6 chaînons ayant 1 ou 2 hétéroatomes choisis indépendamment dans le groupe consistant en N et O, non substitué ou substitué par un seul =O, et lié à groupement sulfonamide par l'un des atomes de carbone du cycle benzénique;
R représente H ou -CH₃;
x est 0 ou 1;
y est 2 ou 3;
et les sels et solvates pharmaceutiquement acceptables de ceux-ci,
avec les conditions que
- si x is 0 et y is 2, alors D est un naphtyle non substitué ou substitué par un seul atome d'halogène, et
- si R représente -CH₃, alors A n'est pas un phényle non substitué ou substitué.

2. Composé selon la revendication 1, dans lequel A représente un phényle non substitué ou substitué par un seul substituant choisi dans le groupe consistant en un atome d'halogène, un C₁-C₃ alkyle, un C₁-C₃ alkyloxy, OH et un phényle.

3. Composé selon la revendication 1, dans lequel A représente un groupe bicyclique à 9 ou 10 chaînons, lié à -(O)ₓ-(CH₂)- par un de ses atomes de carbone aromatiques, consistant en un cycle benzènenique fusionné avec un cycle hétéroaromatique à 5 chaînons contenant 1 ou 2 hétéroatomes choisis indépendamment dans le groupe consistant en N et O, dans lequel, si tel cycle hétéroaromatique contient 2 hétéroatomes alors au moins un est N et dans lequel un tel groupe bicyclique est non substitué ou substitué par un atome d'halogène.

4. Composé selon la revendication 1, dans lequel A représente un groupe bicyclique à 9 ou 10 chaînons, lié à -(O)ₓ-(CH₂)- par un de ses atomes de carbone aromatiques, consistant en un cycle benzènique fusionné avec un cycle hétérocyclique à 5 ou 6 chaînons non aromatique ayant 1 ou 2 atomes de O, et dans lequel cycle hétérocyclique est non substitué ou substitué par un ou plus d'alkyles C1-C3.

5. Composé selon l'une quelconque des revendications 1 à 4, dans lequel D représente un phényle non substitué ou substitué par un ou deux substituants choisis indépendamment parmi un groupe consistant en un C1-C4-alkyle, un C1-C3-alkyloxy, un halogéno-C1-C3-alkyle, un halogéno-C1-C3-alkyloxy, un atome d'halogène, -CN, et un phényle.

6. Composé selon l'une quelconque des revendications 1 à 4, dans lequel D représente un naphtyle non substitué ou substitué par un seul atome d'halogène.

7. Composé selon l'une quelconque des revendications 1 à 4, dans lequel D représente thiophène non substitué ou substitué avec un ou deux substituants choisis indépendamment dans le groupe consistant en alkyle C1-C3-, un atome d'halogène, et un cycle hétéroaromatique à 5 chaînons ayant 1 ou 2 hétéroatomes indépendamment choisis parmi N et O, dans lequel, si le tel cycle hétéroaromatique contient 2 hétéroatomes, alors au moins un est N.

8. Composé selon l'une quelconque des revendications 1 à 4, dans lequel D représente un groupe bicyclique consistant en un cycle imidazole fusionné avec un cycle carbocyclique non aromatique à 5 chaînons, lié à groupement sulfonamide par l'un de ses atomes de carbone aromatiques.

9. Composé selon l'une quelconque des revendications 1 à 4, dans lequel D représente un groupe bicyclique consistant en un cycle benzénique condensé avec un cycle hétéroaromatique à 5 chaînons, ayant 1 ou 2 hétéroatomes choisis indépendamment dans le groupe consistant en N, O et S, dans lequel, si le tel cycle hétéroaromatique contient 2 hétéroatomes alors au moins un est N, et dans lequel le groupe bicyclique est non substitué ou substitué par un ou plus des substituants choisis indépendamment dans le groupe consistant en un C1-C3 alkyle et un atome d'halogène, et est lié à groupement sulfonamide par l'un de ses atomes de carbone aromatiques.

10. Composé selon l'une quelconque des revendications 1 à 4 dans lequel D représente un groupe bicyclique consistant en un cycle benzénique fusionné avec un cycle hétérocyclique nonaromatique à 5 ou 6 chaînons ayant 1 ou 2 hétéroatomes choisis indépendamment dans le groupe consistant en N et O, non substitué ou substitué par un seul =O, et lié à groupement sulfonamide par l'un des atomes de carbone du cycle benzénique.

11. Composé selon l'une quelconque des revendications 1 à 10, dans lequel R représente H, ayant la formule générale (IA):

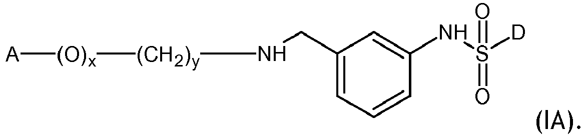

12. Composé selon l'une quelconque des revendications 1 à 10, dans lequel R représente -CH₃, ayant la formule générale (IB):

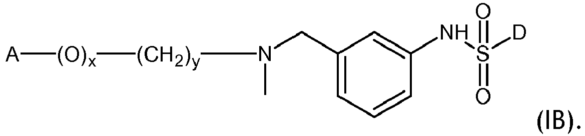

13. Composé selon l'une quelconque des revendications 1 à 12, dans lequel x est 1 et y est 2 ou x est 0 et y est 3.

14. Composé selon la revendication 13, dans lequel x est 1 et y est 2.

15. Composé selon la revendication 13, dans lequel x est 0 et y est 3.

16. Composé selon la revendication 6, dans lequel x est 0 et y est 2.

17. Composé selon la revendication 1 choisi dans le groupe consistant en les composés suivants :
N-[3-[(phényléthylamino)méthyl]phényl]naphtalène-2-sulfonamide,
N-[3-[(2-phénoxyéthylamino)méthyl]phényl]naphtalène-2-sulfonamide,
N-[3-[(3-phénylpropylamino)méthyl]phényl]naphtalène-2-sulfonamide,
N-[3-[[2-(3-fluorophénoxy)éthylamino]méthyl]phényl]naphtalène-2-sulfonamide,
N-[3-[[3-(3-fluorophénoxy)propylamino]méthyl]phényl]naphtalène-2-sulfonamide,
N-[3-[[2-(3-chlorophényl)éthylamino]méthyl]phényl]naphtalène-2-sulfonamide,
N-[3-[[2-(3-chlorophénoxy)éthylamino]méthyl]phényl]naphtalène-2-sulfonamide,
N-[3-[[2-(3-méthylphényl)éthylamino]méthyl]phényl]naphtalène-2-sulfonamide,
N-[3-[[2-(2-méthoxyphényl)éthylamino]méthyl]phényl]naphtalène-2-sulfonamide,
N-[3-[[2-(2-méthoxyphénoxy)éthylamino]méthyl]phényl]naphtalène-2-sulfonamide,
N-[3-[[2-(3-méthoxyphényl)éthylamino]méthyl]phényl]naphtalène-2-sulfonamide,
N-[3-[[2-(2,3-dihydro-1,4-benzodioxin-5-yloxy)éthylamino]méthyl]phényl]-4-propylbenzènesulfonamide,
N-[3-[[2-(3-méthoxyphénoxy)éthylamino]méthyl]phényl]naphtalène-2-sulfonamide,
N-[3-[[2-(4-méthoxyphényl)éthylamino]méthyl]phényl]naphtalène-2-sulfonamide,
N-[3-[[2-(4-méthoxyphénoxy)éthylamino]méthyl]phényl]naphtalène-2-sulfonamide,
N-[3-[[3-(4-méthoxyphényl)propylamino]méthyl]phényl]naphtalène-2-sulfonamide,
N-[3-[[2-(3-hydroxyphényl)éthylamino]méthyl]phényl]naphtalène-2-sulfonamide,
N-[3-[[2-(2,3-dihydro-1,4-benzodioxin-5-yloxy)éthylamino]méthyl]phényl]-benzènesulfonamide,
N-[3-[[2-(2,3-dihydro-1,4-benzodioxin-5-yloxy)éthylamino]méthyl]phényl]-3-fluorobenzènesulfonamide,
N-[3-[[2-(2,3-dihydro-1,4-benzodioxin-5-yloxy)éthylamino]méthyl]phényl]-4-fluorobenzènesulfonamide,
4-chloro-N-[3-[[2-(2,3-dihydro-1,4-benzodioxin-5-yloxy)éthylamino]méthyl]-phényl]benzènesulfonamide,
N-[3-[[2-(2,3-dihydro-1,4-benzodioxin-5-yloxy)éthylamino]méthyl]phényl]-4-iodo-benzènesulfonamide,
N-[3-[[2-(2,3-dihydro-1,4-benzodioxin-5-yloxy)éthylamino]méthyl]phényl]-3-méthylbenzènesulfonamide,
N-[3-[[2-(2,3-dihydro-1,4-benzodioxin-5-yloxy)éthylamino]méthyl]phényl]-3-(trifluorométhyl)benzènesulfonamide,
N-[3-[[2-(2,3-dihydro-1,4-benzodioxin-5-yloxy)éthylamino]méthyl]phényl]-3-méthoxybenzènesulfonamide,
N-[3-[[2-(2,3-dihydro-1,4-benzodioxin-5-yloxy)éthylamino]méthyl]phényl]-4-méthoxybenzènesulfonamide,
N-[3-[[2-(2,3-dihydro-1,4-benzodioxin-5-yloxy)éthylamino]méthyl]phényl]-4-(trifluorométhoxy)benzènesulfonamide,
3-cyano-N-[3-[[2-(2,3-dihydro-1,4-benzodioxin-5-yloxy)éthylamino]méthyl]-phényl]benzènesulfonamide,
4-cyano-N-[3-[[2-(2,3-dihydro-1,4-benzodioxin-5-yloxy)éthylamino]méthyl]-phényl]benzènesulfonamide,
N-[3-[[2-(2,3-dihydro-1,4-benzodioxin-5-yloxy)éthylamino]méthyl]phényl]-4-phénylbenzènesulfonamide,
N-[3-[[2-(2,3-dihydro-1,4-benzodioxin-5-yloxy)éthylamino]méthyl]phényl]-thiophène-2-sulfonamide,
N-[3-[[2-(2,3-dihydro-1,4-benzodioxin-5-yloxy)éthylamino]méthyl]phényl]-thiophène-3-sulfonamide,
N-[3-[[2-(2,3-dihydro-1,4-benzodioxin-5-yloxy)éthylamino]méthyl]phényl]-2,5-diméthyl-thiophène-3-sulfonamide,
N-[3-[[2-(2,3-dihydro-1,4-benzodioxin-5-yloxy)éthylamino]méthyl]phényl]-5-isoxazol-5-yl-thiophène-2-sulfonamide,
N-[3-[[2-(2,3-dihydro-1,4-benzodioxin-5-yloxy)éthylamino]méthyl]phényl]-naphtalène-1-sulfonamide,
N-[3-[[2-(2,3-dihydro-1,4-benzodioxin-5-yloxy)éthylamino]méthyl]phényl]-naphtalène-2-sulfonamide,
6-chloro-N-[3-[[2-(2,3-dihydro-1,4-benzodioxin-5-yloxy)éthylamino]méthyl]-phényl]naphtalène-2-sulfonamide,
N-[3-[[2-(2,3-dihydro-1,4-benzodioxin-5-yloxy)éthylamino]méthyl]phényl]-1,3-benzodioxole-5-sulfonamide,
N-[3-[[2-(2,3-dihydro-1,4-benzodioxin-5-yloxy)éthylamino]méthyl]phényl]-6,7-dihydro-5H-pyrrole[1,2-a]imidazole-3-sulfonamide,
N-[3-[[2-(2,3-dihydro-1,4-benzodioxin-5-yloxy)éthylamino]méthyl]phényl]-1-méthyl-indole-4-sulfonamide,
N-[3-[[2-(2,3-dihydro-1,4-benzodioxin-5-yloxy)éthylamino]méthyl]phényl]-1-méthyl-indole-5-sulfonamide,
N-[3-[[2-(2,3-dihydro-1,4-benzodioxin-5-yloxy)éthylamino]méthyl]phényl]-benzofuran-2-sulfonamide,
N-[3-[[2-(2,3-dihydro-1,4-benzodioxin-5-yloxy)éthylamino]méthyl]phényl]benzothiophène-3-sulfonamide,
N-[3-[[2-(2,3-dihydro-1,4-benzodioxin-5-yloxy)éthylamino]méthyl]phényl]-5-fluoro-3-méthyl-benzothiophène-2-sulfonamide,
3-chloro-N-[3-[[2-(2,3-dihydro-1,4-benzodioxin-5-yloxy)éthylamino]méthyl]-phényl]-4-fluorobenzènesulfonamide,
N-[3-[[2-[(2,2-diméthyl-3H-benzofuran-7-yl)oxy]éthylamino]méthyl]phényl]-3-méthylbenzènesulfonamide,
N-[3-[[2-[(2,2-diméthyl-3H-benzofuran-7-yl)oxy]éthylamino]méthyl]phényl]-naphtalène-1-sulfonamide,
N-[3-[[2-[(2,2-diméthyl-3H-benzofuran-7-yl)oxy]éthylamino]méthyl]phényl]-naphtalène-2-sulfonamide,
N-[3-[[2-(1H-indol-4-yloxy)éthylamino]méthyl]phényl]-3-méthylbenzènesulfonamide,
N-[3-[[2-(1H-indol-4-yloxy)éthylamino]méthyl]phényl]-4-phénylbenzènesulfonamide,
N-[3-[[2-(1H-indol-4-yloxy)éthylamino]méthyl]phényl]naphtalène-1-sulfonamide,
N-[3-[[2-(1H-indol-4-yloxy)éthylamino]méthyl]phényl]naphtalène-2-sulfonamide,
N-[3-[[3-(5-fluoro-1,2-benzoxazol-3-yl)propylamino]méthyl]phényl]-3-méthylbenzènesulfonamide,
N-[3-[[3-(6-fluoro-1,2-benzoxazol-3-yl)propylamino]méthyl]phényl]naphtalène-1-sulfonamide,
N-[3-[[3-(6-fluoro-1,2-benzoxazol-3-yl)propylamino]méthyl]phényl]naphtalène-2-sulfonamide,
N-[3-[[2-(3-méthylphénoxy)éthylamino]méthyl]phényl]naphtalène-2-sulfonamide,
N-[3-[[2-(3-hydroxyphénoxy)éthylamino]méthyl]phényl]naphtalène-2-sulfonamide,
N-[3-[[2-(1H-indol-4-yloxy)éthylamino]méthyl]phényl]-4-propyl-benzènesulfonamide,
N-[3-[[2-(1H-indol-4-yloxy)éthylamino]méthyl]phényl]thiophène-2-sulfonamide,
N-[3-[[2-(1H-indol-4-yloxy)éthylamino]méthyl]phényl]thiophène-3-sulfonamide,
N-[3-[[2-(1H-indol-4-yloxy)éthylamino]méthyl]phényl]-2,5-diméthyl-thiophène-3-sulfonamide,
N-[3-[[2-(2,3-dihydro-1,4-benzodioxin-5-yloxy)éthyl-méthyl-amino]méthyl]-phényl]benzènesulfonamide,
N-[3-[[2-[(2,2-diméthyl-3H-benzofuran-7-yl)oxy]éthyl-méthylamino]méthyl]-phényl]benzènesulfonamide,
N-[3-[[2-(1 H-indol-4-yloxy)éthylméthylamino]méthyl]phényl]benzenesulfonamide,
N-[3-[[3-(6-fluoro-1,2-benzoxazol-3-yl)propylméthylamino]méthyl]phényl]-benzènesulfonamide,
4-fluoro-N-[3-[[3-(6-fluoro-1, 2-benzoxazol-3-yl)propylméthylamino] méthyl]-phényl]benzènesulfonamide,
3,4-difluoro-N-[3-[[3-(6-fluoro-1,2-benzoxazol-3-yl)propylméthylamino]méthyl]-phényl]benzènesulfonamide,
3-chloro-N-[3-[[3-(6-fluoro-1, 2-benzoxazol-3-yl)propylméthylamino] méthyl]-phényl]benzènesulfonamide,
4-chloro-N-[3-[[3-(6-fluoro-1, 2-benzoxazol-3-yl)propylméthylamino] méthyl]-phényl]benzènesulfonamide,
3,4-dichloro-N-[3-[[3-(6-fluoro-1, 2-benzoxazol-3-yl)propylméthylamino] méthyl]-phényl]benzènesulfonamide,
3-bromo-N-[3-[[3-(6-fluoro-1, 2-benzoxazol-3-yl)propylméthylamino] méthyl]-phényl]benzènesulfonamide,
4-bromo-N-[3-[[3-(6-fluoro-1, 2-benzoxazol-3-yl)propylméthylamino] méthyl]-phényl]benzènesulfonamide,
N-[3-[[3-(6-fluoro-1,2-benzoxazol-3-yl)propylméthylamino]méthyl]phényl]-4-iodobenzènesulfonamide,
N-[3-[[3-(6-fluoro-1,2-benzoxazol-3-yl)propylméthylamino]méthyl]phényl]-3-méthylbenzènesulfonamide,
N-[3-[[3-(6-fluoro-1,2-benzoxazol-3-yl)propylméthylamino]méthyl]phényl]-4-méthylbenzènesulfonamide,
N-[3-[[3-(6-fluoro-1,2-benzoxazol-3-yl)propylméthylamino]méthyl]phényl]-4-propylbenzènesulfonamide,
4-tert-butyl-N-[3-[[3-(6-fluoro-1,2-benzoxazol-3-yl)propylméthylamino]méthyl]-phényl] benzènesulfonamide,
N-[3-[[3-(6-fluoro-1,2-benzoxazol-3-yl)propylméthylamino]méthyl]phényl]-3-(trifluorométhyl)benzènesulfonamide,
N-[3-[[3-(6-fluoro-1,2-benzoxazol-3-yl)propylméthylamino]méthyl]phényl]-4-(trifluorométhyl)benzènesulfonamide,
N-[3-[[3-(6-fluoro-1,2-benzoxazol-3-yl)propylméthylamino]méthyl]phényl]-3-méthoxybenzènesulfonamide,
N-[3-[[3-(6-fluoro-1,2-benzoxazol-3-yl)propylméthylamino]méthyl]phényl]-4-méthoxy-benzènesulfonamide,
N-[3-[[3-(6-fluoro-1,2-benzoxazol-3-yl)propylméthylamino]méthyl]phényl]-4-(trifluorométhoxy)benzènesulfonamide,
3-cyano-N-[3-[[3-(6-fluoro-1, 2-benzoxazol-3-yl)propylméthylamino] méthyl]-phényl] benzènesulfonamide,
N-[3-[[3-(6-fluoro-1,2-benzoxazol-3-yl)propylméthylamino]méthyl]phényl]-4-phénylbenzènesulfonamide,
N-[3-[[3-(6-fluoro-1,2-benzoxazol-3-yl)propylméthylamino]méthyl]phényl]-thiophène-2-sulfonamide,
N-[3-[[3-(6-fluoro-1,2-benzoxazol-3-yl)propylméthylamino]méthyl]phényl]-thiophène-3-sulfonamide,
N-[3-[[3-(6-fluoro-1,2-benzoxazol-3-yl)propylméthylamino]méthyl]phényl]-2,5-diméthyl-thiophène-3-sulfonamide,
N-[3-[[3-(6-fluoro-1,2-benzoxazol-3-yl)propylméthylamino]méthyl]phényl]-5-isoxazol-5-yl-thiophène-2-sulfonamide,
N-[3-[[3-(6-fluoro-1,2-benzoxazol-3-yl)propylméthylamino]méthyl]phényl]-naphtalène-1-sulfonamide,
N-[3-[[3-(6-fluoro-1,2-benzoxazol-3-yl)propylméthylamino]méthyl]phényl]-naphtalène-2-sulfonamide,
6-chloro-N-[3-[[3-(6-fluoro-1, 2-benzoxazol-3-yl)propylméthylamino] méthyl]-phényl]naphtalène-2-sulfonamide,
N-[3-[[3-(6-fluoro-1,2-benzoxazol-3-yl)propylméthylamino]méthyl]phényl]-2,3-dihydro-1,4-benzodioxino-6-sulfonamide,
N-[3-[[3-(6-fluoro-1,2-benzoxazol-3-yl)propylméthylamino]méthyl]phényl]-1,3-benzodioxole-5-sulfonamide,
N-[3-[[3-(6-fluoro-1,2-benzoxazol-3-yl)propylméthylamino]méthyl]phényl]-2-oxo-indolino-5-sulfonamide,
N-[3-[[3-(6-fluoro-1,2-benzoxazol-3-yl)propylméthylamino]méthyl]phényl]-6,7-dihydro-5H-pyrrole[1,2-a]imidazole-3-sulfonamide,
N-[3-[[3-(6-fluoro-1,2-benzoxazol-3-yl)propylméthylamino]méthyl]phényl]-1-méthyl-indole-4-sulfonamide,
N-[3-[[3-(6-fluoro-1,2-benzoxazol-3-yl)propylméthylamino]méthyl]phényl]-1-méthyl-indole-5-sulfonamide,
N-[3-[[3-(6-fluoro-1,2-benzoxazol-3-yl)propylméthylamino]méthyl]phényl]-1,3-benzothiazole-4-sulfonamide,
N-[3-[[3-(6-fluoro-1,2-benzoxazol-3-yl)propylméthylamino]méthyl]phényl]-1,3-benzothiazole-5-sulfonamide,
N-[3-[[3-(6-fluoro-1,2-benzoxazol-3-yl)propylméthylamino]méthyl]phényl]-benzofuran-2-sulfonamide,
N-[3-[[3-(6-fluoro-1,2-benzoxazol-3-yl)propylméthylamino]méthyl]phényl]benzothiophène-3-sulfonamide,
5-fluoro-N-[3-[[3-(6-fluoro-1, 2-benzoxazol-3-yl)propylméthylamino] méthyl]-phényl]-3-méthyl-benzothiophène-2-sulfonamide,
5-chloro-N-[3-[[3-(6-fluoro-1, 2-benzoxazol-3-yl)propylméthylamino] méthyl]-phényl]-3-méthyl-benzothiophène-2-sulfonamide,
3-chloro-4-fluoro-N-[3-[[3-(6-fluoro-1, 2-benzoxazol-3-yl)propylméthylamino]-méthyl] phényl] benzènesu lfonamide,
et ses sels pharmaceutiquement acceptables et solvates.

18. Les composés de formula (I) tels que définis dans l'une quelconque des revendications 1 -17 pour une utilisation comme médicament.

19. Composition pharmaceutique comprenant un composé de formule (I) tel que défini dans l'une quelconque des revendications 1 -17 comme l'ingrédient actif en combinaison avec véhicule(s) et / ou excipient (s) pharmaceutiquement acceptable(s).

20. Le composé de formule (I) tel que défini dans l'une quelconque des revendications 1 - 17 pour une utilisation dans une méthode de traitement et / ou la prévention de troubles du système nerveux central.

21. Composé pour utilisation selon la revendication 20, dans laquelle le trouble du système nerveux central est choisi dans le groupe consistant en schizophrénie, troubles schizo-affectifs, les troubles schizophréniformes, les syndromes délirants et d'autres états psychotiques associés et pas liées à la prise de substances psychoactives, trouble affectif, trouble bipolaire, la manie, la dépression, les troubles anxieux de diverses étiologies, les réactions de stress, les troubles de conscience, le coma, délire d'étiologie alcoolique et l'autre, l'agressivité, l'agitation psychomotrice et d'autres troubles du comportement, troubles du sommeil de diverses étiologies, les syndromes de sevrage de diverses étiologies, la toxicomanie, les syndromes de douleur de diverses étiologies, l'intoxication par des substances psychoactives, les troubles circulatoires cérébraux de diverses étiologies, troubles psychosomatiques de diverses étiologies, les troubles de conversion, les troubles dissociatifs, troubles de la miction, l'autisme et autres troubles du développement, y compris la nycturie, le bégaiement, tics, troubles cognitifs de différents types, comme la maladie d'Alzheimer, des symptômes psychopathologiques et des troubles neurologiques au cours d'autres maladies des systèmes nerveux central et périphérique.
